(19) Europäisches Patentamt / European Patent Office / Office européen des brevets

(11) EP 4 223 739 A1

(12) **EUROPEAN PATENT APPLICATION**

(43) Date of publication:
**09.08.2023 Bulletin 2023/32**

(21) Application number: **22154720.1**

(22) Date of filing: **02.02.2022**

(51) International Patent Classification (IPC):
***C07C 255/51*** *(2006.01)* ***C07D 213/26*** *(2006.01)*
***C07D 213/85*** *(2006.01)* ***C07D 239/26*** *(2006.01)*
***H01L 51/00*** *(2006.01)*

(52) Cooperative Patent Classification (CPC):
**C07D 213/26; C07C 255/51; C07D 213/85;**
**C07D 239/26; H10K 85/60; H10K 85/654;**
C07C 2601/16; H10K 50/17; H10K 50/19

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Novaled GmbH**
**01099 Dresden (DE)**

(72) Inventors:
- **LUSCHTINETZ, Regina**
**01099 Dresden (DE)**
- **NÜLLEN, Max Peter**
**01099 Dresden (DE)**
- **PINTER, Piemaria**
**01099 Dresden (DE)**
- **SCHULZE, Benjamin**
**01099 Dresden (DE)**
- **WUDARCZYK, Jakob Jacek**
**01099 Dresden (DE)**

(74) Representative: **Michalski Hüttermann & Partner Patentanwälte mbB**
**Kaistraße 16A**
**40221 Düsseldorf (DE)**

Remarks:
A request for correction of the claims has been filed pursuant to Rule 139 EPC. A decision on the request will be taken during the proceedings before the Examining Division (Guidelines for Examination in the EPO, A-V, 3.).

(54) **3,6-BIS(CYANOMETHYLIDENE)CYCLOHEXA-1,4-DIENE COMPOUNDS AND THEIR USE IN ORGANIC ELECTRONIC DEVICES**

(57) The invention is directed to a compound of formula (I) for use in organic electronic devices, an organic semiconductor layer comprising the compound of formula (I), an organic electronic device comprising the organic semiconductor layer, and a display device comprising the organic electronic device.

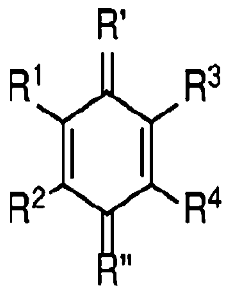
(I),
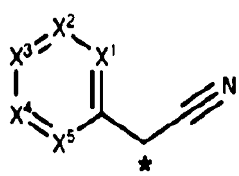
(II);
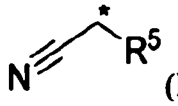
(III);

wherein $R^1$, $R^2$, $R^3$ and $R^4$ are independently selected from the group comprising F, Cl, CN or $CF_3$; R' is selected from formula (II); R" is selected from formula (III); $R^5$ is selected from substituted or unsubstituted aryl, substituted or unsubstituted heteroaryl or CN; and the " * " denotes the binding position.

EP 4 223 739 A1

## Description

## Technical Field

The present invention relates to a compound of formula (I) for use in organic electronic devices, an organic semiconductor layer comprising the compound of formula (I), an organic electronic device comprising the organic semiconductor layer, and a display device comprising the organic electronic device.

## Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the semiconductor layer, and among them, may be affected by characteristics of metal complexes which are also contained in the semiconductor layer.

There remains a need to improve performance of organic semiconductor materials, semiconductor layers, as well as organic electronic devices thereof, in particular to achieve improved operating voltage, improved efficiency, improved lifetime and/or improved voltage stability over time through improving the characteristics of the compounds comprised therein. In addition, there remains a need to improved LUMO energy, improve the dipole moment and/or improve the thermal properties compared to comparative examples, in particular to improve thermal stability and/or processing properties at elevated temperatures.

## DISCLOSURE

An aspect of the present invention provides a compound of formula (I):

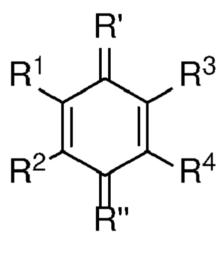

(I),

wherein

$R^1$, $R^2$, $R^3$ and $R^4$ are independently selected from the group comprising F, Cl, CN or $CF_3$;
R' is selected from formula (II)

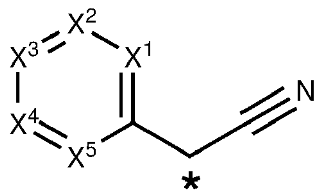

(II);

wherein

$X^1$ is selected from $CR^6$ or N;
$X^2$ is selected from $CR^7$ or N;
$X^3$ is selected from $CR^8$ or N;
$X^4$ is selected from $CR^9$ or N;
$X^5$ is selected from $CR^{10}$ or N; wherein

$R^6$, $R^7$, $R^8$, $R^9$ and $R^{10}$ are independently selected from CN, partially fluorinated or perfluorinated $C_1$ to $C_8$ alkyl, halogen, Cl, F, D or H,

wherein at least one $X^1$ to $X^5$ is selected from CH, CD or N;
R" is selected from formula (III)

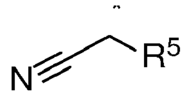
(III);

wherein

$R^5$ is selected from substituted or unsubstituted $C_6$ to $C_{19}$ aryl, substituted or unsubstituted $C_6$ to $C_{19}$ aryl with one, two or three aromatic six-member rings, substituted or unsubstituted $C_9$ to $C_{19}$ aryl with two or three fused aromatic six-member rings, substituted or unsubstituted $C_6$ to $C_{12}$ aryl with one or two aromatic six-member rings, substituted or unsubstituted phenyl, substituted or unsubstituted biphenyl, substituted or unsubstituted $C_3$ to $C_{20}$ heteroaryl, substituted or unsubstituted $C_3$ to $C_{20}$ heteroaryl with one, two or three aromatic six-member rings, substituted or unsubstituted $C_7$ to $C_{20}$ heteroaryl with two or three fused aromatic six-member rings or CN,

wherein the substituents are selected from CN, partially fluorinated or perfluorinated $C_1$ to $C_8$ alkyl, halogen, Cl, F, $C_1$ to $C_8$ alkyl or D; wherein the "*" denotes the binding position; and optional the following formula (E1) is excluded:

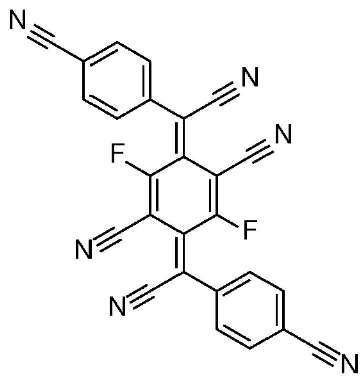
(E1).

It should be noted that throughout the application and the claims any $R^1$, $R^2$, $R^3$, $R^4$, $R^5$, $R^6$, $R^7$, $R^8$, $R^9$, $R^{10}$, R', R", etc. always refer to the same moieties, unless otherwise noted.

In the present specification, when a definition is not otherwise provided, "partially fluorinated" refers to a $C_1$ to $C_8$ alkyl group in which only part of the hydrogen atoms are replaced by fluorine atoms.

In the present specification, when a definition is not otherwise provided, "perfluorinated" refers to a $C_1$ to $C_8$ alkyl group in which all hydrogen atoms are replaced by fluorine atoms.

In the present specification, when a definition is not otherwise provided, "substituted" refers to one substituted with a deuterium, $C_1$ to $C_{12}$ alkyl and $C_1$ to $C_{12}$ alkoxy.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, an "alkyl group" refers to a saturated aliphatic hydrocarbyl group. The alkyl group may be a $C_1$ to $C_{12}$ alkyl group. More specifically, the alkyl group may be a $C_1$ to $C_{10}$ alkyl group or a $C_1$ to $C_6$ alkyl group. For example, a $C_1$ to $C_4$ alkyl group includes 1 to 4 carbons in alkyl

chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an isopropyl group, a butyl group, an iso-butyl group, a sec-butyl group, a tert-butyl group, a pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphthyl or fluoren-2-yl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common $sp^2$-hybridized carbon atoms.

The term "six-member ring" is understood to mean a ring formed by 6 atoms. The ring-forming atoms of the "six-member ring" may be bonded to further atoms outside the ring, for example hydrogen atoms.

The term "five-member ring" is understood to mean a ring formed by 5 atoms. The ring-forming atoms of the "five-member ring "may be bonded to further atoms outside the ring, for example hydrogen atoms.

In the present specification, the single bond refers to a direct bond.

The term "free of', "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "p-type charge generation layer", "p-CGL" and "hole generation layer" are used synonymously.

The terms "n-type charge generation layer", "n-CGL" and "electron generation layer" are used synonymously.

The terms "OLED", "organic light-emitting diode" and "organic light-emitting device" are used synonymously.

The terms "anode", "anode layer" and "anode electrode" are used synonymously.

The terms "cathode", "cathode layer" and "cathode electrode" are used synonymously.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

**Advantageous Effects**

Surprisingly, it was found that the organic compound of the present invention according to formula (I) solves the problem underlying the present invention by enabling devices in various aspects superior over the organic electroluminescent devices known in the art, with respect to operating voltage, efficiency and/or lifetime, and in particular with respect to operating voltage, efficiency and voltage stability over time. It has be further surprisingly found that compounds of formula (I) may have improved LUMO energy, improved dipole moment and/or improved thermal properties compared to comparative examples, in particular improved thermal stability and/or processing properties at elevated temperatures.

Without being bound by theory, the thermal properties may be improved when R' in formula (I) is selected in the claimed range. Thereby, the thermal stability, as determined by TGA5%, and volatility, as determined by the rate onset temperature, may be in the range required for mass production.

Surprisingly, it was found that the LUMO energy may be in the range required for efficient hole injection and/or hole generation, when the R' is selected in the claimed range.

**Compound of formula (I)**

According to one embodiment of the present invention, the compound of formula (I) is represented by:

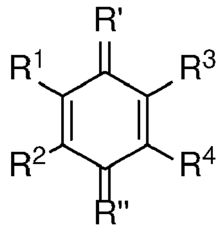

(I),

wherein

$R^1$, $R^2$, $R^3$ and $R^4$ are independently selected from the group comprising F, Cl, CN or $CF_3$;
R' is selected from formula (II)

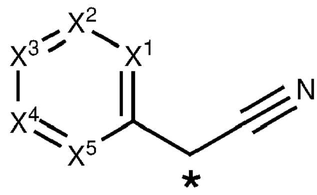

(II);

wherein

$X^1$ is selected from $CR^6$ or N;
$X^2$ is selected from $CR^7$ or N;
$X^3$ is selected from $CR^8$ or N;
$X^4$ is selected from $CR^9$ or N;
$X^5$ is selected from $CR^{10}$ or N; wherein

$R^6$, $R^7$, $R^8$, $R^9$ and $R^{10}$ are independently selected from CN, partially fluorinated or perfluorinated $C_1$ to $C_8$ alkyl, halogen, Cl, F, D or H,

wherein at least one $X^1$ to $X^5$ is selected from CH, CD or N;
R" is selected from formula (III)

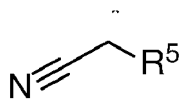

(III);

wherein

$R^5$ is selected from substituted or unsubstituted $C_6$ to $C_{19}$ aryl, substituted or unsubstituted $C_6$ to $C_{19}$ aryl with one, two or three aromatic six-member rings, substituted or unsubstituted $C_9$ to $C_{19}$ aryl with two or three fused aromatic six-member rings, substituted or unsubstituted $C_6$ to $C_{12}$ aryl with one or two aromatic six-member rings, substituted or unsubstituted phenyl, substituted or unsubstituted biphenyl, substituted or unsubstituted $C_3$ to $C_{20}$ heteroaryl, substituted or unsubstituted $C_3$ to $C_{20}$ heteroaryl with one, two or three aromatic six-member rings, substituted or unsubstituted $C_7$ to $C_{20}$ heteroaryl with two or three fused aromatic six-member rings or CN,

wherein the substituents are selected from CN, partially fluorinated or perfluorinated $C_1$ to $C_6$ alkyl, halogen, Cl, F, $C_1$ to $C_6$ alkyl or D;
wherein the "*" denotes the binding position; wherein the six-member rings are aromatic six member rings and optional the following formula (E1) is excluded:

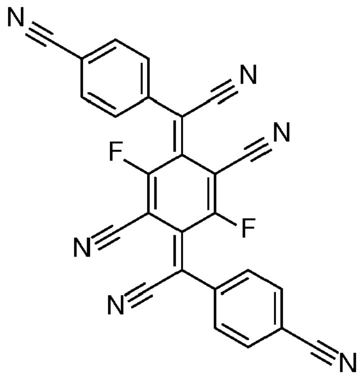
(E1).

According to one embodiment of the present invention, the compound of formula (I) is represented by:

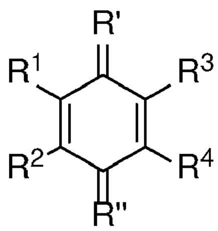
(I),

wherein

$R^1$, $R^2$, $R^3$ and $R^4$ are independently selected from the group comprising F, Cl, CN or $CF_3$;
R' is selected from formula (II)

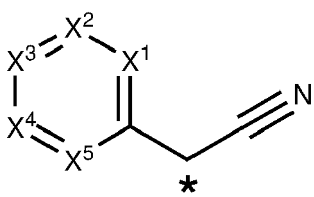
(II);

wherein

$X^1$ is selected from $CR^6$ or N;
$X^2$ is selected from $CR^7$ or N;
$X^3$ is selected from $CR^8$ or N;
$X^4$ is selected from $CR^9$ or N;
$X^5$ is selected from $CR^{10}$ or N;
wherein

$R^6$, $R^7$, $R^8$, $R^9$ and $R^{10}$ are independently selected from CN, partially fluorinated or perfluorinated $C_1$ to $C_8$ alkyl, halogen, Cl, F, D or H,

wherein at least one $X^1$ to $X^5$ is selected from CH, CD or N;
R" is selected from formula (III)

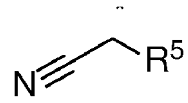
(III);

wherein

$R^5$ is selected from substituted or unsubstituted $C_6$ to $C_{19}$ aryl, substituted or unsubstituted $C_6$ to $C_{19}$ aryl with one, two or three aromatic six-member rings, substituted or unsubstituted $C_9$ to $C_{19}$ aryl with two or three fused aromatic six-member rings, substituted or unsubstituted $C_6$ to $C_{12}$ aryl with one or two aromatic six-member rings, substituted or unsubstituted phenyl, substituted or unsubstituted biphenyl, substituted or unsubstituted $C_3$ to $C_{20}$ heteroaryl, substituted or unsubstituted $C_3$ to $C_{20}$ heteroaryl with one, two or three aromatic six-member rings, substituted or unsubstituted $C_7$ to $C_{20}$ heteroaryl with two or three fused aromatic six-member rings or CN,

wherein the substituents are selected from CN, partially fluorinated or perfluorinated $C_1$ to $C_8$ alkyl, halogen, Cl, F, $C_1$ to $C_8$ alkyl or D;
wherein the "*" denotes the binding position;
wherein the compound of formula (I) is free of a five member ring; and
optional the following formula (E1) is excluded:

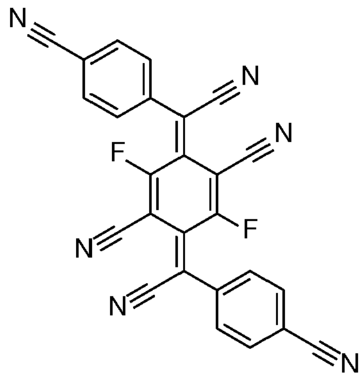
(E1).

According to one embodiment of the present invention, the compound of formula (I) is represented by:

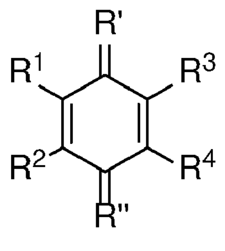
(I),

wherein

$R^1$, $R^2$, $R^3$ and $R^4$ are independently selected from the group comprising F, Cl, CN or $CF_3$;
R' is selected from formula (II)

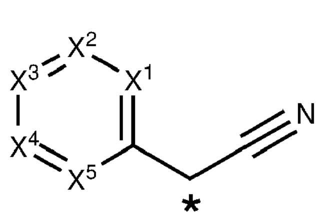
(II);

wherein

$X^1$ is selected from $CR^6$ or N;
$X^2$ is selected from $CR^7$ or N;
$X^3$ is selected from $CR^8$ or N;
$X^4$ is selected from $CR^9$ or N;
$X^5$ is selected from $CR^{10}$ or N;
wherein

$R^6$, $R^7$, $R^8$, $R^9$ and $R^{10}$ are independently selected from CN, partially fluorinated or perfluorinated $C_1$ to $C_8$ alkyl, halogen, Cl, F, D or H,

wherein at least one $X^1$ to $X^5$ is selected from CH, CD or N;
R" is selected from formula (III)

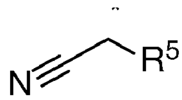
(III);

wherein

$R^5$ is selected from substituted or unsubstituted $C_6$ to $C_{19}$ aryl, substituted or unsubstituted $C_6$ to $C_{19}$ aryl with one, two or three aromatic six-member rings, substituted or unsubstituted $C_9$ to $C_{19}$ aryl with two or three fused aromatic six-member rings, substituted or unsubstituted $C_6$ to $C_{12}$ aryl with one or two aromatic six-member rings, substituted or unsubstituted phenyl, substituted or unsubstituted biphenyl, substituted or unsubstituted $C_3$ to $C_{20}$ heteroaryl, substituted or unsubstituted $C_3$ to $C_{20}$ heteroaryl with one, two or three aromatic six-member rings, substituted or unsubstituted $C_7$ to $C_{20}$ heteroaryl with two or three fused aromatic six-member rings or CN,

wherein the substituents are selected from CN, partially fluorinated or perfluorinated $C_1$ to $C_8$ alkyl, halogen, Cl, F, $C_1$ to $C_8$ alkyl or D;
wherein the "*" denotes the binding position;
wherein the compound of formula (I) comprises 1 to 6, or 2 to 5, or 3 to 4 six-member ring, preferably the six-member rings are aromatic six member rings; and
optional the following formula (E1) is excluded:

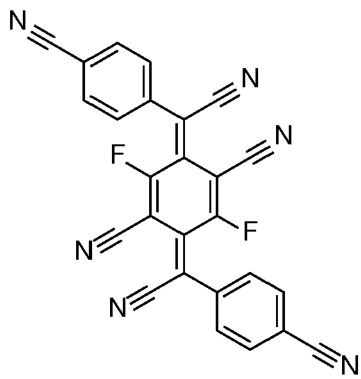
(E1).

According to one embodiment of the present invention, the compound of formula (I) is represented by:

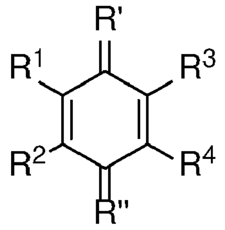
(I),

wherein

$R^1$, $R^2$, $R^3$ and $R^4$ are independently selected from the group comprising F, Cl, CN or $CF_3$;
R' is selected from formula (II)

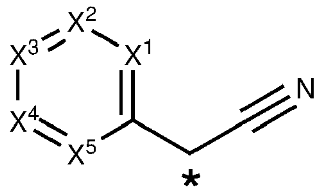
(II);

wherein

$X^1$ is selected from $CR^6$ or N;
$X^2$ is selected from $CR^7$ or N;
$X^3$ is selected from $CR^8$ or N;
$X^4$ is selected from $CR^9$ or N;
$X^5$ is selected from $CR^{10}$ or N;
wherein

$R^6$, $R^7$, $R^8$, $R^9$ and $R^{10}$ are independently selected from CN, partially fluorinated or perfluorinated $C_1$ to $C_8$ alkyl, halogen, Cl, F, D or H,

wherein at least one $X^1$ to $X^5$ is selected from CH, CD or N;
R" is selected from formula (III)

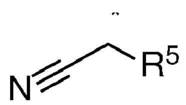
(III);

wherein

$R^5$ is selected from substituted or unsubstituted $C_6$ to $C_{19}$ aryl, substituted or unsubstituted $C_6$ to $C_{19}$ aryl with one, two or three aromatic six-member rings, substituted or unsubstituted $C_9$ to $C_{19}$ aryl with two or three fused aromatic six-member rings, substituted or unsubstituted $C_6$ to $C_{12}$ aryl with one or two aromatic six-member rings, substituted or unsubstituted phenyl, substituted or unsubstituted biphenyl, substituted or unsubstituted $C_3$ to $C_{20}$ heteroaryl, substituted or unsubstituted $C_3$ to $C_{20}$ heteroaryl with one, two or three aromatic six-member rings, substituted or unsubstituted $C_7$ to $C_{20}$ heteroaryl with two or three fused aromatic six-member rings or CN,

wherein the substituents are selected from CN, partially fluorinated or perfluorinated $C_1$ to $C_8$ alkyl, halogen, Cl, F, $C_1$ to $C_8$ alkyl or D;
wherein the "*" denotes the binding position;
wherein the compound of formula (I) is free of a five-member ring;
wherein the compound of formula (I) comprises 1 to 6, or 2 to 5, or 3 to 4, six-member ring, preferably the six-member rings are aromatic six-member rings; and

optional the following formula (E1) is excluded:

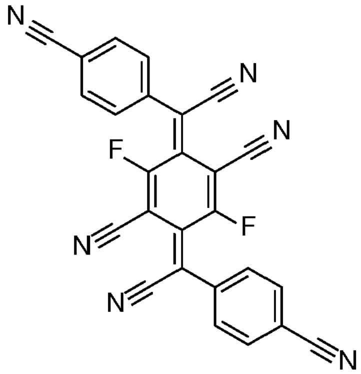
(E1).

According to one embodiment of the present invention, the compound of formula (I) is represented by:

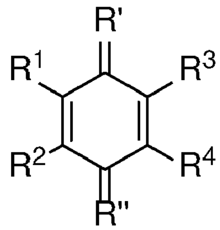
(I),

wherein

$R^1$, $R^2$, $R^3$ and $R^4$ are independently selected from the group comprising F, Cl, CN or $CF_3$;
R' is selected from formula (II)

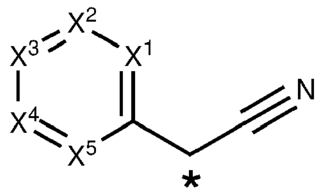
(II);

wherein

$X^1$ is selected from $CR^6$ or N;
$X^2$ is selected from $CR^7$ or N;
$X^3$ is selected from $CR^8$ or N;
$X^4$ is selected from $CR^9$ or N;
$X^5$ is selected from $CR^{10}$ or N;
wherein

$R^6$, $R^7$, $R^8$, $R^9$ and $R^{10}$ are independently selected from CN, partially fluorinated or perfluorinated $C_1$ to $C_8$ alkyl, halogen, Cl, F, D or H,

wherein at least one $X^1$ to $X^5$ is selected from CH, CD or N; and
wherein at most 3 or 4 of $X^1$ to $X^5$ is selected from F;
R" is selected from formula (III)

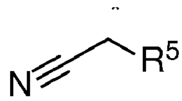

(III);

wherein

$R^5$ is selected from substituted or unsubstituted $C_6$ to $C_{19}$ aryl, substituted or unsubstituted $C_6$ to $C_{19}$ aryl with one, two or three aromatic six-member rings, substituted or unsubstituted $C_9$ to $C_{19}$ aryl with two or three fused aromatic six-member rings, substituted or unsubstituted $C_6$ to $C_{12}$ aryl with one or two aromatic six-member rings, substituted or unsubstituted phenyl, substituted or unsubstituted biphenyl, substituted or unsubstituted $C_3$ to $C_{20}$ heteroaryl, substituted or unsubstituted $C_3$ to $C_{20}$ heteroaryl with one, two or three aromatic six-member rings, substituted or unsubstituted $C_7$ to $C_{20}$ heteroaryl with two or three fused aromatic six-member rings or CN,

wherein the substituents are selected from CN, partially fluorinated or perfluorinated $C_1$ to $C_8$ alkyl, halogen, Cl, F, $C_1$ to $C_8$ alkyl or D;
wherein the "*" denotes the binding position;
optional the compound of formula (I) is free of a five-member ring;
optional the compound of formula (I) comprises 1 to 6, or 2 to 5, or 3 to 4 six-member ring, preferably the six-member rings are aromatic six-member rings; and
optional the following formula (E1) is excluded:

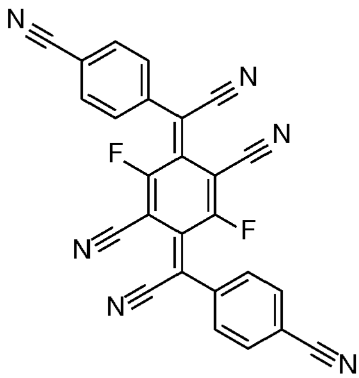

(E1).

According to one embodiment of the present invention, the compound of formula (I) is represented by:

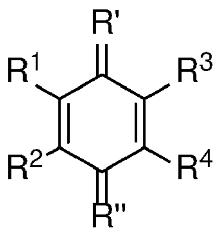

(I),

wherein

$R^1$, $R^2$, $R^3$ and $R^4$ are independently selected from the group comprising F, Cl, CN or $CF_3$;
R' is selected from formula (II)

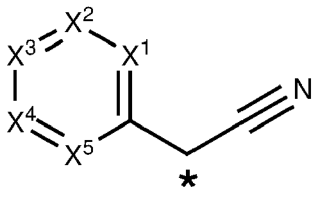

(II);

wherein

$X^1$ is selected from $CR^6$ or N;
$X^2$ is selected from $CR^7$ or N;
$X^3$ is selected from $CR^8$ or N;
$X^4$ is selected from $CR^9$ or N;
$X^5$ is selected from $CR^{10}$ or N;
wherein

$R^6$, $R^7$, $R^8$, $R^9$ and $R^{10}$ are independently selected from CN, partially fluorinated or perfluorinated $C_1$ to $C_8$ alkyl, halogen, Cl, F, D or H,

wherein at least one $X^1$ to $X^5$ is selected from CH, CD or N; and
wherein at most 3 or 4 of $X^1$ to $X^5$ is selected from F and/or at most 2 of $R^1$, $R^2$, $R^3$ and $R^4$ is selected from CN;
R" is selected from formula (III)

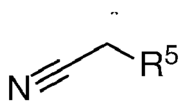

(III);

wherein

$R^5$ is selected from substituted or unsubstituted $C_6$ to $C_{19}$ aryl, substituted or unsubstituted $C_6$ to $C_{19}$ aryl with one, two or three aromatic six-member rings, substituted or unsubstituted $C_9$ to $C_{19}$ aryl with two or three fused aromatic six-member rings, substituted or unsubstituted $C_6$ to $C_{12}$ aryl with one or two aromatic six-member rings, substituted or unsubstituted phenyl, substituted or unsubstituted biphenyl, substituted or unsubstituted $C_3$ to $C_{20}$ heteroaryl, substituted or unsubstituted $C_3$ to $C_{20}$ heteroaryl with one, two or three aromatic six-member rings, substituted or unsubstituted $C_7$ to $C_{20}$ heteroaryl with two or three fused aromatic six-member rings or CN,

wherein the substituents are selected from CN, partially fluorinated or perfluorinated $C_1$ to $C_8$ alkyl, halogen, Cl, F, $C_1$ to $C_8$ alkyl or D;
wherein the "*" denotes the binding position;
optional the compound of formula (I) is free of a five-member ring;
optional the compound of formula (I) comprises 1 to 6, or 2 to 5, or 3 to 4 six-member ring, preferably the six-member rings are aromatic six-member rings; and
optional the following formula (E1) is excluded:

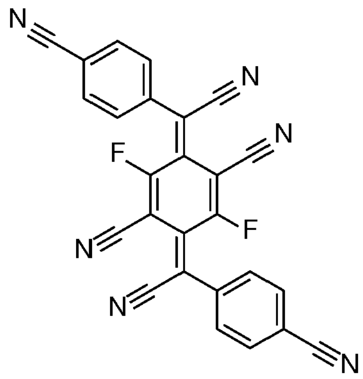

(E1).

According to one embodiment the chemical structure of R' and R" are identical. Alternatively, according to one embodiment the chemical structure of R' and R" are not identical.

**Compound of formula (Ia), (Ib), (Ic) and (Id)**

According to one embodiment of the present invention, the compound of formula (I) is represented formula (Ia), (Ib), (Ic) and (Id):

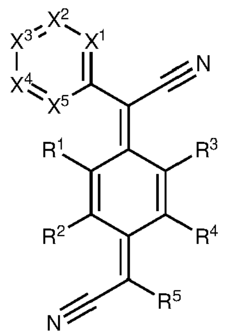

(Ia),

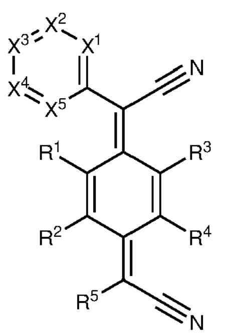

(Ib),

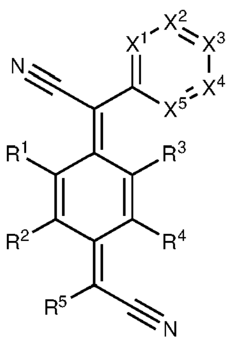

(Ic),

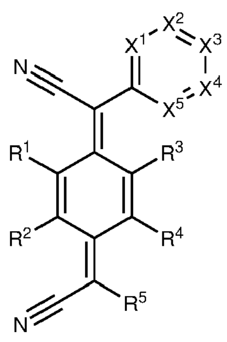

(Id),

wherein

$R^1$ to $R^4$ are independently selected from F, Cl, CN or $CF_3$;

$R^5$ is selected from substituted or unsubstituted $C_6$ to $C_{19}$ aryl, substituted or unsubstituted $C_6$ to $C_{19}$ aryl with one, two or three aromatic six-member rings, substituted or unsubstituted $C_9$ to $C_{19}$ aryl with two or three fused aromatic six-member rings, substituted or unsubstituted $C_6$ to $C_{12}$ aryl with one or two aromatic six-member rings, substituted or unsubstituted phenyl, substituted or unsubstituted biphenyl, substituted or unsubstituted $C_3$ to $C_{20}$ heteroaryl, substituted or unsubstituted $C_3$ to $C_{20}$ heteroaryl with one, two or three aromatic six-member rings, substituted or unsubstituted $C_7$ to $C_{20}$ heteroaryl with two or three fused aromatic six-member rings or CN, wherein

the substituent on $R^5$ is selected from CN, partially fluorinated or perfluorinated $C_1$ to $C_8$ alkyl, halogen, Cl, F, $C_1$ to $C_8$ alkyl or D;

$X^1$ is selected from $CR^6$ or N;
$X^2$ is selected from $CR^7$ or N;
$X^3$ is selected from $CR^8$ or N;
$X^4$ is selected from $CR^9$ or N;
$X^5$ is selected from $CR^{10}$ or N; wherein

$R^6$, $R^7$, $R^8$, $R^9$ and $R^{10}$ are independently selected from CN, partially fluorinated or perfluorinated $C_1$ to $C_8$ alkyl, halogen, Cl, F, D or H,

wherein at least one $X^1$ to $X^5$ is selected from CH, CD or N; and the following formula (E1) is optional excluded:

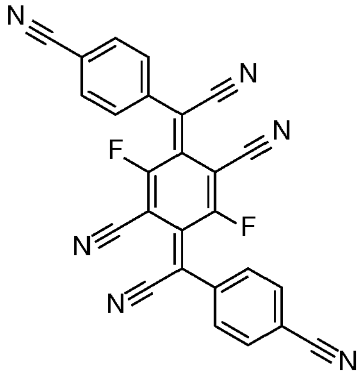
(E1).

According to one embodiment, wherein the compound of formula (I) is represented by formula (Ia), (Ib), (Ic) and (Id):

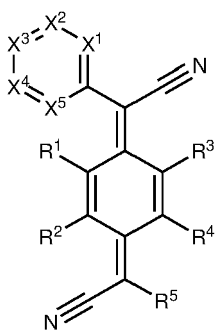
(Ia),
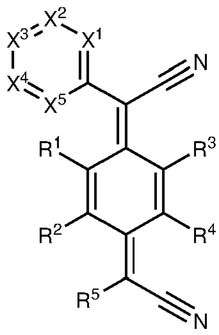
(Ib),
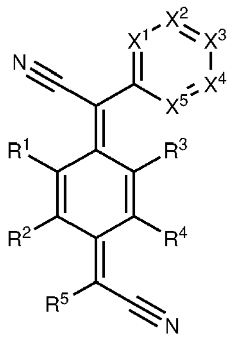
(Ic),
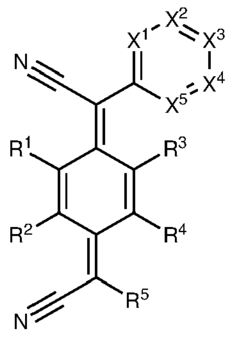
(Id),

wherein

| | |
|---|---|
| $R^1$ to $R^4$ | are independently selected from F, Cl, CN or $CF_3$; |
| $R^5$ | is selected from substituted or unsubstituted $C_6$ to $C_{19}$ aryl, substituted or unsubstituted $C_6$ to $C_{19}$ aryl with one, two or three aromatic six-member rings, substituted or unsubstituted $C_9$ to $C_{19}$ aryl with two or three fused aromatic six-member rings, substituted or unsubstituted $C_6$ to $C_{12}$ aryl with one or two aromatic six-member rings, substituted or unsubstituted phenyl, substituted or unsubstituted biphenyl, substituted or unsubstituted $C_3$ to $C_{20}$ heteroaryl, substituted or unsubstituted $C_3$ to $C_{20}$ heteroaryl with one, two or three aromatic six-member rings, substituted or unsubstituted $C_7$ to $C_{20}$ heteroaryl with two or three fused aromatic six-member rings or CN, wherein |

the substituent on $R^5$ is selected from CN, partially fluorinated or perfluorinated $C_1$ to $C_8$ alkyl, halogen, Cl, F, $C_1$ to $C_8$ alkyl or D;

$X^1$ is selected from $CR^6$ or N;
$X^2$ is selected from $CR^7$ or N;
$X^3$ is selected from $CR^8$ or N;
$X^4$ is selected from $CR^9$ or N;
$X^5$ is selected from $CR^{10}$ or N;
wherein

$R^6$, $R^7$, $R^8$, $R^9$ and $R^{10}$ are independently selected from CN, F, $CF_3$, and at least one $X^1$ to $X^5$ is selected from CH, CD or N; and

the following formula (E1) is optional excluded:

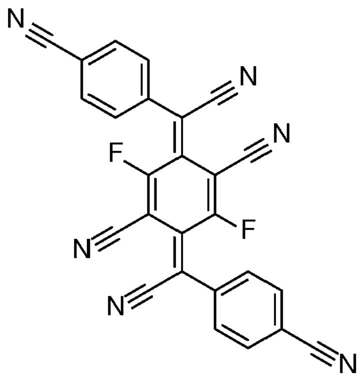

(E1).

**$R^1$, $R^2$, $R^3$ and $R^4$ of Compound of formula (I), (Ia), (Ib), (Ic) and (Id)**

According to one embodiment of Formula (I), (Ia), (Ib), (Ic) and/or (Id), wherein $R^1$, $R^2$, $R^3$ and $R^4$ are independently selected from F or CN, preferably two of $R^1$, $R^2$, $R^3$ and $R^4$ are selected from F or CN and the other two $R^1$, $R^2$, $R^3$ and $R^4$ are selected from F.

According to one embodiment of Formula (I), (Ia), (Ib), (Ic) and/or (Id), wherein $R^1$, $R^2$, $R^3$ and $R^4$ are independently selected from F or CN, preferably two of $R^1$, $R^2$, $R^3$ and $R^4$ are selected from F and the other two of $R^1$, $R^2$, $R^3$ and $R^4$ are selected from CN.

According to one embodiment of Formula (I), (Ia), (Ib), (Ic) and/or (Id), wherein $R^1$, $R^2$, $R^3$ and $R^4$ are selected from F.

According to one embodiment of Formula (I), (Ia), (Ib), (Ic) and/or (Id), wherein $R^1$, $R^2$, $R^3$ and $R^4$ are independently selected from F or CN, preferably two of $R^1$, $R^2$, $R^3$ and $R^4$ are selected from F and the other two of $R^1$, $R^2$, $R^3$ and $R^4$ are selected from CN; or $R^1$, $R^2$, $R^3$ and $R^4$ are selected from F.

According to one embodiment of Formula (I), (Ia), (Ib), (Ic) and/or (Id), wherein $R^1$, $R^2$, $R^3$ and $R^4$ are independently selected from $CF_3$ or CN, preferably two of $R^1$, $R^2$, $R^3$ and $R^4$ are selected from F and the other two of $R^1$, $R^2$, $R^3$ and $R^4$ are selected from CN.

According to one embodiment of Formula (I), (Ia), (Ib), (Ic) and/or (Id), wherein $R^1$, $R^2$, $R^3$ and $R^4$ are selected from $CF_3$.

According to one embodiment of Formula (I), (Ia), (Ib), (Ic) and/or (Id), wherein $R^1$, $R^2$, $R^3$ and $R^4$ are independently selected from F or CN, preferably two of $R^1$, $R^2$, $R^3$ and $R^4$ are selected from F and the other two of $R^1$, $R^2$, $R^3$ and $R^4$ are selected from CN; or $R^1$, $R^2$, $R^3$ and $R^4$ are selected from F; or two of $R^1$, $R^2$, $R^3$ and $R^4$ are selected from $CF_3$ and the other two of $R^1$, $R^2$, $R^3$ and $R^4$ are selected from CN.

According to one embodiment of Formula (I), (Ia), (Ib), (Ic) and/or (Id), wherein $R^1$, $R^2$, $R^3$ and $R^4$ are independently selected from F or CN and at least one or two of $R^1$, $R^2$, $R^3$ and $R^4$ are selected from $CF_3$.

According to one embodiment of Formula (I), (Ia), (Ib), (Ic) and/or (Id), wherein $R^1$ and $R^4$ are selected from F and $R^2$ and $R^3$ are selected from F or CN; or $R^1$ and $R^4$ are selected from F and $R^2$ and $R^3$ are selected from CN; or $R^1$ and $R^4$ are selected from F and $R^2$ and $R^3$ are selected from F; or $R^1$ and $R^4$ are selected from F or CN and $R^2$ and $R^3$ are selected from F; or $R^1$ and $R^4$ are selected from CN and $R^2$ and $R^3$ are selected from F.

According to one embodiment of Formula (I), (Ia), (Ib), (Ic) and/or (Id), wherein $R^1$ and $R^4$ are selected from $CF_3$ and $R^2$ and $R^3$ are selected from $CF_3$ or CN; or $R^1$ and $R^4$ are selected from $CF_3$ and $R^2$ and $R^3$ are selected from CN; or $R^1$ and $R^4$ are selected from $CF_3$ and $R^2$ and $R^3$ are selected from $CF_3$; or $R^1$ and $R^4$ are selected from $CF_3$ or CN and $R^2$ and $R^3$ are selected from $CF_3$; or $R^1$ and $R^4$ are selected from CN and $R^2$ and $R^3$ are selected from $CF_3$.

**$X^1$, $X^2$, $X^3$, $X^4$ and $X^5$ of Compound of formula (I), (Ia), (Ib), (Ic) and (Id)**

According to one embodiment of Formula (I), (Ia), (Ib), (Ic) and/or (Id), wherein $\geq 0$ and $\leq 3$ of $X^1$, $X^2$, $X^3$, $X^4$ and $X^5$ are selected from N.

According to one embodiment of Formula (I), (Ia), (Ib), (Ic) and/or (Id), wherein at least one or at most two of $X^1$, $X^2$, $X^3$, $X^4$ and $X^5$ are selected from N.

According to one embodiment a total of 0, 1, or 2 of $X^1$, $X^2$, $X^3$, $X^4$ and $X^5$ are N.

According to one embodiment of Formula (I), (Ia), (Ib), (Ic) and/or (Id), wherein at least two $X^1$ to $X^5$ are selected from CH, CD or N.

According to one embodiment of Formula (I), (Ia), (Ib), (Ic) and/or (Id), wherein at least 3 to 5 of $X^1$ to $X^5$ are selected from CH, CD or N.

According to one embodiment of Formula (I), (Ia), (Ib), (Ic) and/or (Id), wherein at least four of $X^1$ to $X^5$ are selected from CH, CD or N.

According to one embodiment of Formula (I), (Ia), (Ib), (Ic) and/or (Id), wherein at least five of $X^1$ to $X^5$ are selected from CH, CD or N.

According to one embodiment of Formula (I), (Ia), (Ib), (Ic) and/or (Id), wherein $X^1$ to $X^5$ may be selected as shown in Table 1 below.

Table 1

| $X^1$ | $X^2$ | $X^3$ | $X^4$ | $X^5$ |
|---|---|---|---|---|
| $CR^6$ | $CR^7$ | $CR^8$ | $CR^9$ | $CR^{10}$ |
| $CR^6$ | $CR^7$ | N | $CR^9$ | $CR^{10}$ |
| $CR^6$ | N | $CR^8$ | $CR^9$ | $CR^{10}$ |
| $CR^6$ | $CR^7$ | $CR^8$ | N | $CR^{10}$ |
| N | $CR^7$ | $CR^8$ | $CR^9$ | $CR^{10}$ |
| $CR^6$ | $CR^7$ | $CR^8$ | $CR^9$ | N |
| $CR^6$ | N | $CR^8$ | N | $CR^{10}$ |
| N | $CR^7$ | $CR^8$ | $CR^9$ | N |
| N | $CR^7$ | N | $CR^9$ | $CR^{10}$ |
| N | $CR^7$ | $CR^8$ | N | $CR^{10}$ |
| N | $CR^7$ | N | $CR^9$ | N |

According to one embodiment of Formula (I), (Ia), (Ib), (Ic) and/or (Id), wherein $X^1$ to $X^5$ may be preferably selected as shown in Table 2 below.

Table 2

| $X^1$ | $X^2$ | $X^3$ | $X^4$ | $X^5$ |
|---|---|---|---|---|
| CH | $C-CF_3$ | CH | C-CN | CH |
| CH | $C-CF_3$ | CH | $C-CF_3$ | CH |
| CH | C-CN | CH | C-CN | CH |
| CH | C-CN | $C-CF_3$ | CH | CH |
| CH | $C-CF_3$ | C-CN | CH | CH |
| $C-CF_3$ | CH | C-CN | CH | CH |
| C-CN | CH | C-CN | CH | CH |
| $C-CF_3$ | CH | C-CN | CH | CH |
| $C-CF_3$ | CH | $C-CF_3$ | CH | CH |
| $C-CF_3$ | CH | $C-CF_3$ | CH | $C-CF_3$ |
| CH | C-CN | $C-CF_3$ | C-CN | CH |
| CH | C-CN | C-CN | $C-CF_3$ | CH |
| CH | $C-CF_3$ | $C-CF_3$ | C-CN | CH |
| CH | $C-CF_3$ | C-CN | $C-CF_3$ | CH |
| CH | C-CN | C-CN | C-CN | CH |
| $C-CF_3$ | CH | $C-CF_3$ | CH | $C-CF_3$ |
| $C-CF_3$ | CH | C-CN | CH | $C-CF_3$ |

(continued)

| $X^1$ | $X^2$ | $X^3$ | $X^4$ | $X^5$ |
|---|---|---|---|---|
| C-CN | CH | C-CN | CH | C-CN |
| C-$CF_3$ | CH | C-CN | C-$CF_3$ | CH |
| C-CN | CH | C-CN | CH | C-CN |
| C-$CF_3$ | CH | C-CN | CH | C-$CF_3$ |
| C-$CF_3$ | CH | C-CN | C-$CF_3$ | CH |
| C-$CF_3$ | CH | C-CN | CH | C-CN |
| C-$CF_3$ | CH | C-$CF_3$ | CH | C-CN |
| C-$CF_3$ | CH | C-CN | C-CN | CH |
| C-$CF_3$ | C-CN | C-$CF_3$ | CH | CH |
| C-$CF_3$ | C-$CF_3$ | C-CN | CH | CH |
| C-CN | C-$CF_3$ | C-CN | CH | CH |
| C-$CF_3$ | C-CN | C-CN | CH | CH |
| CH | CF | CF | CF | CF |
| CH | CF | C-CN | CF | CF |
| CH | CF | C-$CF_3$ | CF | CF |
| C-$CF_3$ | C-$CF_3$ | C-CN | C-CN | CH |
| C-CN | C-$CF_3$ | C-CN | C-$CF_3$ | CH |
| C-$CF_3$ | C-CN | C-$CF_3$ | C-CN | CH |
| C-$CF_3$ | C-CN | C-CN | C-$CF_3$ | CH |
| C-$CF_3$ | CH | C-CN | C-$CF_3$ | C-CN |
| C-$CF_3$ | CH | C-CN | C-CN | C-$CF_3$ |
| C-$CF_3$ | C-CN | CH | C-CN | C-$CF_3$ |
| C-$CF_3$ | C-CN | CH | C-$CF_3$ | C-CN |
| C-CN | C-$CF_3$ | CH | C-$CF_3$ | C-CN |
| C-$CF_3$ | C-$CF_3$ | C-CN | CH | C-CN |
| C-$CF_3$ | C-$CF_3$ | C-CN | CH | C-$CF_3$ |
| C-CN | C-CN | CH | C-CN | C-$CF_3$ |
| C-$CF_3$ | C-CN | C-CN | CH | C-CN |
| CH | C-$CF_3$ | N | C-$CF_3$ | CH |
| CH | C-CN | N | C-CN | CH |
| CH | C-CN | N | C-$CF_3$ | CH |
| CH | N | C-$CF_3$ | CH | CH |
| CH | N | C-CN | CH | CH |
| CH | N | C-$CF_3$ | CH | CH |
| CH | N | C-$CF_3$ | C-CN | CH |
| CH | N | C-CN | C-$CF_3$ | CH |
| CH | N | C-$CF_3$ | CH | C-$CF_3$ |
| C-$CF_3$ | N | C-$CF_3$ | CH | C-$CF_3$ |

(continued)

| $X^1$ | $X^2$ | $X^3$ | $X^4$ | $X^5$ |
|---|---|---|---|---|
| C-$CF_3$ | N | C-$CF_3$ | C-CN | C-$CF_3$ |
| CH | N | C-$CF_3$ | C-CN | CH |
| CH | N | C-$CF_3$ | CH | CN |
| CH | N | C-$CF_3$ | N | CH |
| C-$CF_3$ | N | C-$CF_3$ | N | C-$CF_3$ |
| C-$CF_3$ | N | C-$CF_3$ | N | CH |
| C-$CF_3$ | N | C-$CF_3$ | N | C-CN |
| C-$CF_3$ | N | C-CN | N | C-$CF_3$ |
| N | C-$CF_3$ | N | C-$CF_3$ | CH |
| N | C-$CF_3$ | N | CH | CH |
| N | C-$CF_3$ | N | CH | C-$CF_3$ |
| N | C-$CF_3$ | C-$CF_3$ | N | CF |
| N | C-$CF_3$ | C-$CF_3$ | N | CH |
| N | C-$CF_3$ | C-$CF_3$ | N | C-$CF_3$ |
| N | C-$CF_3$ | CH | C-$CF_3$ | N |
| N | C-$CF_3$ | N | C-$CF_3$ | N |

According to an embodiment of the compound of formula (I), wherein $R^5$ may be selected from formula (IV) or CN, preferably $R^5$ is CN.

**Formula (II) and/or Formula (III)**

According to one embodiment, wherein formula (II) and/or formula (III) are independently selected from the group comprising of A1 to A142:

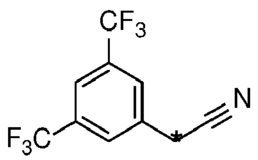
(A1),

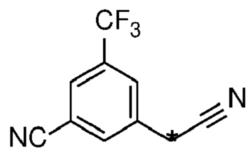
(A2),

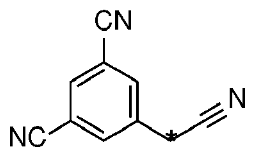
(A3),

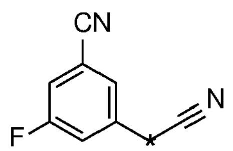
(A4),

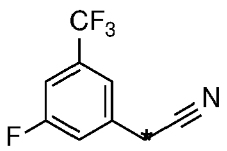
(A5),

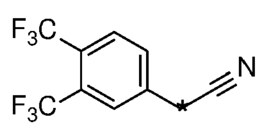
(A6),

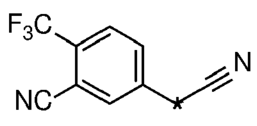
(A7),

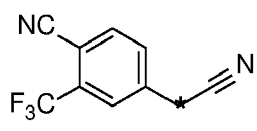
(A8),

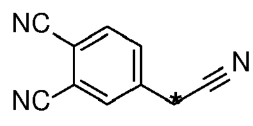
(A9),

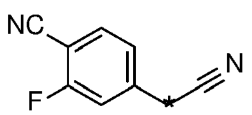
(A10),
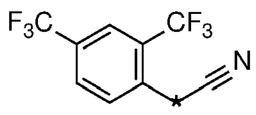
(A11),
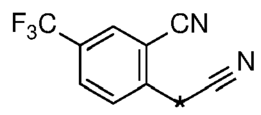
(A12),

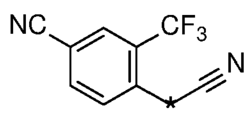
(A13),
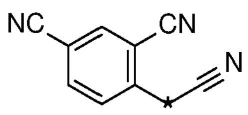
(A14),
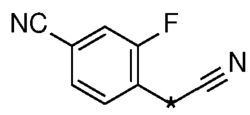
(A15),

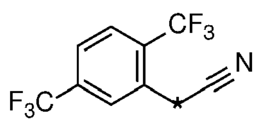
(A16),
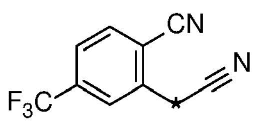
(A17),
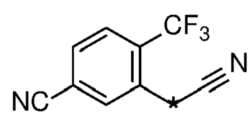
(A18),

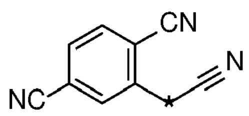
(A19),
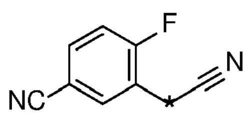
(A20),
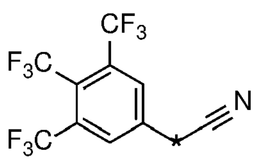
(A21),

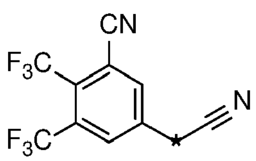
(A22),
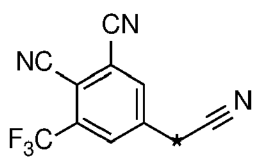
(A23),
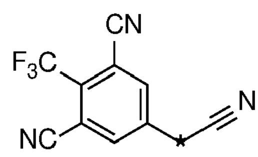
(A24),

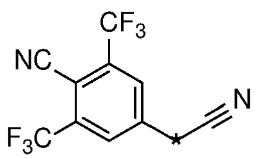
(A25),
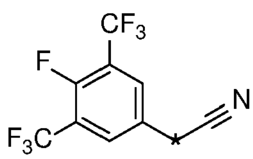
(A26),
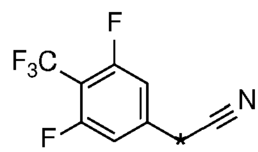
(A27),

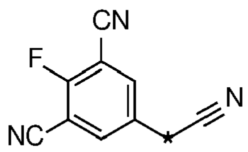
(A28),
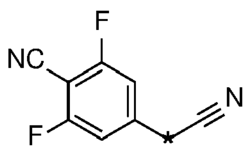
(A29),
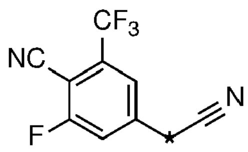
(A30),

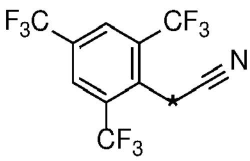 (A31), 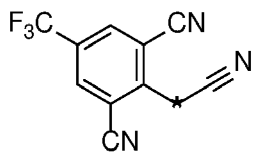 (A32), 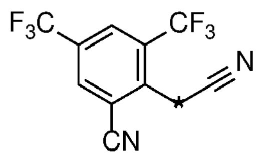 (A33),

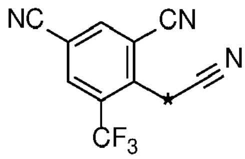 (A34), 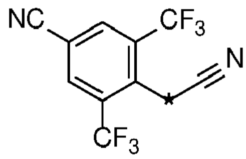 (A35), 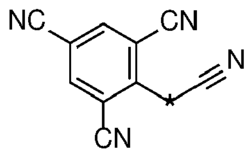 (A36),

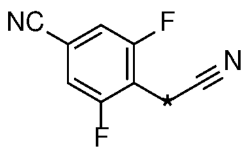 (A37), 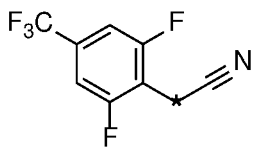 (A38), 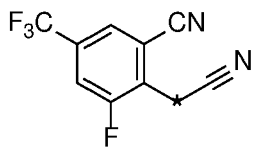 (A39),

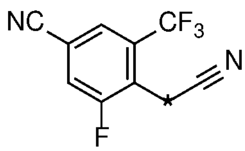 (A40), 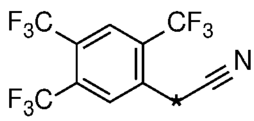 (A41), 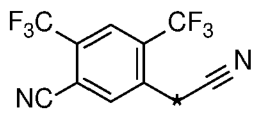 (A42),

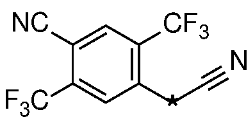 (A43), 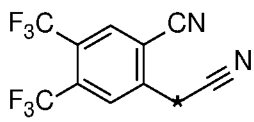 (A44), 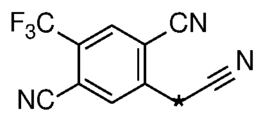 (A45),

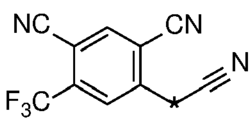 (A46), 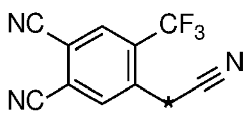 (A47), 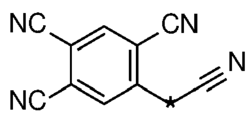 (A48),

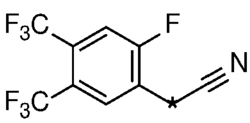 (A49), 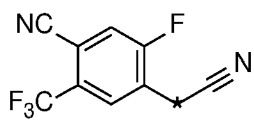 (A50), 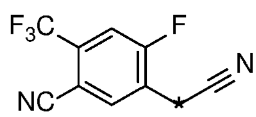 (A51),

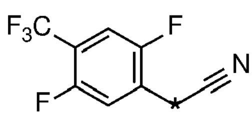 (A52), 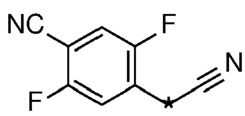 (A53), 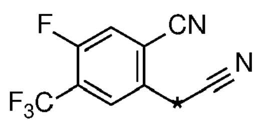 (A54),

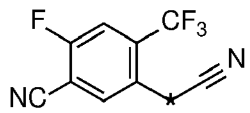
(A55),
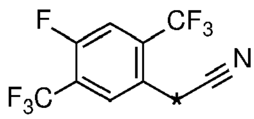
(A56),
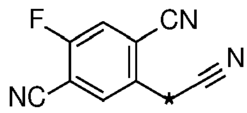
(A57),

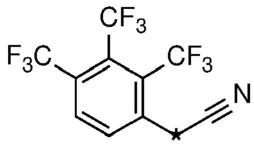
(A58),
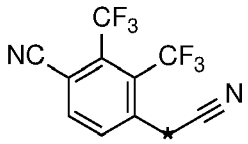
(A59),
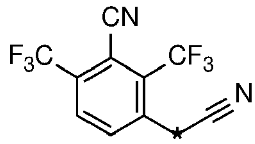
(A60),

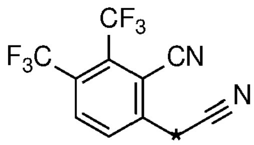
(A61),
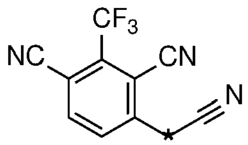
(A62),
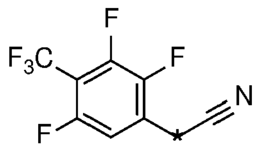
(A63),

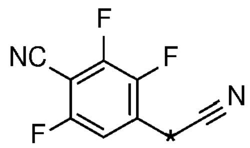
(A64),
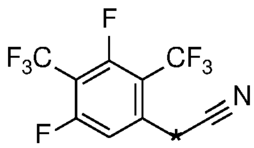
(A65),
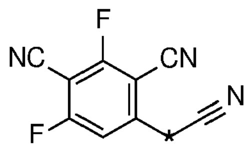
(A66),

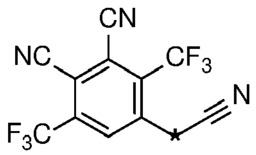
(A67),
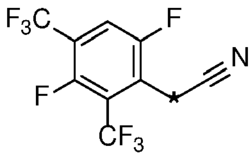
(A68),
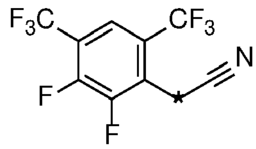
(A69),

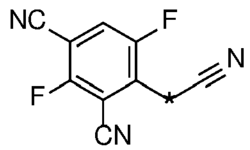
(A70),
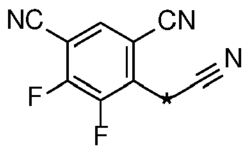
(A71),
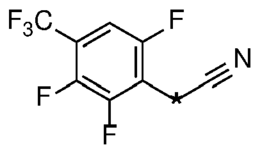
(A72),

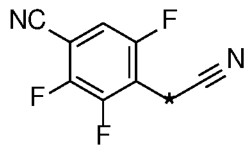
(A73),
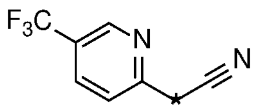
(A74),
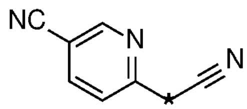
(A75),

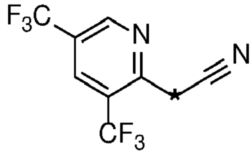 (A76), 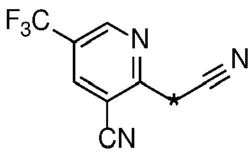 (A77), 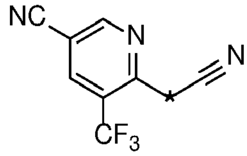 (A78),

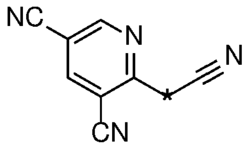 (A79), 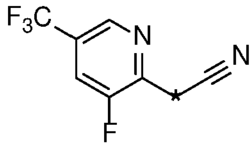 (A80), 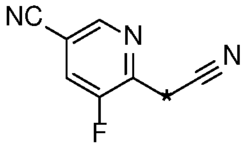 (A81),

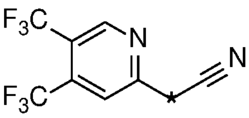 (A82), 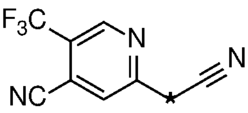 (A83), 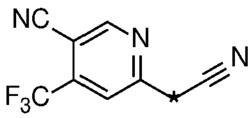 (A84),

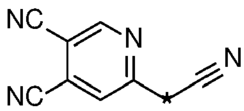 (A85), 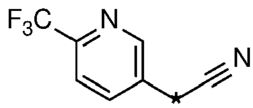 (A86), 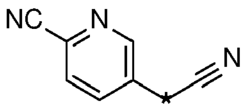 (A87),

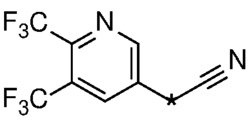 (A88), 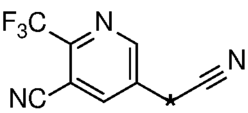 (A89), 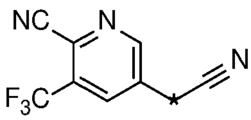 (A90),

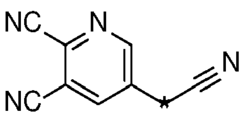 (A91), 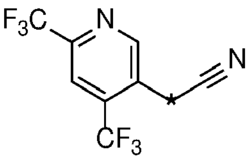 (A92), 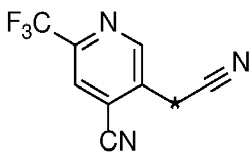 (A93),

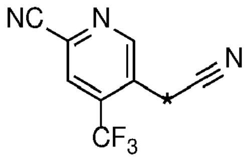 (A94), 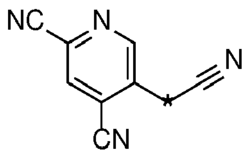 (A95), 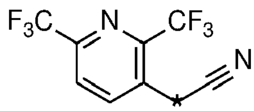 (A96),

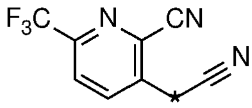 (A97), 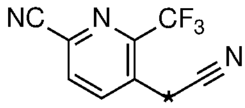 (A98), 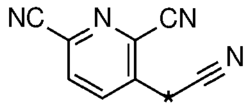 (A99),

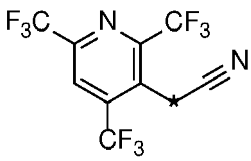 (A100), 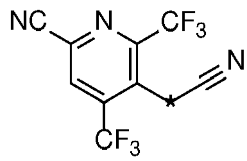 (A101), 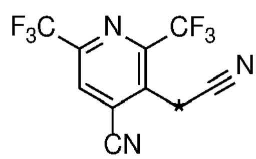 (A102),

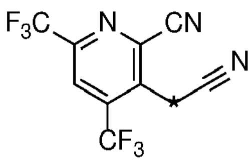 (A103), 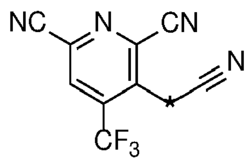 (A104), 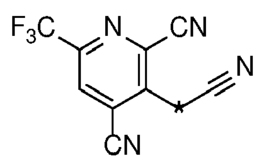 (A105),

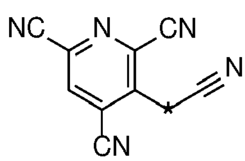 (A106), 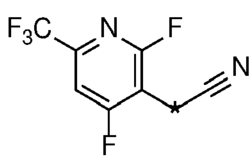 (A107), 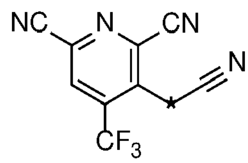 (A108),

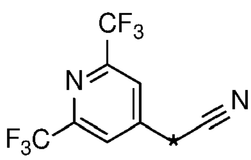 (A109), 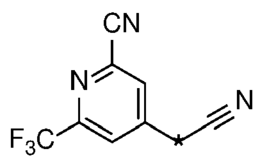 (A110), 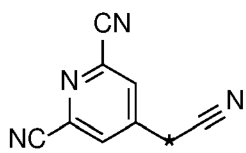 (A111),

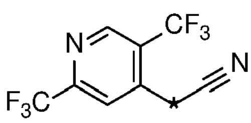 (A112), 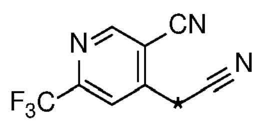 (A113), 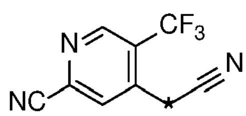 (A114),

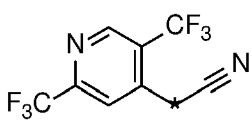 (A115), 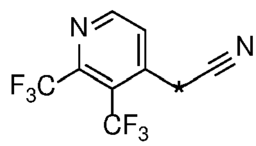 (A116), 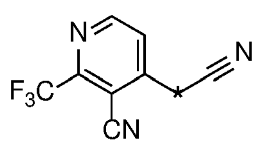 (A117),

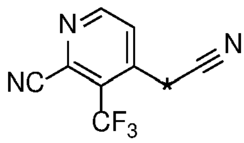 (A118), 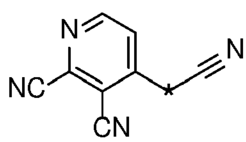 (A119), 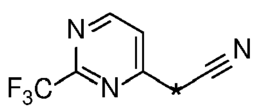 (A120),

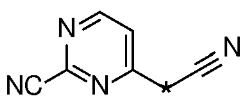 (A121), 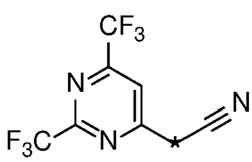 (A122), 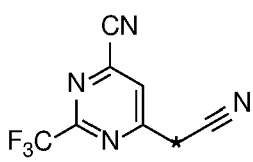 (A123),

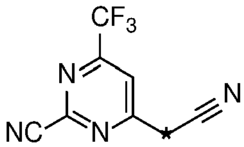 (A124), 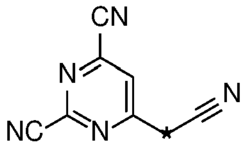 (A125), 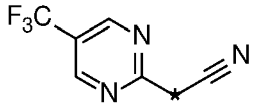 (A126),

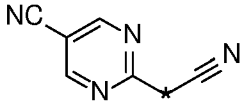 (A127), 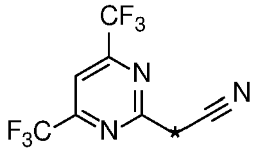 (A128), 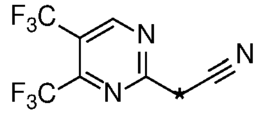 (A129),

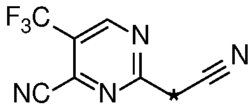 (A130), 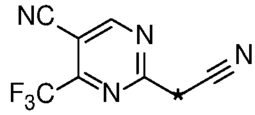 (A131), 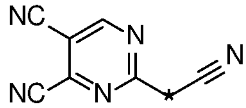 (A132),

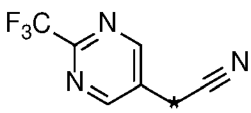 (A133), 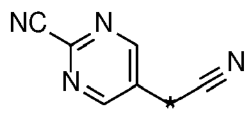 (A134), 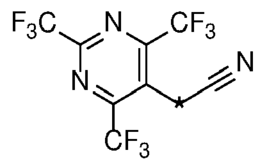 (A135),

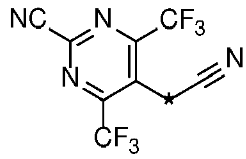 (A136), 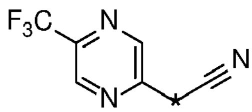 (A137), 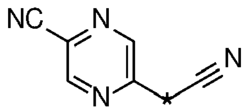 (A138),

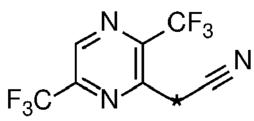 (A139), 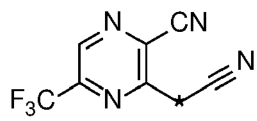 (A140), 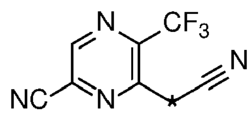 (A141),

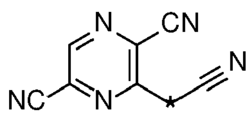 (A142);

wherein the "*" denotes the binding position.

According to one embodiment formula (II) and/or formula (III) are independently selected from the group comprising of A6 to A142, preferably A21 to A142.

According to one embodiment formula (II) and formula (III) are selected different.

According to one embodiment formula (II) and formula (III) are selected the same.

**Formula (III)**

According to an embodiment, the compound of formula (I), wherein formula (III) is selected from the group comprising of A1 to A143, wherein A1 to A142 are defined above and formula A143 has the following formula:

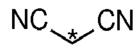

(A143)

; wherein the "*" denotes the binding position.

**Formulae (IVa) or (IVb)**

According to one embodiment wherein $R^5$ has the formulae (IVa) or (IVb), formula (IVa) is represented by:

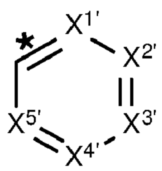

(IVa),

wherein

$X^{1'}$ is selected from $CR^{6'}$ or N;
$X^{2'}$ is selected from $CR^{7'}$ or N;
$X^{3'}$ is selected from $CR^{8'}$ or N;
$X^{4'}$ is selected from $CR^{9'}$ or N;
$X^{5'}$ is selected from $CR^{10'}$ or N;
wherein

$R^{6'}$, $R^{7'}$, $R^{8'}$, $R^{9'}$ and $R^{10'}$ are independently selected from CN, partially fluorinated or perfluorinated $C_1$ to $C_8$ alkyl, halogen, Cl, F, D, H, substituted or unsubstituted $C_6$ to $C_{13}$ aryl or substituted or unsubstituted $C_3$ to $C_{14}$ heteroaryl, preferably CN, F, $CF_3$, substituted or unsubstituted $C_6$ aryl or substituted or unsubstituted $C_3$ to $C_5$ heteroaryl;

wherein the substituent on substituted $C_6$ to $C_{13}$ aryl, substituted $C_3$ to $C_{14}$ heteroaryl, substituted or unsubstituted C6 aryl, substituted or unsubstituted $C_3$ to $C_5$ heteroaryl is selected from CN, partially fluorinated or perfluorinated $C_1$ to $C_8$ alkyl, halogen, Cl, F, $C_1$ to $C_8$ alkyl or D, preferably CN, F, $CF_3$;
or
formula (IVb) is represented by:

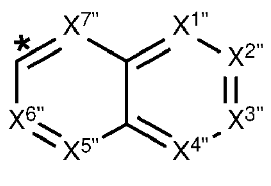

(IVb),

wherein

$X^{1''}$ is selected from $CR^{6''}$ or N;
$X^{2''}$ is selected from $CR^{7''}$ or N;
$X^{3''}$ is selected from $CR^{8''}$ or N;
$X^{4''}$ is selected from $CR^{9''}$ or N;
$X^{5''}$ is selected from $CR^{10''}$ or N;
$X^{6''}$ is selected from $CR^{11''}$ or N;

$X^{7"}$ is selected from $CR^{12"}$ or N; wherein

$R^{6"}$, $R^{7"}$, $R^{8"}$, $R^{9"}$, $R^{10"}$, $R^{11"}$ and $R^{12"}$ are independently selected from CN, partially fluorinated or perfluorinated $C_1$ to $C_8$ alkyl, halogen, Cl, F, D or H, preferably CN, F or $CF_3$;

wherein the "*" denotes the binding position.

According to one embodiment wherein formula (IVa) is selected from the group comprising of D1 to D170:

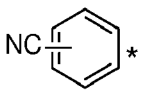 (D1), 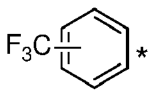 (D2), 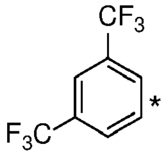 (D3), 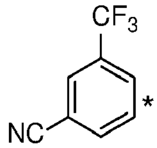 (D4), 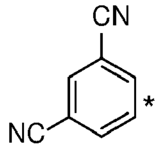 (D5),

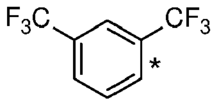 (D6), 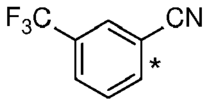 (D7), 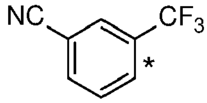 (D8), 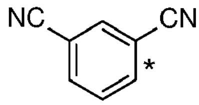 (D9),

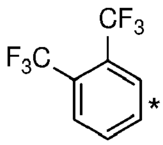 (D10), 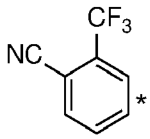 (D11), 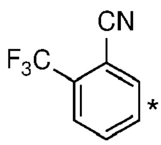 (D12), 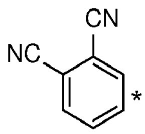 (D13), 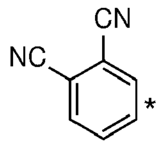 (D14),

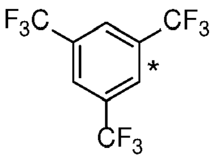 (D15), 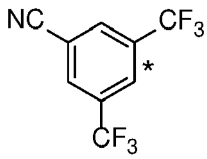 (D16), 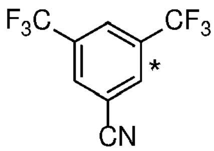 (D17), 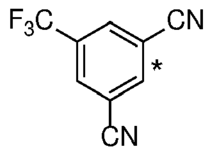 (D18),

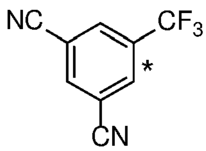 (D19), 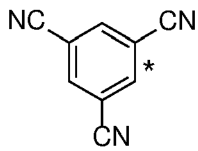 (D20), 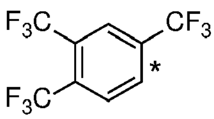 (D21), 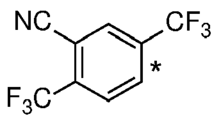 (D22),

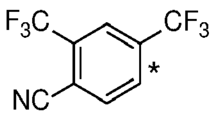 (D23), 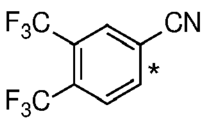 (D24), 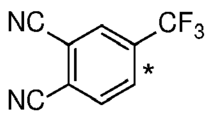 (D25), 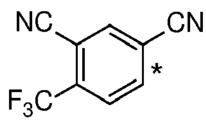 (D26),

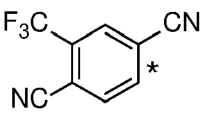 (D27), 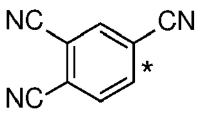 (D28), 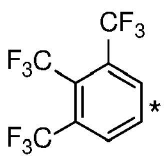 (D29), 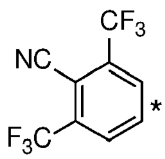 (D30),

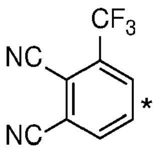 (D31), 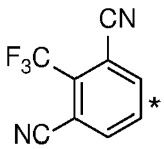 (D32), 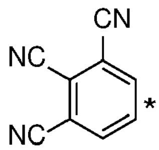 (D33), 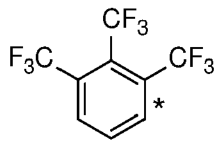 (D34),

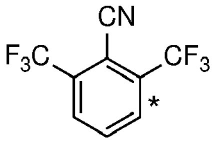 (D35), 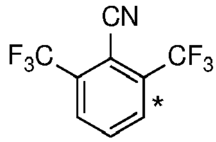 (D36), 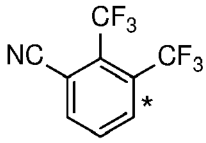 (D37), 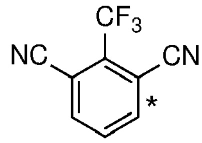 (D38),

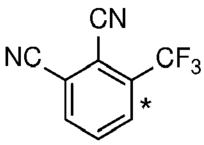 (D39), 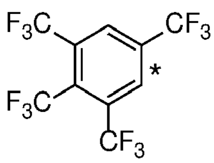 (D40), 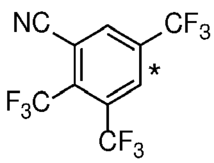 (D41), 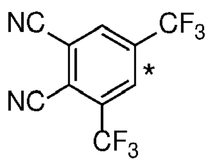 (D42),

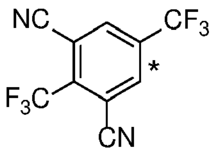 (D43), 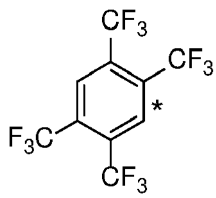 (D44), 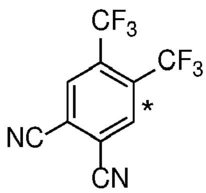 (D45), 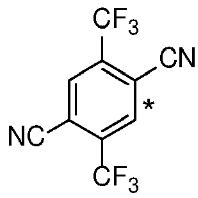 (D46),

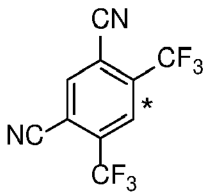 (D47), 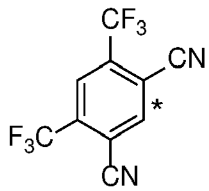 (D48), 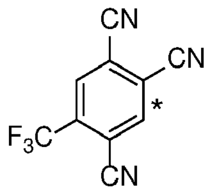 (D49), 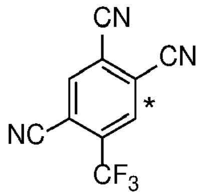 (D50),

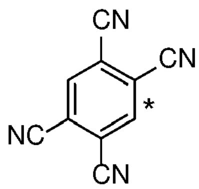 (D51), 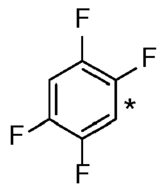 (D52), 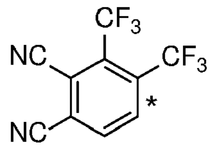 (D53), 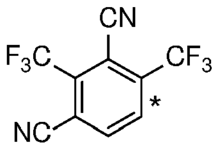 (D54),

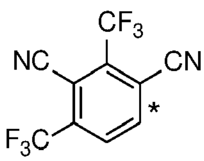 (D55), 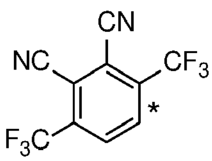 (D56), 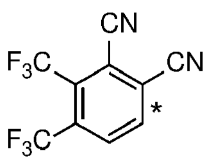 (D57), 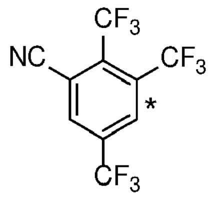 (D58),

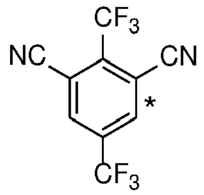 (D59), 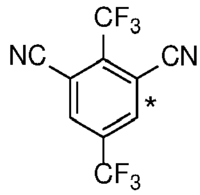 (D60), 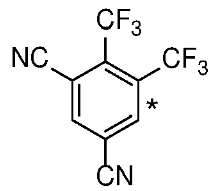 (D61), 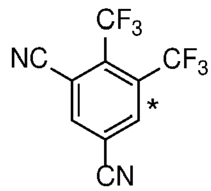 (D62),

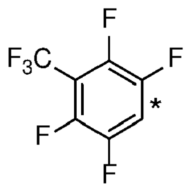 (D63), 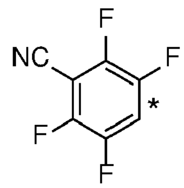 (D64), 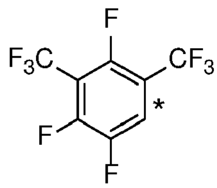 (D65), 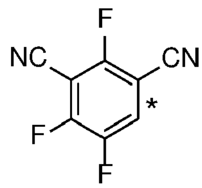 (D66),

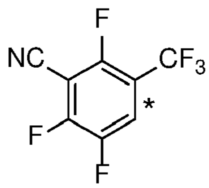 (D67), 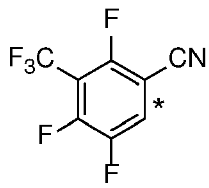 (D68), 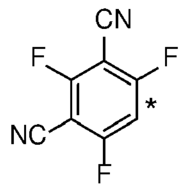 (D69), 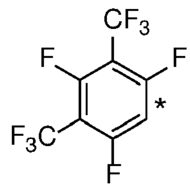 (D70),

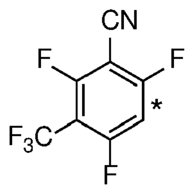 (D71), 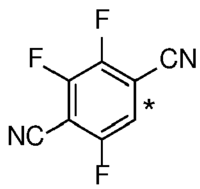 (D72), 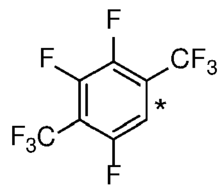 (D73), 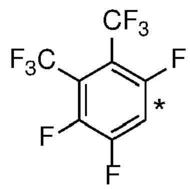 (D74),

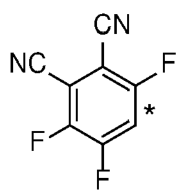 (D75), 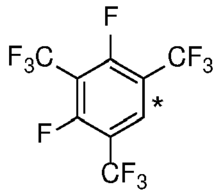 (D76), 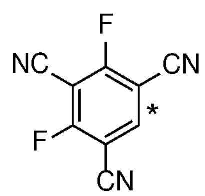 (D77), 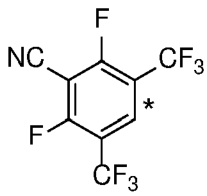 (D78),

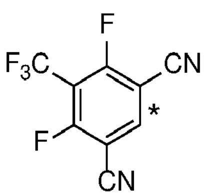 (D79), 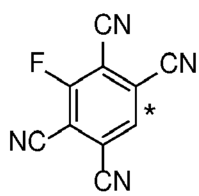 (D80), 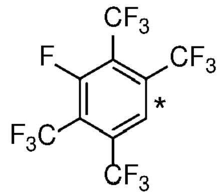 (D81), 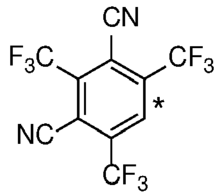 (D82),

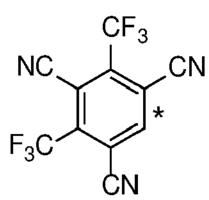 (D83), 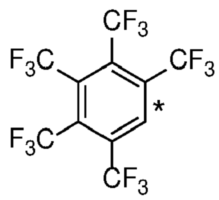 (D84), 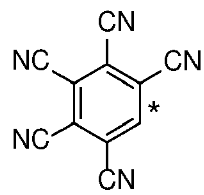 (D85), 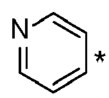 (D86), 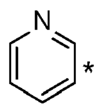

(D87), 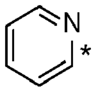 (D88), 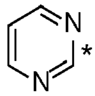 (D89), 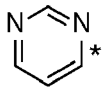 (D90), 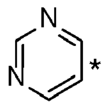 (D91), 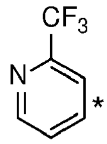 (D92),

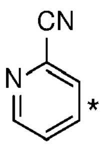 (D93), 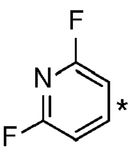 (D94), 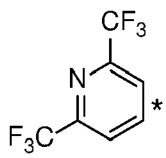 (D95), 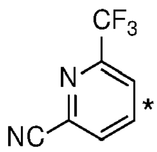 (D96), 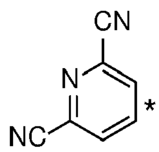 (D97),

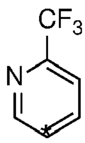 (D98), 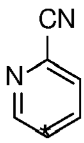 (D99), 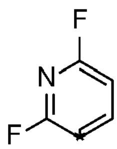 (D100), 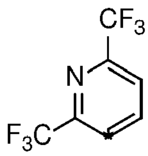 (D101), 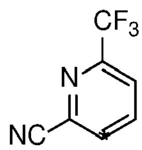 (D102),

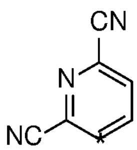 (D103), 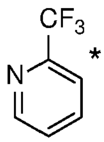 (D104), 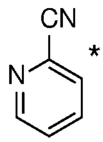 (D105), 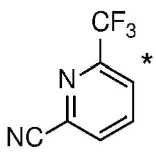 (D106), 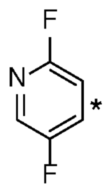 (D107),

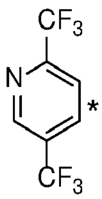 (D108), 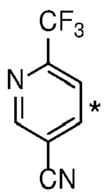 (D109), 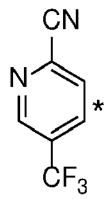 (D110), 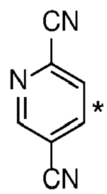 (D111), 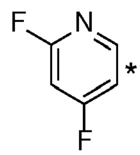 (D112),

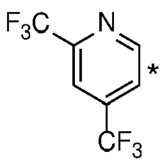 (D113), 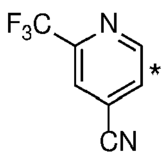 (D114), 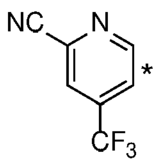 (D115), 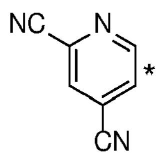 (D116),

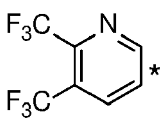 (D117), 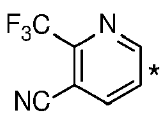 (D118), 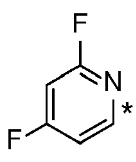 (D119), 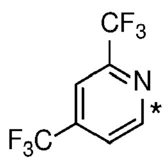 (D120),

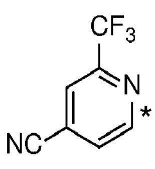 (D121), 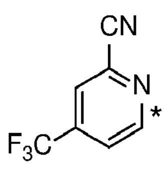 (D122), 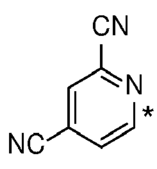 (D123), 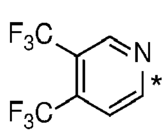 (D124),

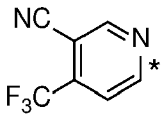 (D125), 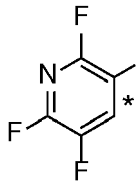 (D126), 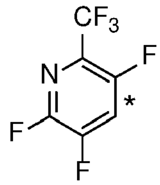 (D127), 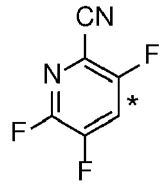 (D128),

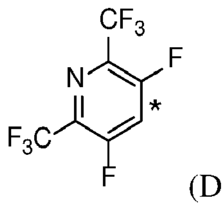 (D129), 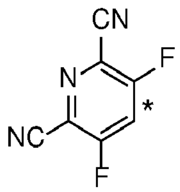 (D130), 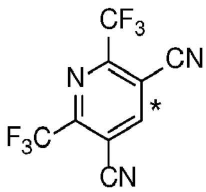 (D131), 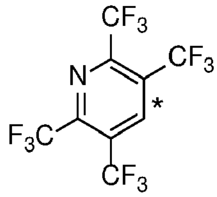 (D132),

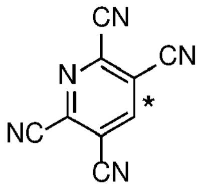 (D133), 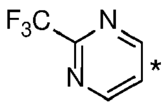 (D134), 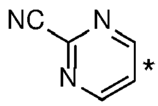 (D135), 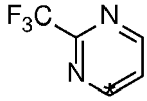 (D136),

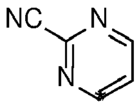 (D137), 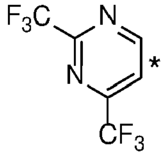 (D138), 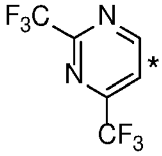 (D139), 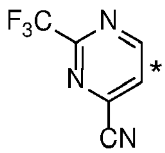 (D140),

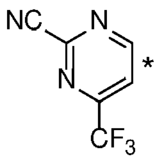 (D141), 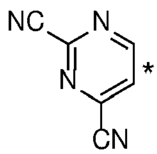 (D142), 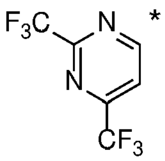 (D143), 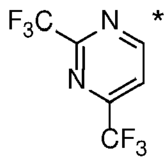 (D144),

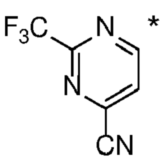 (D145), 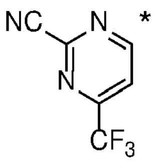 (D146), 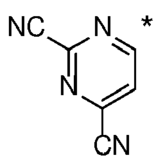 (D147), 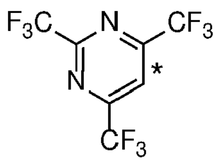 (D148),

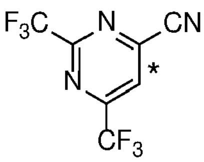 (D149), 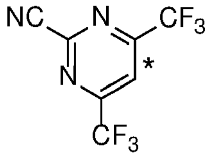 (D150), 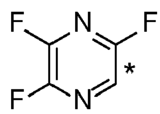 (D151), 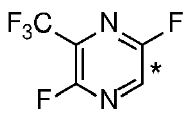 (D152),

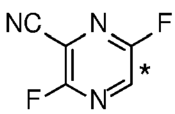 (D153), 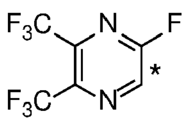 (D154), 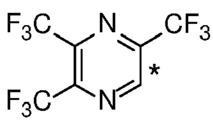 (D155), 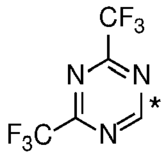 (D156),

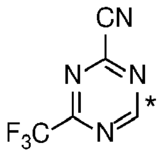 (D157), 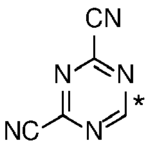 (D158), 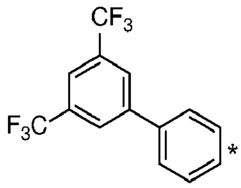 (D159), 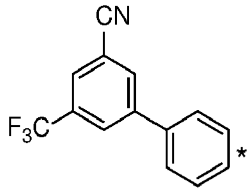 (D160),

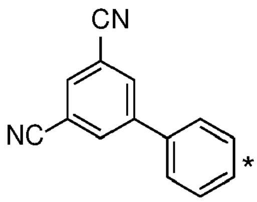 (D161), 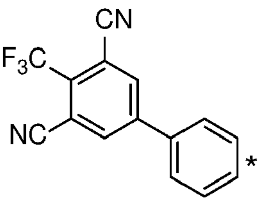 (D162), 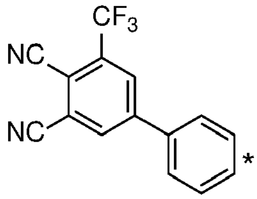 (D163),

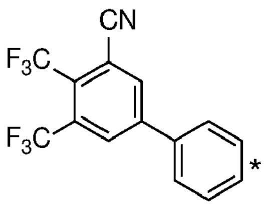 (D164), 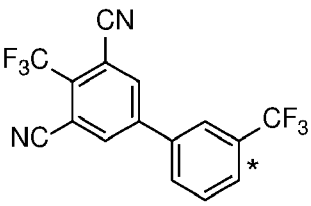 (D165), 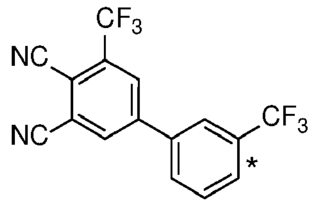 (D166),

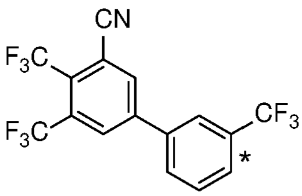 (D167), 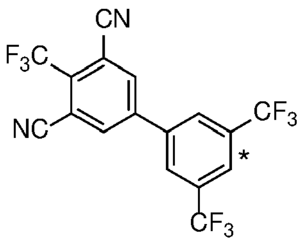 (D168), 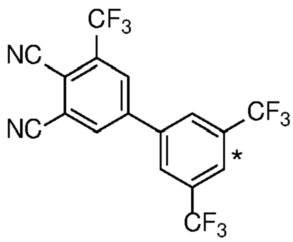 (D169),

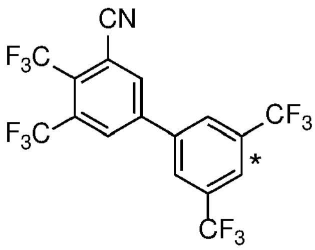 (D170);

wherein the "*" denotes the binding position.

According to one embodiment, wherein formula (IVa) is selected from the group comprising of D3 to D85 and D92 to D170, preferably D3 to D62 and D92 to D158.

According to one embodiment, wherein formula (IVa) is selected from the group comprising of D1 to D85 and D159 to D170, preferably D3 to D85 and D159 to D170.

According to one embodiment wherein formula (IVb) is selected from the group comprising of F1 to F7:

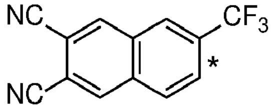 (F1), 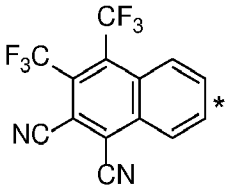 (F2), 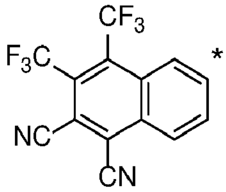 (F3), 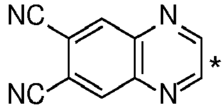 (F4),

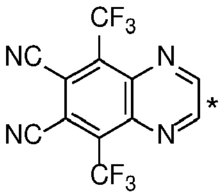 (F5), 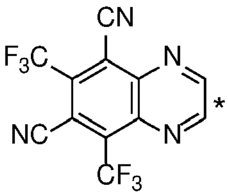 (F6), 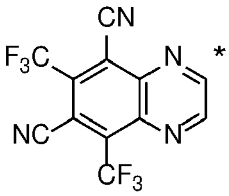 (F7);

wherein the "*" denotes the binding position.

According to an embodiment of formula (IVa) and/or (IVb), wherein at least one $X^{1'}$ to $X^{6'}$ is selected from CH, CD or N.

According to an embodiment, $R^5$ of Formula (I) may be selected from formula (IV) or CN, preferably $R^5$ may be CN.

**Compound of formula (I)**

According to one embodiment wherein the compound of formula (I) is selected from the group comprising of G1 to G19; wherein the "*" denotes the binding position:

| Name | $R^1$ | $R^2$ | $R^3$ | $R^4$ | R' | R" |
|---|---|---|---|---|---|---|
| G1 | F | F | F | F | 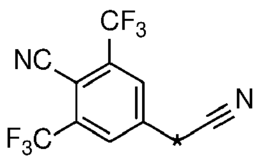 | 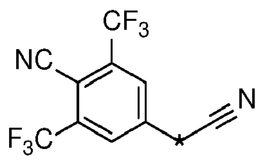 |
| G2 | F | F | F | F | 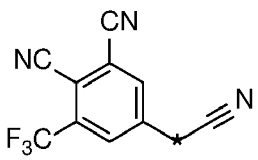 | 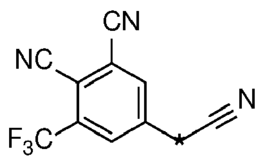 |
| G3 | Cl | F | F | Cl | 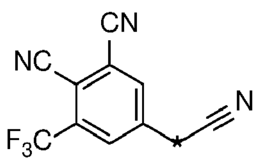 | 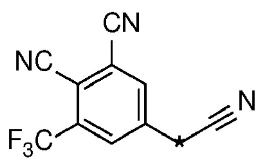 |
| G4 | $CF_3$ | F | F | $CF_3$ | 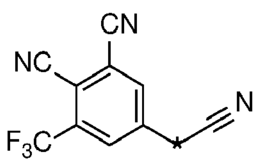 | 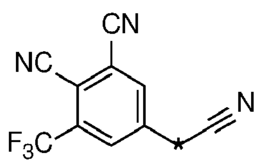 |

(continued)

| Name | $R^1$ | $R^2$ | $R^3$ | $R^4$ | R' | R" |
|---|---|---|---|---|---|---|
| G5 | CN | CN | F | F | 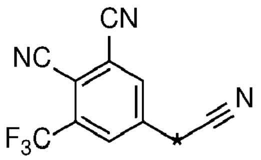 | 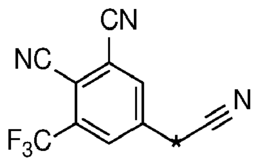 |
| G6 | CN | F | F | CN | 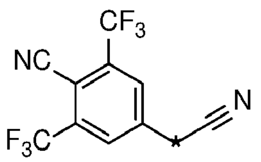 | 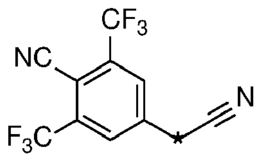 |
| G7 | CN | F | F | CN | 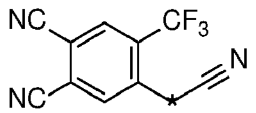 | 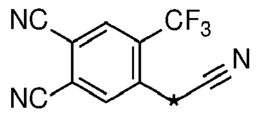 |
| G8 | CN | F | F | CN | 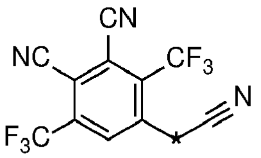 | 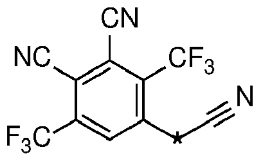 |
| G9 | CN | F | F | CN | 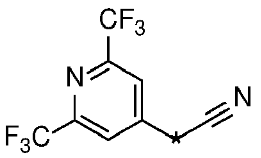 | 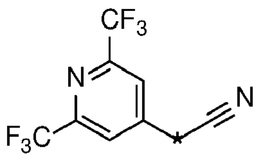 |
| G10 | CN | F | F | CN | 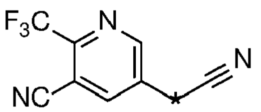 | 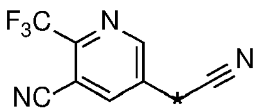 |
| G11 | CN | F | F | CN | 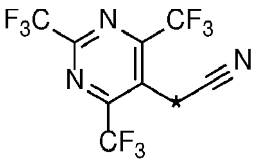 | 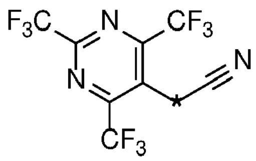 |
| G12 | CN | F | F | CN | 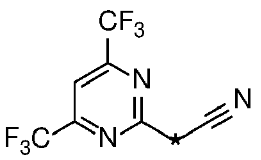 | 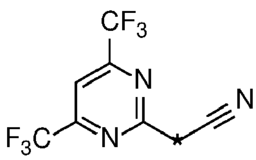 |
| G13 | CN | F | F | CN | 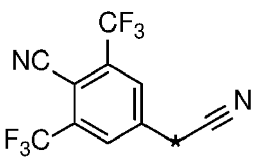 | 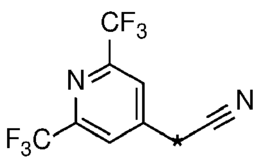 |

(continued)

| Name | $R^1$ | $R^2$ | $R^3$ | $R^4$ | R' | R" |
|---|---|---|---|---|---|---|
| G14 | F | F | F | F | 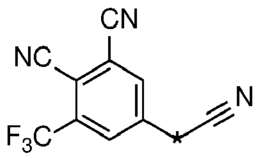 | 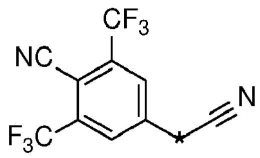 |
| G15 | CN | F | F | CN | 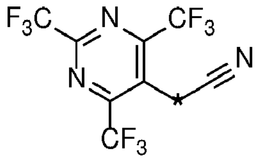 | 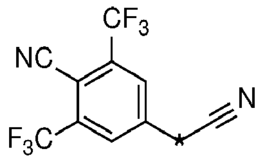 |
| G16 | $CF_3$ | CN | CN | $CF_3$ | 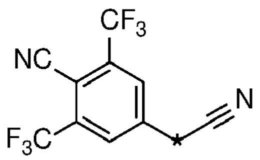 | 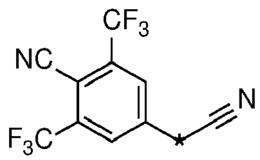 |
| G17 | F | F | F | F | 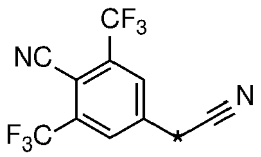 | 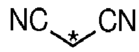 |
| G18 | CN | F | CN | F | 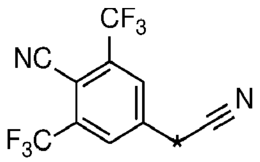 | 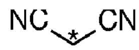 |
| G19 | CN | F | CN | F | 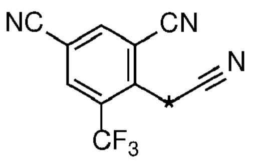 | 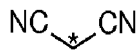 |

According to one embodiment wherein the compound of formula (I) is selected from the group comprising of H1 to H19:

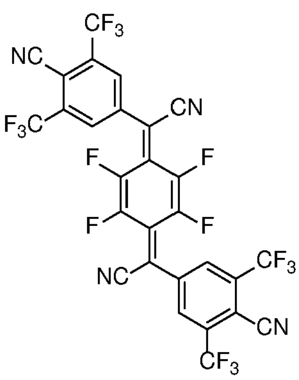 (H1), 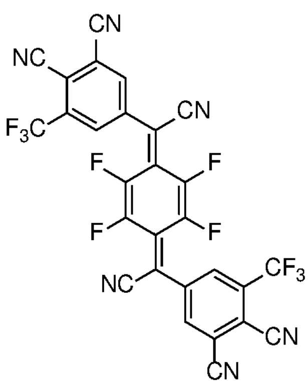 (H2), 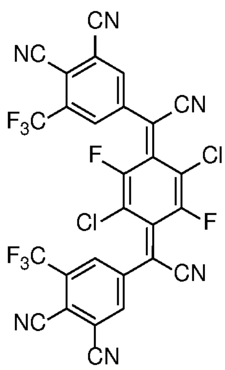 (H3),

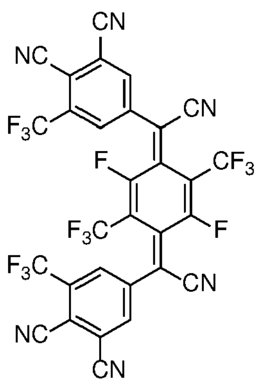 (H4), 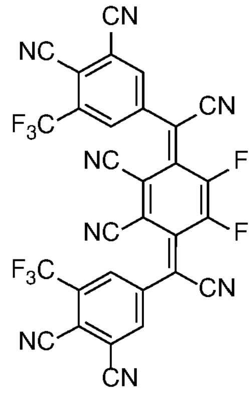 (H5), 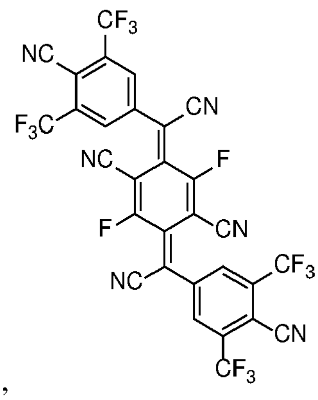 (H6),

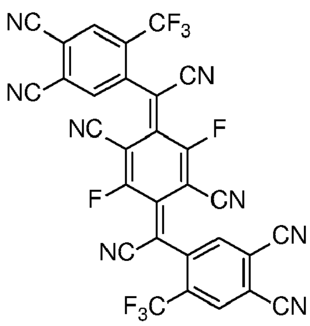 (H7), 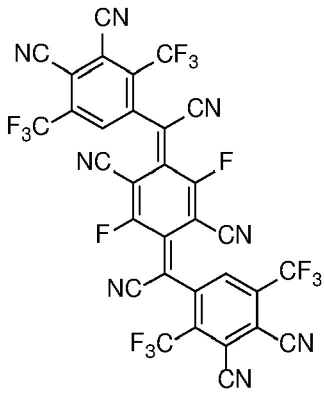 (H8), 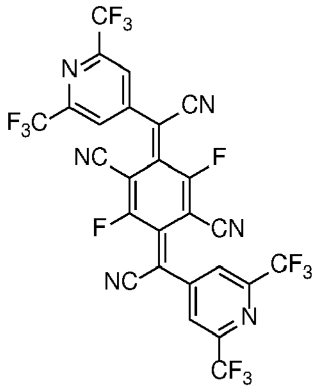 (H9),

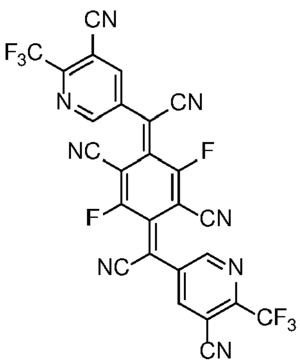 (H10), 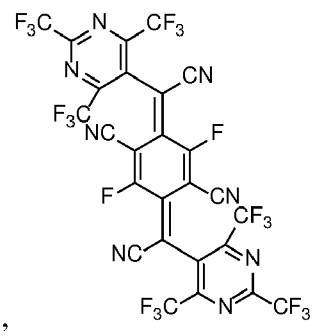 (H11), 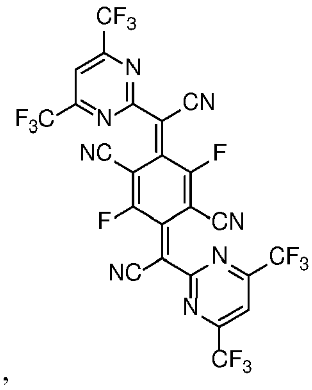 (H12),

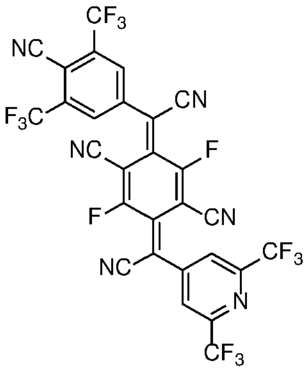 (H13), 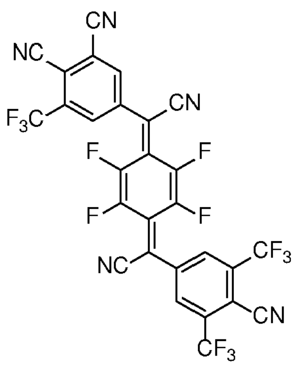 (H14), 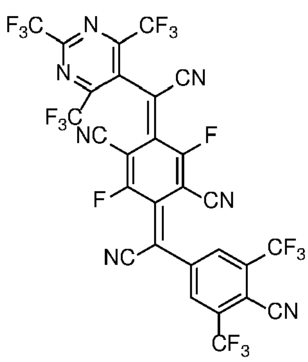 (H15),

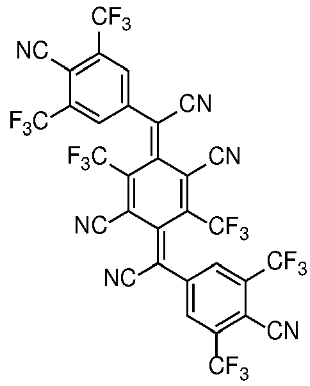 (H16), 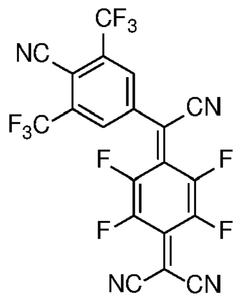 (H17), 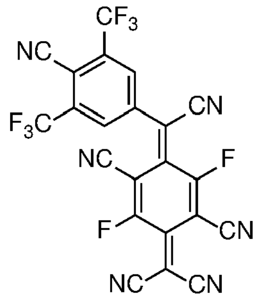 (H18),

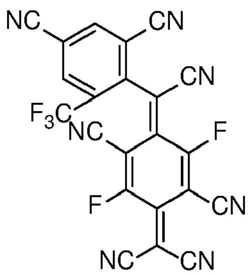 (H19).

According to one embodiment of the present invention, the compound of formula (I) comprises less than nine CN groups, preferably less than eight CN groups.

According to one embodiment of the present invention, the compound of formula (I) comprises ≥ 6 F and ≤ 24 F groups, preferably ≥ 8 F and ≤ 18 F groups.

According to one embodiment of the present invention, the calculated LUMO of the compound of Formula (I) is in the range of ≤ -4.9 eV to ≥ -5.75 eV, when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase, preferably ≤ -4.95 eV to ≥ -5.75 eV; even more preferred ≤ 4.95 eV to ≥ 5.6 eV.

## Organic semiconductor layer

The present invention furthermore relates to an organic semiconductor layer, whereby the organic semiconductor layer comprises a compound according to the present invention or a composition according to the present invention.

The present invention furthermore relates to an organic semiconductor layer, whereby the organic semiconductor layer comprises a compound according to formula (I).

The present invention furthermore relates to an organic semiconductor layer, whereby the organic semiconductor layer comprises a compound according to formula (I), (Ia), (Ib), (Ic), (Id).

In case the organic semiconductor layer comprises a compound according to the invention, throughout this application text the term "compound of formula (I)" shall also intend to include a composition, wherein the composition comprises at least one compound according to the invention as described above.

According to one embodiment of the present invention the organic semiconductor layer and/or the compound of formula (I) are non-emissive.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

According to one embodiment of the invention, the at least one organic semiconductor layer may further comprise at least one matrix compound, also named covalent matrix compound, or substantially covalent matrix compound.

### Substantially covalent matrix compound

The organic semiconductor layer may further comprises a covalent matrix compound also named substantially covalent matrix compound. According to one embodiment the substantially covalent matrix compound may be selected

from at least one organic compound. The substantially covalent matrix may consists substantially from covalently bound C, H, O, N, S, which optionally comprise in addition covalently bound B, P, As and/or Se.

According to one embodiment of the organic electronic device, the organic semiconductor layer further comprises a substantially covalent matrix compound, wherein the substantially covalent matrix compound may be selected from organic compounds consisting substantially from covalently bound C, H, O, N, S, which optionally comprise in addition covalently bound B, P, As and/or Se.

Organometallic compounds comprising covalent bonds carbon-metal, metal complexes comprising organic ligands and metal salts of organic acids are further examples of organic compounds that may serve as substantially covalent matrix compounds of the hole injection layer.

In one embodiment, the substantially covalent matrix compound lacks metal atoms and majority of its skeletal atoms may be selected from C, O, S, N. Alternatively, the substantially covalent matrix compound lacks metal atoms and majority of its skeletal atoms may be selected from C and N.

According to one embodiment, the substantially covalent matrix compound may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

Preferably, the substantially covalent matrix compound comprises at least one arylamine moiety, alternatively a diarylamine moiety, alternatively a triarylamine moiety.

Preferably, the substantially covalent matrix compound is free of metals and/or ionic bonds.

Compound of formula (VI) or a compound of formula (VII)

According to another aspect of the present invention, the at least one matrix compound, also referred to as "substantially covalent matrix compound", may comprises at least one arylamine compound, diarylamine compound, triarylamine compound, a compound of formula (VI) or a compound of formula (VII)

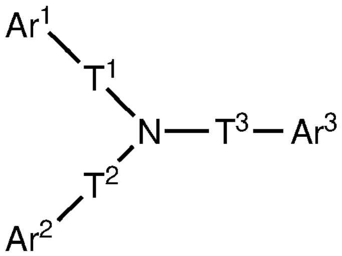 (VI), 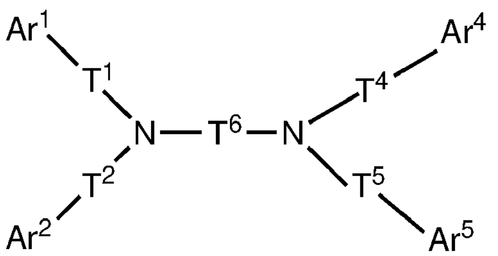 (VII),

wherein:

$T^1$, $T^2$, $T^3$, $T^4$ and $T^5$ are independently selected from a single bond, phenylene, biphenylene, terphenylene or naphthenylene, preferably a single bond or phenylene;

$T^6$ is phenylene, biphenylene, terphenylene or naphthenylene;

$Ar^1$, $Ar^2$, $Ar^3$, $Ar^4$ and $Ar^5$ are independently selected from substituted or unsubstituted C6 to $C_{20}$ aryl, or substituted or unsubstituted $C_3$ to $C_{20}$ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;

wherein the substituents of $Ar^1$, $Ar^2$, $Ar^3$, $Ar^4$ and $Ar^5$ are selected the same or different from the group comprising H, D, F, $C(-O)R^2$, CN, $Si(R^2)_3$, $P(-O)(R^2)_2$, $OR^2$, $S(-O)R^2$, $S(-O)_2R^2$, substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted alkoxy groups having 1 to 20 carbon atoms, substituted or unsubstituted aromatic ring systems having 6 to 40 aromatic ring atoms, and substituted or unsubstituted heteroaromatic ring systems having 5 to 40 aromatic ring atoms, unsubstituted $C_6$ to $C_{18}$ aryl, unsubstituted $C_3$ to $C_{18}$ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle, wherein $R^2$ may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, $C_6$ to $C_{18}$ aryl or $C_3$ to $C_{18}$ heteroaryl.

According to an embodiment wherein $T^1$, $T^2$, $T^3$, $T^4$ and $T^5$ may be independently selected from a single bond, phenylene, biphenylene or terphenylene. According to an embodiment wherein $T^1$, $T^2$, $T^3$, $T^4$ and $T^5$ may be independently selected from phenylene, biphenylene or terphenylene and one of $T^1$, $T^2$, $T^3$, $T^4$ and $T^5$ are a single bond. According to an embodiment wherein $T^1$, $T^2$, $T^3$, $T^4$ and $T^5$ may be independently selected from phenylene or biphenylene and one of $T^1$, $T^2$, $T^3$, $T^4$ and $T^5$ are a single bond. According to an embodiment wherein $T^1$, $T^2$, $T^3$, $T^4$ and $T^5$ may be independently selected from phenylene or biphenylene and two of $T^1$, $T^2$, $T^3$, $T^4$ and $T^5$ are a single bond.

According to an embodiment wherein $T^1$, $T^2$ and $T^3$ may be independently selected from phenylene and one of $T^1$, $T^2$ and $T^3$ are a single bond. According to an embodiment wherein $T^1$, $T^2$ and $T^3$ may be independently selected from phenylene and two of $T^1$, $T^2$ and $T^3$ are a single bond.

According to an embodiment wherein $T^6$ may be phenylene, biphenylene, terphenylene. According to an embodiment wherein $T^6$ may be phenylene. According to an embodiment wherein $T^6$ may be biphenylene. According to an embodiment wherein $T^6$ may be terphenylene.

According to an embodiment wherein $Ar^1$, $Ar^2$, $Ar^3$, $Ar^4$ and $Ar^5$ may be independently selected from K1 to K16:

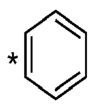 (K1), 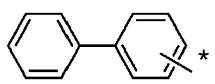 (K2), 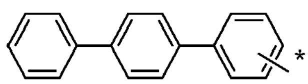 (K3), 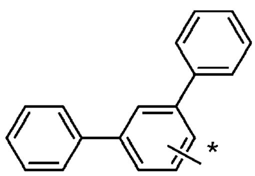 (K4),

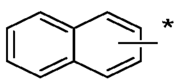 (K5), 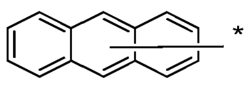 (K6), 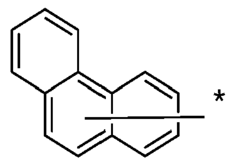 (K7), 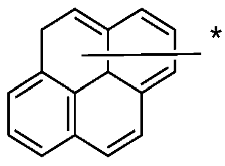 (K8),

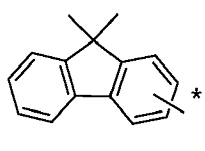 (K9), 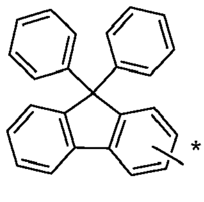 (K10), 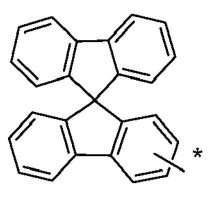 (K11),

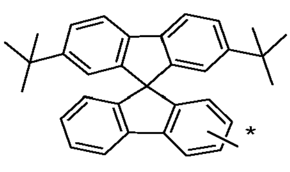 (K12), 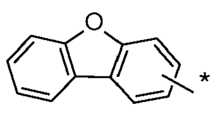 (K13), 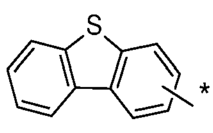 (K14),

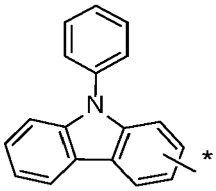 (K15), 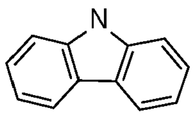 (K16),

, wherein the asterix "*" denotes the binding position.

According to an embodiment, wherein $Ar^1$, $Ar^2$, $Ar^3$, $Ar^4$ and $Ar^5$ may be independently selected from K1 to K15; alternatively selected from K1 to K10 and K13 to K15.

According to an embodiment, wherein $Ar^1$, $Ar^2$, $Ar^3$, $Ar^4$ and $Ar^5$ may be independently selected from the group consisting of K1, K2, K5, K7, K9, K10, K13 to K16.

The rate onset temperature may be in a range particularly suited to mass production, when $Ar^1$, $Ar^2$, $Ar^3$, $Ar^4$ and $Ar^5$ are selected in this range.

The "matrix compound of formula (VI) or formula (VII)" may be also referred to as "hole transport compound".

According to one embodiment, the substantially covalent matrix compound comprises at least one naphthyl group, carbazole group, dibenzofuran group, dibenzothiophene group and/or substituted fluorenyl group, wherein the substituents are independently selected from methyl, phenyl or fluorenyl.

According to an embodiment of the electronic device, wherein the matrix compound of formula (VI) or formula (VII) are selected from L1 to L18:

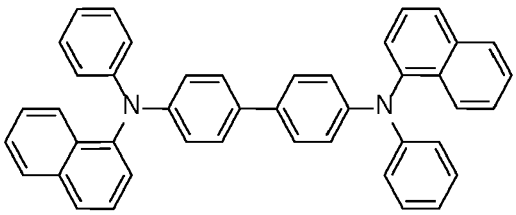 (L1), 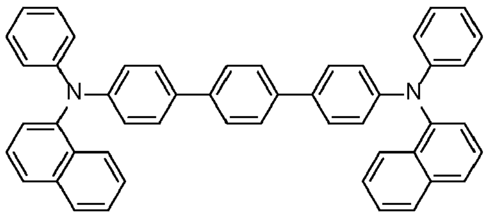 (L2),

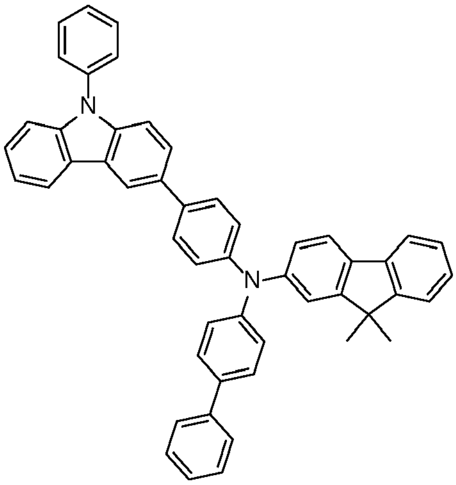 (L3), 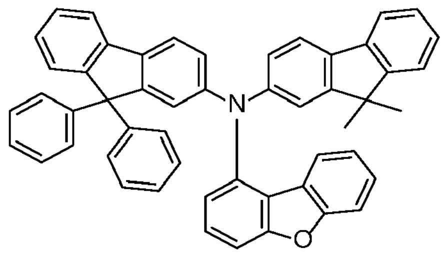 (L4),

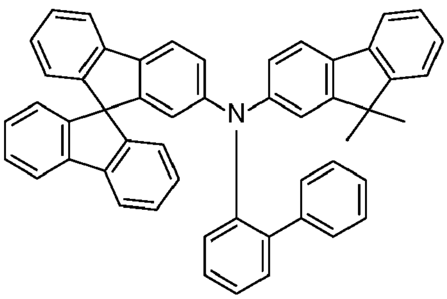 (L5), 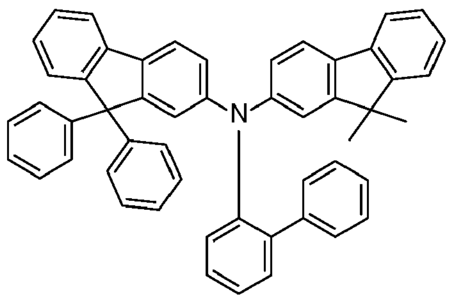 (L6),

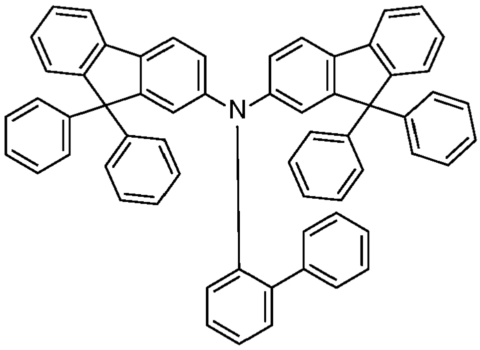

(L7),

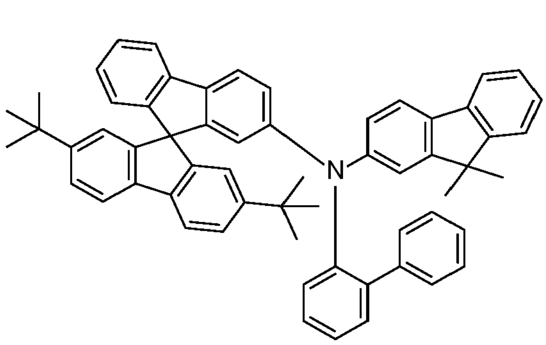

(L8),

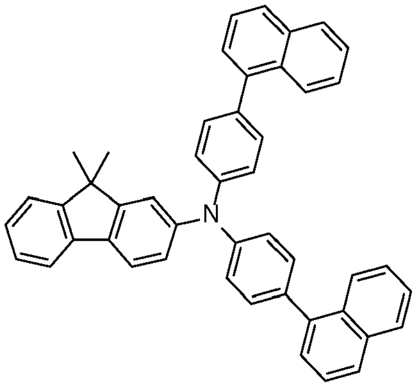

(L9),

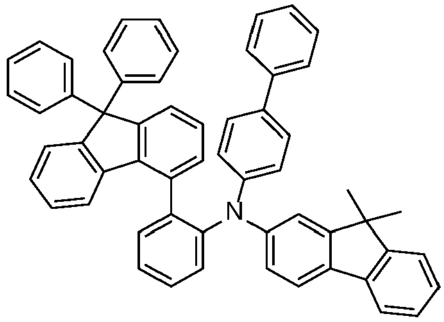

(L10),

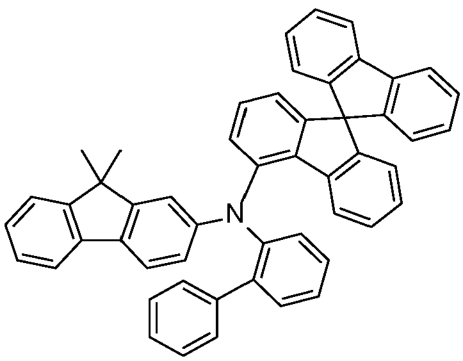

(L11),

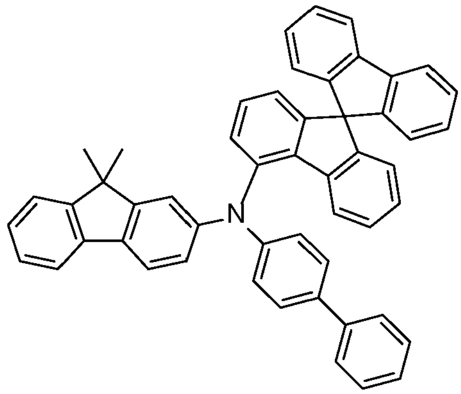

(L12),

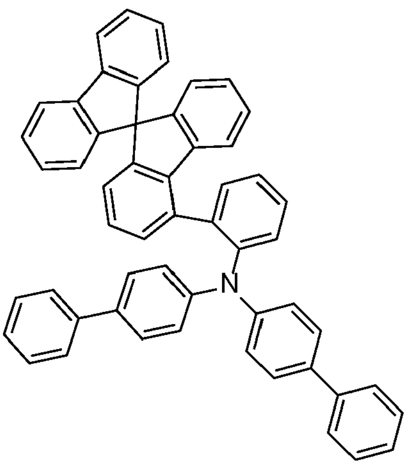

(L13),

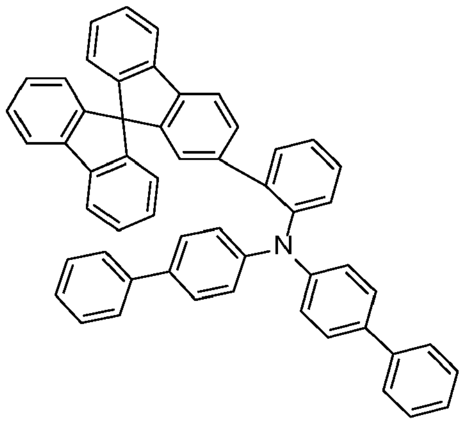

(L14),

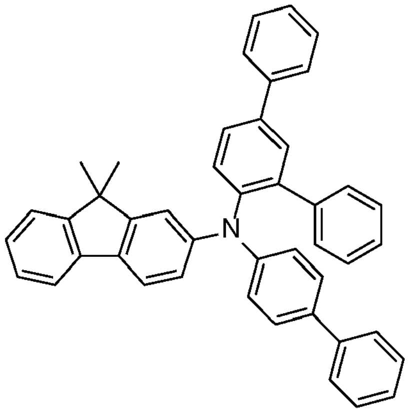
(L15),
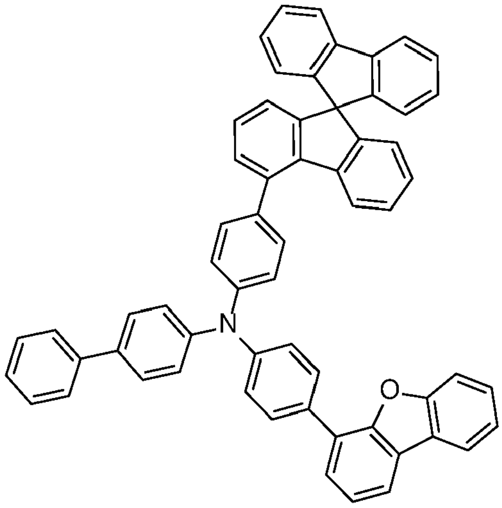
(L16),
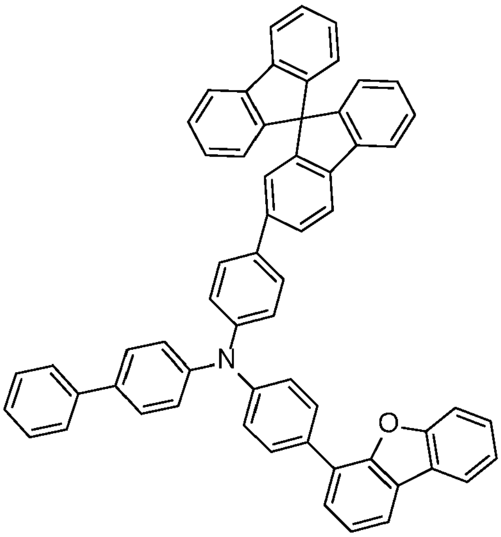
(L17)
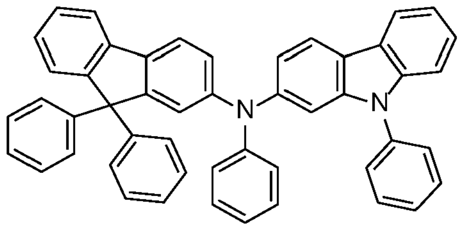
(L18).

**Organic electronic device**

The present invention furthermore relates to an organic electronic device comprising an anode layer, a cathode layer, and at least one organic semiconductor layer, wherein the at least one organic semiconductor layer is arranged between the anode layer and the cathode layer, and wherein the at least one organic semiconductor layer is the organic semiconductor layer according to the present invention.

According to one embodiment of the organic electronic device according to the invention whereby the anode layer comprises at least a first anode sub-layer and a second anode sub-layer.

According to one embodiment of the present invention, wherein the organic electronic device further comprises at least one photoactive layer, wherein the at least one photoactive layer is arranged between the anode layer and the cathode layer and at least one of the at least one organic semiconductor layers is arranged between the anode layer and the at least one photoactive layer.

According to one embodiment of the present invention, wherein the organic semiconductor layer is a hole injection layer.

According to one embodiment of the present invention, the hole injection layer is in direct contact with the anode layer.

According to one embodiment of the present invention, the hole injection layer is in direct contact with the anode layer and the anode layer is in direct contact with the substrate, wherein the substrate is selected from a glass substrate, a plastic substrate, a metal substrate or a backplane.

According to one embodiment of the present invention, wherein the photoactive layer is a light emitting layer.

According to one embodiment of the present invention, wherein the organic electronic device is an electroluminescent device, an organic light emitting diode (OLED), a light emitting device, thin film transistor, a battery, a display device or an organic photovoltaic cell (OPV).

According to one embodiment of the present invention, wherein the organic semiconductor layer is a p-type charge generation layer.

According to one embodiment of the present invention, the organic electronic device comprises at least two emission layers, wherein at least one of the at least one organic semiconductor layers is arranged between the first and the second emission layer.

According to one embodiment of the present invention, the organic electronic device is an electroluminescent device, preferably an organic light emitting diode.

According to one embodiment of the present invention, the organic electronic device further comprises a substrate.

According to one embodiment of the invention, the electronic organic device is an organic light emitting diode comprising a substrate, an anode layer, an organic semiconductor layer comprising a compound of formula (I), an emission layer, an optional electron transport layer, an optional electron injection and a cathode layer. The organic light emitting diode may further comprise a p-type charge generation layer, the p-type charge generation layer is arranged between the anode layer and the cathode layer; and the p-type layer comprises a compound of formula (I).

According to one embodiment of the invention, the electronic organic device is an organic light emitting diode comprising a substrate, an anode layer, a hole injection layer optionally comprising a compound of formula (I), a first emission layer, a charge generation layer comprising a p-type charge generation layer comprising a compound of formula (I), a second emission layer, an optional electron transport layer, an optional electron injection and a cathode layer.

The present invention furthermore relates to a display device comprising an organic electronic device according to the present invention.

### Further layers

In accordance with the invention, the organic electronic device may comprise, besides the layers already mentioned above, further layers. Exemplary embodiments of respective layers are described in the following:

### Substrate

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate, a silicon substrate or a backplane.

### Anode layer

The anode layer may be formed by depositing or sputtering a material that is used to form the anode layer. The material used to form the anode layer may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode layer may also be formed using metals, typically silver (Ag), gold (Au), or metal alloys.

According to one embodiment of the present invention, the anode layer comprises a first anode sub-layer and a second anode sub-layer, wherein

- the first anode sub-layer comprises a first metal having a work function in the range of ≥ 4 and ≤ 6 eV, and
- the second anode sub-layer comprises a transparent conductive oxide; and
- the second anode sub-layer is arranged closer to the hole injection layer.

According to one embodiment of the present invention, the first metal of the first anode sub-layer may be selected from the group comprising Ag, Mg, Al, Cr, Pt, Au, Pd, Ni, Nd, Ir, preferably Ag, Au or Al, and more preferred Ag.

According to one embodiment of the present invention, the first anode sub-layer has have a thickness in the range of 5 to 200 nm, alternatively 8 to 180 nm, alternatively 8 to 150 nm, alternatively 100 to 150 nm.

According to one embodiment of the present invention, the first anode sub-layer is formed by depositing the first metal via vacuum thermal evaporation.

It is to be understood that the first anode layer is not part of the substrate.

According to one embodiment of the present invention, the transparent conductive oxide of the second anode sub layer is selected from the group selected from the group comprising indium tin oxide or indium zinc oxide, more preferred indium tin oxide.

According to one embodiment of the present invention, the second anode sub-layer may has a thickness in the range of 3 to 200 nm, alternatively 3 to 180 nm, alternatively 3 to 150 nm, alternatively 3 to 20 nm.

According to one embodiment of the present invention, the second anode sub-layer may be formed by sputtering of the transparent conductive oxide.

According to one embodiment of the present invention, anode layer of the organic electronic device comprises in addition a third anode sub-layer comprising a transparent conductive oxide, wherein the third anode sub-layer is arranged between the substrate and the first anode sub-layer.

According to one embodiment of the present invention, the third anode sub-layer comprises a transparent oxide, preferably from the group selected from the group comprising indium tin oxide or indium zinc oxide, more preferred indium tin oxide.

According to one embodiment of the present invention, the third anode sub-layer may has a thickness in the range of 3 to 200 nm, alternatively 3 to 180 nm, alternatively 3 to 150 nm, alternatively 3 to 20 nm.

According to one embodiment of the present invention, the third anode sub-layer may be formed by sputtering of the transparent conductive oxide.

It is to be understood that the third anode layer is not part of the substrate.

According to one embodiment of the present invention, the anode layer comprises a first anode sub-layer comprising of Ag, a second anode sub-layer comprising of transparent conductive oxide, preferably ITO, and a third anode sub-layer comprising of transparent conductive oxide, preferably ITO; wherein the first anode sub-layer is arranged between the second and the third anode sub-layer.

**Hole transport layer**

According to one embodiment of the present invention, the organic electronic device comprises a hole transport layer, wherein the hole transport layer is arranged between the hole injection layer and the at least one first emission layer.

The hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010 and incorporated by reference. Examples of the compound that may be used to form the HTL are: carbazole derivatives, such as N-phenyl-carbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

According to one embodiment of the present invention, the hole transport layer may comprise a substantially covalent matrix compound as described above.

According to one embodiment of the present invention, the hole transport layer may comprise a compound of formula (VI) or (VII) as described above.

According to one embodiment of the present invention, the hole injection layer and the hole transport layer comprises the same substantially covalent matrix compound as described above.

According to one embodiment of the present invention, the hole injection layer and the hole transport layer comprises the same compound of formula (VI) or (VII) as described above.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 110 nm to about 140 nm.

When the thickness of the HTL is within this range, the HTL may have excellent hole transporting characteristics, without a substantial penalty in driving voltage.

**Electron blocking layer**

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer.

The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

**Photoactive layer (PAL)**

The photoactive layer converts an electrical current into photons or photons into an electrical current. The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL. It may be provided that the photoactive layer does not comprise the a metal complex according to formula (I). The photoactive layer may be a light-emitting layer or a light-absorbing layer.

**Emission layer (EML)**

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

According to one embodiment of the present invention, the emission layer does not comprise the compound of formula (I).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

**Hole blocking layer (HBL)**

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, phenanthroline derivatives and azine derivatives, preferably triazine or pyrimidine derivatives.

The HBL may have a thickness in the range from about 5 nm to about 100 nm, for example, from about 10 nm

to about 30 nm. When the thickness of the HBL is within this range, the HBL may have excellent hole-blocking properties, without a substantial penalty in driving voltage.

**Electron transport layer (ETL)**

The organic electronic device according to the present invention may further comprise an electron transport layer (ETL).

According to another embodiment of the present invention, the electron transport layer may further comprise an azine compound, preferably a triazine compound.

In one embodiment, the electron transport layer may further comprise a dopant selected from an alkali organic complex, preferably LiQ.

The thickness of the ETL may be in the range from about 15 nm to about 50 nm, for example, in the range from about 20 nm to about 40 nm. When the thickness of the EIL is within this range, the ETL may have satisfactory electron-injecting properties, without a substantial penalty in driving voltage.

According to another embodiment of the present invention, the organic electronic device may further comprise a hole blocking layer and an electron transport layer, wherein the hole blocking layer and the electron transport layer comprise an azine compound. Preferably, the azine compound is a triazine compound.

**Electron injection layer (EIL)**

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

**Charge generation layer**

The organic electronic device according to the present invention may further comprise a charge generation layer, wherein the organic semiconductor layer of the present invention is a p-type charge generation layer (p-CGL), wherein the p-type charge generation layer is arranged closer to the cathode layer.

The charge generation layer may further comprise a n-type charge generation layer (n-CGL), wherein the n-type charge generation layer is arranged between the p-type charge generation layer and the anode layer.

Preferably, the n-type charge generation layer and the p-type charge generation layer are arranged in direct contact.

The thickness of the n-CGL may be in the range from about 0.5 nm to about 15 nm, for example, in the range from about 1 nm to about 10 nm. When the thickness of the n-CGL is within this range, the EIL may have satisfactory electron-injecting properties, without a substantial penalty in driving voltage.

The n-CGL may comprise a metal dopant, wherein the metal dopant is selected from an alkali metal, alkaline earth metal or rare earth metal.

The n-CGL may comprise an azine compound. According to a preferred embodiment, the nCGL may comprise an azine compound and a metal dopant, wherein the metal dopant is selected from an alkali metal, alkaline earth metal or rare earth metal.

**Cathode layer**

The cathode layer is formed on the ETL or optional EIL. The cathode layer may be formed of a metal, an alloy, an electrically conductive compound, or a mixture thereof. The cathode electrode may have a low work function. For example, the cathode layer may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode layer may be in the range from about 5 nm to about 1000 nm, for example, in the range from about 10 nm to about 100 nm. When the thickness of the cathode layer is in the range from about 5 nm to about 50 nm, the cathode layer may be transparent or semitransparent even if formed from a metal or metal alloy.

It is to be understood that the cathode layer is not part of an electron injection layer or the electron transport layer.

**Organic light-emitting diode (OLED)**

The organic electronic device according to the invention may be an organic light-emitting device.

According to one aspect of the present invention, there is provided an organic light-emitting diode (OLED) comprising: a substrate; an anode layer formed on the substrate; a hole injection layer comprising a compound of formula (I), a hole transport layer, an emission layer, an electron transport layer and a cathode layer.

According to another aspect of the present invention, there is provided an OLED comprising: a substrate; an anode layer formed on the substrate; a hole injection layer comprising a compound of formula (I), a hole transport layer, an electron blocking layer, an emission layer, a hole blocking layer, an electron transport layer and a cathode layer.

According to another aspect of the present invention, there is provided an OLED comprising: a substrate; an anode layer formed on the substrate; a hole injection layer comprising a compound of formula (I), a hole transport layer, an electron blocking layer, an emission layer, a hole blocking layer, an electron transport layer, an electron injection layer, and a cathode layer.

According to various embodiments of the present invention, there may be provided OLEDs layers arranged between the above mentioned layers, on the substrate or on the top layer.

For example, the OLED according to Fig. 3 may be formed by a process, wherein on a substrate (110), an anode (120), a hole injection layer (130) which may comprise compound of formula (I), a hole transport layer (140), an electron blocking layer (145), an emission layer (150), a hole blocking layer (155), an electron transport layer (160), an electron injection layer (180) and the cathode layer (190) are subsequently formed in that order.

According to one aspect, the OLED may comprise a layer structure of a substrate that is adjacent arranged to an anode layer, the anode layer is adjacent arranged to a hole injection layer, the hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a p-type charge generation layer, the p-type charge generation layer is adjacent arranged to a second hole transport layer, the second hole transport layer is adjacent arranged to a second electron blocking layer, the second electron blocking layer is adjacent arranged to a second emission layer, between the second emission layer and the cathode layer an optional electron transport layer and/or an optional injection layer are arranged.

The organic semiconductor layer according to the invention may be the first hole injection layer and/or the p-type charge generation layer.

**Organic electronic device**

The organic electronic device according to the invention may be a light emitting device, or a photovoltaic cell, and preferably a light emitting device.

According to another aspect of the present invention, there is provided a method of manufacturing an organic electronic device, the method using:

- at least one deposition source, preferably two deposition sources and more preferred at least three deposition sources.

The methods for deposition that can be suitable comprise:

- deposition via vacuum thermal evaporation;
- deposition via solution processing, preferably the processing is selected from spin-coating, printing, casting; and/or
- slot-die coating.

According to various embodiments of the present invention, there is provided a method using:

- a first deposition source to release the compound of formula (I) according to the invention, and
- a second deposition source to release the substantially covalent matrix compound; the method comprising the steps of forming the hole injection layer; whereby for an organic light-emitting diode (OLED):
- the hole injection layer is formed by releasing the compound of formula (I) according to the invention from the first deposition source and the substantially covalent matrix compound from the second deposition source.

According to various embodiments of the present invention, the method may further include forming on the anode layer, at least one layer selected from the group consisting of forming a hole transport layer or forming a hole

blocking layer, and an emission layer between the anode layer and the first electron transport layer.

According to various embodiments of the present invention, the method may further include the steps for forming an organic light-emitting diode (OLED), wherein

- on a substrate an anode layer is formed,
- on the anode layer a hole injection layer comprising a compound of formula (I) is formed,
- on the hole injection layer comprising a compound of formula (I) a hole transport layer is formed,
- on the hole transport layer an emission layer is formed,
- on the emission layer an electron transport layer is formed, optionally a hole blocking layer is formed on the emission layer,
- and finally a cathode layer is formed,
- optional a hole blocking layer is formed in that order between the first anode layer and the emission layer,
- optional an electron injection layer is formed between the electron transport layer and the cathode layer.

According to various embodiments, the OLED may have the following layer structure, wherein the layers having the following order:
anode layer, hole injection layer comprising a compound of formula (I) according to the invention, first hole transport layer, second hole transport layer, emission layer, optional hole blocking layer, electron transport layer, optional electron injection layer, and cathode layer.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

Hereinafter, the embodiments are illustrated in more detail with reference to examples. However, the present disclosure is not limited to the following examples. Reference will now be made in detail to the exemplary aspects.

**Description of the Drawings**

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.

FIG. 1 is a schematic sectional view of an organic electronic device, according to an exemplary embodiment of the present invention;
FIG. 2 is a schematic sectional view of an organic electronic device, according to an exemplary embodiment of the present invention;
FIG. 3 is a schematic sectional view of an organic electronic device , according to an exemplary embodiment of the present invention;
FIG. 4 is a schematic sectional view of an OLED, according to an exemplary embodiment of the present invention.
FIG. 5 is a schematic sectional view of an OLED, according to an exemplary embodiment of the present invention.
FIG. 6 is a schematic sectional view of an OLED, according to an exemplary embodiment of the present invention.
FIG. 7 is a schematic sectional view of an OLED, according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic electronic device 100, according to an exemplary embodiment of the present invention. The organic electronic device 100 includes an anode layer 120 and an organic semiconductor

layer 131 which may comprise a compound of formula (I). The organic semiconductor layer 131 is disposed on the anode layer 120. Onto the organic semiconductor layer 131 a cathode layer 190 are disposed.

FIG. 2 is a schematic sectional view of an organic electronic device 100, according to an exemplary embodiment of the present invention. The organic electronic device 100 includes a substrate 110, an anode layer 120 and a hole injection layer (HIL) 130 which may comprise a compound of formula (I). The HIL 130 is disposed on the anode layer 120. Onto the HIL 130, a photoactive layer (PAL) 170 and a cathode layer 190 are disposed.

FIG. 3 is a schematic sectional view of an organic light-emitting diode (OLED) 100, according to an exemplary embodiment of the present invention. The OLED 100 includes a substrate 110, an anode layer 120 and a hole injection layer (HIL) 130 which may comprise a compound of formula (I). The HIL 130 is disposed on the anode layer 120. Onto the HIL 130, a hole transport layer (HTL) 140, an emission layer (EML) 150, an electron transport layer (ETL) 160, an electron injection layer (EIL) 180 and a cathode layer 190 are disposed. Instead of a single electron transport layer 160, optionally an electron transport layer stack (ETL) can be used.

FIG. 4 is a schematic sectional view of an OLED 100, according to another exemplary embodiment of the present invention. Fig. 4 differs from Fig. 3 in that the OLED 100 of Fig. 4 comprises an electron blocking layer (EBL) 145 and a hole blocking layer (HBL) 155.

Referring to Fig. 4, the OLED 100 includes a substrate 110, an anode layer 120, a hole injection layer (HIL) 130 which may comprise a compound of formula (I), a hole transport layer (HTL) 140, an electron blocking layer (EBL) 145, an emission layer (EML) 150, a hole blocking layer (HBL) 155, an electron transport layer (ETL) 160, an electron injection layer (EIL) 180 and a cathode layer 190.

FIG. 5 is a schematic sectional view of an organic electronic device 100, according to an exemplary embodiment of the present invention. The organic electronic device 100 includes a substrate 110, an anode layer 120 that comprises a first anode sub-layer 121, a second anode sub-layer 122 and a third anode sub-layer 123, and a hole injection layer (HIL) 130. The HIL 130 is disposed on the anode layer 120. Onto the HIL 130, an hole transport layer (HTL) 140, a first emission layer (EML) 150, a hole blocking layer (HBL) 155, an electron transport layer (ETL) 160, and a cathode layer 190 are disposed. The hole injection layer 130 may comprise a compound of formula (I).

FIG. 6 is a schematic sectional view of an organic electronic device 100, according to an exemplary embodiment of the present invention. The organic electronic device 100 includes a substrate 110, an anode layer 120 that comprises a first anode sub-layer 121, a second anode sub-layer 122 and a third anode sub-layer 123, and a hole injection layer (HIL) 130. The HIL 130 is disposed on the anode layer 120. Onto the HIL 130, a hole transport layer (HTL) 140, an electron blocking layer (EBL) 145, a first emission layer (EML) 150, a hole blocking layer (HBL) 155, an electron transport layer (ETL) 160, an electron injection layer (EIL) 180 and a cathode layer 190 are disposed. The hole injection layer 130 may comprise a compound of formula (I).

FIG. 7 is a schematic sectional view of an organic electronic device 100, according to an exemplary embodiment of the present invention. The organic electronic device 100 includes a substrate 110, an anode layer 120, a hole injection layer (HIL) 130, a first hole transport layer (HTL) 140, a first electron blocking layer (EBL1) 145, a first emission layer (EML1) 150, an optional first hole blocking layer (HBL) 155, a first electron transport layer (ETL1) 160, an n-type charge generation layer (n-CGL) 185, a p-type charge generation layer (p-GCL) 135 which may comprise compound of formula (I), a second hole transport layer (HTL2) 141, a second electron blocking layer (EBL2) 146, a second emission layer (EML2) 151, an optional second hole blocking layer (HBL2) 156, a second electron transport layer (ETL2) 161, an electron injection layer (EIL) 180 and a cathode layer 190. The HIL may also comprise a compound of formula (I). The hole injection layer may comprise compound of formula (I). The anode layer may comprise a first anode sub-layer, a second anode sub-layer, and an optional third anode sub-layer.

While not shown in Fig. 1 to Fig. 7 , a capping and/or sealing layer may further be formed on the cathode layer 190, in order to seal the organic electronic device 100. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

## Detailed description

The invention is furthermore illustrated by the following examples which are illustrative only and non-binding.

Compounds of formula (I) may be prepared by methods known in the art, for example US2005121667A1, or as described below.

Scheme 1: Method for preparation of compounds of formula (I), (Ia), (Ib), (Ic), (Id)

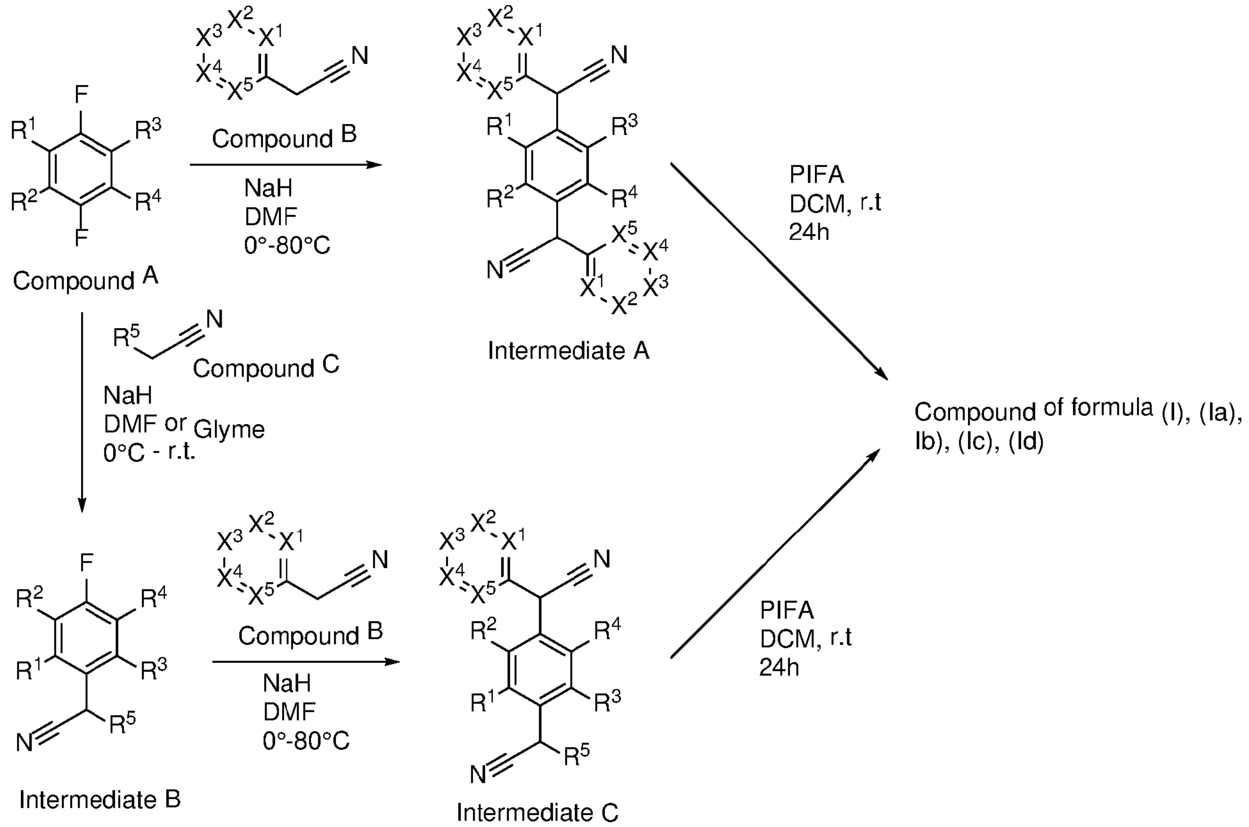


**Intermediate A**

In a 3-neck round-bottle flask equipped with a reflux condenser dropping funnel and a septum, 2.4 equivalents of 100% sodium hydride was suspended in dry DMF and cooled below 10°C using an ice bath under inert gas atmosphere. After dropwise addition of a solution of 2.3 equivalents of Compound B in anhydrous DMF, the mixture was stirred for 15 minutes at 0 to 10°C. Then 1 equivalent Compound A was added as a solid in one portion and the mixture was stirred for 15 minutes at 0°C, 15 minutes at room temperature and at 80°C overnight. If the reaction was incomplete on the next morning, the reaction mixture was added dropwise to a suspension of 0.5 to 2 equivalents of additional Compound B deprotonated with a small excess of 100% sodium hydride (0.6 to 2.1 equivalents respectively) and stirred at 80°C for further 24 hours. After completion of the reaction, the mixture was poured on an ice/water mixture and acidified with hydrochloric acid. The aqueous phase was extracted with ethyl acetate twice and the organic phase was then washed with half-concentrated brine three times and additionally with concentrated brine and dried using sodium sulfate. After removal of solvents, the product was stirred in DCM over night at room temperature, filtered and dissolved in a small amount of ethyl acetate again. The concentrated solution was then added dropwise into an 8-fold excess of dichloromethane. The precipitated product was filtered, washed with dichloromethane twice and had a purity of > 98% by HPLC.

**Intermediate B**

In a 3-neck round-bottle flask equipped with a reflux condenser dropping funnel and a septum, 1.1 equivalents of 100% sodium hydride was suspended in dry DMF and cooled below 10°C using an ice bath under inert gas atmosphere. After dropwise addition of a solution of 1 equivalent of Compound C in dry DMF, the mixture was stirred for 15 minutes at 0 to 10°C. Then 1 equivalent Compound A was added as a solid in one portion and the mixture was stirred for 15 minutes at 0°C and at room temperature overnight. If the reaction was found to be incomplete on the next morning, the reaction mixture was added dropwise to a suspension of 0.5 to 1 equivalents of additional Compound C deprotonated with a small excess of 100% sodium hydride (0.6 to 1.1 equivalents respectively) and stirred for further 24 hours at room temperature. After completion of the reaction, the mixture was poured on an ice/water mixture and acidified with hydrochloric acid. The aqueous phase was extracted with ethyl acetate twice and the organic phase was then washed with half-concentrated brine three times and additionally with concentrated brine and dried using sodium sulfate. After removal

of solvents, the product was stirred in toluene over night at room temperature, filtered off, washed with toluene several times to yield a purity of >95% by HPLC.

**Intermediate C**

In a 3-neck round-bottle flask equipped with a reflux condenser dropping funnel and a septum, 1.5 equivalents of 100% sodium hydride was suspended in dry DMF and cooled down below 10°C using an ice bath under inert gas atmosphere. After dropwise addition of a solution of 1.4 equivalents of Compound B in anhydrous DMF, the mixture was stirred for 15 minutes at 0° to 10°C. Then 1 equivalent Intermediate B was added as a solid in one portion and the mixture was stirred for 15 minutes at 0°C, 15 minutes at room temperature and at 80°C overnight. If the reactions was incomplete on the next morning, the reaction mixture was added dropwise to a suspension of 0.5 to 1.5 equivalents of additional Compound B deprotonated with a small excess of 100% sodium hydride (0.6 to 1.6 equivalents respectively) and stirred at 80°C for additional 24 hours. After completion of the reaction, the mixture was poured on an ice/water mixture and acidified with hydrochloric acid. The aqueous phase was extracted with ethyl acetate twice and the organic phase was then washed with half-concentrated brine three times and additionally with concentrated brine and dried using sodium sulfate. After removal of solvents, the product was stirred in DCM over night at room temperature, filtered and dissolved in a small amount of ethyl acetate again. The concentrated solution was then added dropwise into an 8-fold excess of dichloromethane. The precipitated product was filtered, washed with dichloromethane twice and had a purity of >98% by HPLC.

**Compound (I), (Ia), (Ib), (Ic), (Id)**

Prior to oxidation, the Intermediate A / Intermediate C was converted into a potassium salt by suspending in a potassium carbonate solution. The salt was then extracted with ethyl acetate and the solution was concentrated in vacuum. By dropwise addition of the concentrated solution into an excess of n-hexane, the potassium salt was precipitated and filtered off. After drying in vacuum for two hours, the potassium salt was suspended in dry DCM under inert gas atmosphere, 1.1 equivalents of PIFA were added in one portion and the mixture was stirred under exclusion of light over night at room temperature. The product was filtered off, washed with DCM three times and purified by stirring in refluxing chloroform and ethyl acetate at room temperature to yield a yellow powder. The product may be purified further by methods known in the art, for example US2005121667A1.

**Calculated HOMO energy**

The HOMO energy is calculated with the program package TURBOMOLE V6.5 (TURBOMOLE GmbH, Litzenhardtstrasse 19, 76135 Karlsruhe, Germany). The optimized geometries and the HOMO energy levels of the molecular structures are determined by applying the hybrid functional B3LYP with a 6-31G* basis set in the gas phase. If more than one conformation is viable, the conformation with the lowest total energy is selected.

**Calculated LUMO energy**

The LUMO energy is calculated with the program package TURBOMOLE V6.5 (TURBOMOLE GmbH, Litzenhardtstrasse 19, 76135 Karlsruhe, Germany). The optimized geometries and the HOMO energy levels of the molecular structures are determined by applying the hybrid functional B3LYP with a 6-31G* basis set in the gas phase. If more than one conformation is viable, the conformation with the lowest total energy is selected.

**Melting point**

The melting point (Tm) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 $\mu$L Mettler Toledo aluminum pan with lid, a <1 mm hole is pierced into the lid).

**Glass transition temperature**

The glass transition temperature (Tg) is measured under nitrogen and using a heating rate of 10 K per min in a Mettler Toledo DSC 822e differential scanning calorimeter as described in DIN EN ISO 11357, published in March 2010.

**Thermogravimetric analysis**

The term "TGA5%" denotes the temperature at which 5 % weight loss occurs during thermogravimetric analysis and is measured in °C.

The TGA5% value may be determined by heating a 9-11 mg sample in a thermogravimetric analyzer at a heating rate of 10 K/min in an open 100 μL aluminum pan, under a stream of nitrogen at a flow rate of 20 mL/min in the balance area and of 30 mL/min in the oven area.

The TGA5% value may provide an indirect measure of the volatility and/or decomposition temperature of a compound. In first approximation, the higher the TGA5% value the lower is the volatility of a compound and/or the higher the decomposition temperature.

According to one embodiment, the TGA5% value of compound of formula (I) is selected in the range of≥ 280 °C and ≤ 420 °C; preferably of≥ 290 °C and ≤ 410 °C, also preferred of≥ 295 °C and ≤ 410 °C.

**Rate onset temperature**

The rate onset temperature ($T_{RO}$) is determined by loading 100 mg compound into a VTE source. As VTE source a point source for organic materials may be used as supplied by Kurt J. Lesker Company (www.lesker.com) or CreaPhys GmbH (http://www.creaphys.com). The VTE source is heated at a constant rate of 15 K/min at a pressure of less than $10^{-5}$ mbar and the temperature inside the source measured with a thermocouple. Evaporation of the compound is detected with a QCM detector which detects deposition of the compound on the quartz crystal of the detector. The deposition rate on the quartz crystal is measured in Angstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 185 to 280 °C. If the rate onset temperature is below 185 °C the evaporation may be too rapid and therefore difficult to control. If the rate onset temperature is above 280 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

**Dipole moment**

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom $i$ in the molecule. The dipole moment was obtained from the optimized geometries at the same level of theory. The geometries of the molecular structures are optimized using the hybrid functional B3LYP with the 6-31G* basis set in the gas phase (program package TURBOMOLE V6.5, TURBOMOLE GmbH, Litzenhardtstrasse 19, 76135 Karlsruhe, Germany). If more than one conformation is viable, the conformation with the lowest total energy is selected to determine the structural and electronic parameters of the molecules.

General procedure for fabrication of OLEDs wherein the organic semiconductor layer is a hole injection layer (HIL)

For Example 1-1 to 1-10 and comparative examples 1-1 to 1-3 in Table 6, a glass substrate with an anode layer comprising a first anode sub-layer of 120 nm Ag, a second anode sub-layer of 8 nm ITO and a third anode sub-layer of 10 nm ITO was cut to a size of 50 mm x 50 mm x 0.7 mm, ultrasonically washed with water for 60 minutes and then with isopropanol for 20 minutes. The liquid film was removed in a nitrogen stream, followed by plasma treatment,

to prepare the anode layer. The plasma treatment was performed in nitrogen atmosphere or in an atmosphere comprising 98 vol.-% nitrogen and 2 vol.-% oxygen.

Then, matrix compound and the dopant were co-deposited in vacuum on the anode layer, to form a hole injection layer (HIL) having a thickness of 10 nm. The dopant was selected from compound of formula (I) or comparative compound. The percentage of compound of formula (I) and comparative compound in the HIL can be seen in Table 6.

Then, the same matrix compound was vacuum deposited on the HIL, to form a HTL having a thickness of 122 nm.

Then N-([1,1'-biphenyl]-4-yl)-9,9-diphenyl-N-(4-(triphenylsilyl)phenyl)-9H-fluoren-2-amine (CAS 1613079-70-1) was vacuum deposited on the HTL, to form an electron blocking layer (EBL) having a thickness of 5 nm.

Then 97 vol.-% H09 (Sun Fine Chemicals, Korea) as EML host and 3 vol.-% BD200 (Sun Fine Chemicals, Korea) as fluorescent blue emitter dopant were deposited on the EBL, to form a blue-emitting first emission layer (EML) with a thickness of 20 nm.

Then a hole blocking layer was formed with a thickness of 5 nm by depositing 2-(3'-(9,9-dimethyl-9H-fluoren-2-yl)-[1,1'-biphenyl]-3-yl)-4,6-diphenyl-1,3,5-triazine on the emission layer EML.

Then the electron transporting layer having a thickness of 31 nm was formed on the hole blocking layer by co-depositing 50 wt.-% 4'-(4-(4-(4,6-diphenyl-1,3,5-triazin-2-yl)phenyl)naphthalen-1-yl)-[1,1'-biphenyl]-4-carbonitrile and 50 wt.-% of LiQ.

Then an electron injection layer having a thickness of 2 nm was formed on the ETL by depositing Ytterbium.

Then Ag:Mg (90:10 vol.-%) was evaporated at a rate of 0.01 to 1 Å/s at $10^{-7}$ mbar to form a cathode layer with a thickness of 13 nm on the electron injection layer.

Then, Biphenyl-4-yl(9,9-diphenyl-9H-fluoren-2-yl)-[4-(9-phenyl-9H-carbazol-3-yl)phenyl]-amine was deposited on the cathode layer to form a capping layer with a thickness of 75 nm.

The OLED stack is protected from ambient conditions by encapsulation of the device with a glass slide. Thereby, a cavity is formed, which includes a getter material for further protection.

General procedure for fabrication of OLEDs wherein the organic semiconductor layer is a p-type charge generation layer (p-CGL)

For Example 2-1 to 2-3 in Table 7, a glass substrate with an anode layer comprising a first anode sub-layer of 120 nm Ag, a second anode sub-layer of 8 nm ITO and a third anode sub-layer of 10 nm ITO was cut to a size of 50 mm x 50 mm x 0.7 mm, ultrasonically washed with water for 60 minutes and then with isopropanol for 20 minutes. The liquid film was removed in a nitrogen stream, followed by plasma treatment, to prepare the anode layer. The plasma treatment was performed in nitrogen atmosphere or in an atmosphere comprising 98 vol.-% nitrogen and 2 vol.% oxygen.

Then, a hole injection layer (HIL) having a thickness of 10 nm is formed on the anode layer by co-depositing compound 98 wt.-% L3 and 2 wt.-% 2,2',2"-(cyclopropane-1,2,3-triylidene)tris(2-(p-cyanotetrafluorophenyl)acetonitrile) CC3.

Then, a first hole transport layer (HTL1) having a thickness of 29 nm is formed on the HIL by depositing L3.

Then, a first electron blocking layer (EBL1) having a thickness of 5 nm is formed on the HTL1 by depositing N,N-di([1,1'-biphenyl]-4-yl)-3'-(9H-carbazol-9-yl)-[1,1'-biphenyl]-4-amine.

Then, a first emission layer (EML1) having a thickness of 20 nm is formed on the EBL1 by co-depositing 97 vol.-% H09 (Sun Fine Chemicals, Korea) as EML host and 3 vol.-% BD200 (Sun Fine Chemicals, Korea) as fluorescent blue dopant.

Then, a first electron transport layer (ETL1) having a thickness of 5 nm is formed on the first emission layer by depositing 2,2'-(1,3-Phenylene)bis[9-phenyl-1,10-phenanthroline].

Then, the n-CGL having a thickness of 10 nm is formed on the ETL by co-depositing 99 vol.-% 2,2'-(1,3-Phenylene)bis[9-phenyl-1,10-phenanthroline] and 1 vol.-% Yb.

Then, the p-CGL having a thickness of 10 nm is formed on the n-CGL by co-depositing a matrix compound and a compound of formula (I). The composition of the p-CGL can be seen in Table 7.

Then, a second hole transport layer (HTL2) having a thickness of 41 nm is formed on the p-CGL by depositing L3.

Then, a second electron blocking layer (EBL2) having a thickness of 5 nm is formed on the HTL2 by depositing N,N-di([1,1'-biphenyl]-4-yl)-3'-(9H-carbazol-9-yl)-[1,1'-biphenyl]-4-amine.

Then, a second emission layer (EML2) having a thickness of 20 nm is formed on the EBL2 by co-depositing 97 vol.-% H09 (Sun Fine Chemicals, Korea) as EML host and 3 vol.-% BD200 (Sun Fine Chemicals, Korea) as fluorescent blue dopant.

Then, a hole blocking layer (HBL) having a thickness of 5 nm is formed on the EML2 by depositing 2-(3'-(9,9-dimethyl-9H-fluoren-2-yl)-[1,1'-biphenyl]-3-yl)-4,6-diphenyl-1,3,5-triazine.

Then, a second electron transport layer (ETL2) having a thickness of 31 nm is formed on the HBL by co-depositing 3'-(4-phenyl-6-(spiro[fluorene-9,9'-xanthen]-2'-yl)-1,3,5-triazin-2-yl)-[1,1'-biphenyl]-4-carbonitrile and LiQ in a ratio of 50:50 wt.-%.

Then, an electron injection layer (EIL) having a thickness of 2 nm is formed on the ETL2 by depositing Yb.

Then, the cathode layer having a thickness of 13 nm is formed on the EIL by co-depositing Ag:Mg (90:10 vol.-%) at a rate of 0.01 to 1 Å/s at $10^{-7}$ mbar.

Then, a capping layer having a thickness of 75 nm is formed on the cathode layer by depositing compound of formula L3.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/$m^2$ using an Instrument Systems CAS-140CT array spectrometer (calibrated by Deutsche Akkreditierungsstelle (DAkkS)) for each of the voltage values. The cd/A efficiency at 10 mA/cm2 is determined by interpolating the luminance-voltage and current-voltage characteristics, respectively.

In bottom emission devices, the emission is predominately Lambertian and quantified in percent external quantum efficiency (EQE). To determine the efficiency EQE in % the light output of the device is measured using a calibrated photodiode at 10 mA/cm2.

In top emission devices, the emission is forward directed, non-Lambertian and also highly dependent on the micro-cavity. Therefore, the efficiency EQE will be higher compared to bottom emission devices. To determine the efficiency EQE in % the light output of the device is measured using a calibrated photodiode at 10 mA/$cm^2$.

Lifetime LT of the device is measured at ambient conditions (20°C) and 30 mA/$cm^2$, using a Keithley 2400 source meter, and recorded in hours.

The brightness of the device is measured using a calibrated photo diode. The lifetime LT is defined as the time till the brightness of the device is reduced to 97 % of its initial value.

The voltage rise over time U(1-100h) is determined by measuring the operating voltage after one hour and after 100 hours at 30 mA/$cm^2$. The difference in operating voltage after 1 hour and after 100 hours provides an indication of the long-term stability of the organic electronic device. The lower the value U(1-100h) the higher the stability.

## Technical Effect of the invention

In Table 3 are shown the physical properties of compounds of formula (I) wherein more than one structural isomer is present.

In Table 4 are shown physical properties of the structural isomer of compound of formula (I) with the lowest total energy.

As can be seen in Table 3 and 4, the LUMO energy of compounds of formula (I) is in the range of -4.9 to -5.48 eV. Without being bound by theory, a LUMO energy in this range may be enable efficient electron transfer from the HOMO of the matrix compound to the LUMO of the compound of formula (I). Thereby, efficient hole injection and/or hole generation may be enabled in an organic electronic device.

As can be seen in Table 3 and 4, the dipole moment of compounds of formula (I) is in the range of > 0 to ≤ 7 Debye. Without being bound by theory, a dipole moment in this range may enable efficient hole injection and/or hole generation in an organic electronic device.

In Table 5 are shown further physical properties of compounds of formula (I) and comparative compounds 1 and 2.

As can be seen in Table 5, comparative compound 1 (CC-1) has a rate onset temperature of 170 °C.

Comparative compound 2 (CC-2) has a rate onset temperature of 180 °C. The TGA5% is 345 °C.

The rate onset temperature of compound of formula (I) H6 is improved to 254 °C compared to comparative compounds 1 and 2. Additionally, the TGA5% is improved to 406 °C compared to comparative compound 2.

A high rate onset temperature may be desirable for good control of the evaporation rate in mass production of organic electronic devices.

A high TGA5% may be desirable for improved thermal stability.

As comparative compound CC-1 could not be processed, data are presented for an organic electronic device comprising compound of formula (I) or comparative compound CC-2, see Table 6.

In comparative examples 1-1 to 1-3, the hole injection layer comprises matrix compound L3 and comparative compound 2 (CC-2) as dopant. The HOMO energy of L3 is -4.69 eV.

In comparative example 1-1, the hole injection layer comprises 3 wt.-% comparative compound 2 (CC-2). As can be seen in Table 6, the operating voltage is 4.01 V, the efficiency is 5.81 cd/A, the external quantum efficiency (EQE) is 12.65 %. The lifetime could not be determined due to the very high voltage rise over time U(1-100h) of 0.489 V.

In comparative examples 1-2 and 1-3, the hole injection layer comprises comparative compound 2 (CC-2) at

a higher concentration. As can be seen in Table 6, the operating voltage is improved to 3.94 and 3.92 V, respectively. The cd/A efficiency is reduced compared to comparative example 1-1. The lifetime is 126 and 120 hours, respectively. The voltage rise over time U(1-100h) is improved to 0.073 and 0.032 V, respectively.

In Example 1-1, the hole injection layer comprises the same matrix compound as in comparative examples 1-1 to 1-3. Compound of formula (I) H6 has been used instead of comparative compound 1-2 as dopant. As can be seen in Table 6, the operating voltage is improved to 3.73 V. The efficiency is improved to 6.28 cd/A and 13.79 % EQE. The lifetime is still high at 115 h and the voltage rise over time is 0.075 V.

In Example 1-2, the concentration of compound of formula (I) H6 has been increased from 2 to 3 wt.-%. As can be seen in Table 6, the operating voltage, efficiency and lifetime are substantially unchanged compared to Example 1-1. The voltage rise over time further improved to 0.033 V compared to Example 1-1.

In Example 1-3, the hole injection layer comprises matrix compound L18. As can be seen in Table 6, the HOMO energy of L18 is further away from vacuum level compared to the HOMO energy of L3. As can be seen in Table 6, the efficiency is further improved to 6.93 cd/A and 14.95 % EQE.

In Example 1-4, the concentration of compound of formula (I) has been increased from 3 to 4 wt.-%. The efficiency is still high, while the voltage rise over time has been improved to 0.051 V compared to Example 1-3. Surprisingly, the operating voltage and EQE are improved over comparative examples 1-1 to 1-3.

In Examples 1-5 to 1-7, the hole injection layer comprises matrix compound L10. As can be seen in Table 6, the HOMO energy of L10 is further away from vacuum level compared to the HOMO energy of L3 and L18. Surprisingly, the operating voltage and EQE are improved over comparative examples 1-1 to 1-3. As can be seen in Table 6, the lifetime and voltage rise over time have been improved further compared to Examples 1-1 to 1-4, and also compared to comparative examples 1-1 to 1-3.

In Examples 1-8 and 1-9, the hole injection layer comprises matrix compound L4. As can be seen in Table 6, the HOMO energy of L4 is further away from vacuum level compared to the HOMO energy of L3 and L18. Surprisingly, the operating voltage, cd/A efficiency, EQE and voltage rise over time are improved over comparative examples 1-1 to 1-3.

In Table 7 are shown data for an organic electroluminescent device comprising an organic semiconductor layer comprising compound of formula (I), wherein the organic semiconductor layer is a p-type charge generation layer (p-CGL).

In Example 2-1, the organic semiconductor layer comprises 6 wt.-% compound of formula (I) H6. The operating voltage is 7.63 V, the cd/A efficiency is 13.62 cd/A, the external quantum efficiency EQE is 23.3 %, the lifetime is 110 hours and the voltage rise over time U(1-100h) is 0.416 V. In Examples 2-2 and 2-3, a higher concentration of compound of formula (I) was used. The operating voltage, lifetime and voltage rise over time are improved compared to Example 2-1.

In summary, an improvement in operating voltage, efficiency, lifetime and/or voltage rise over time may be obtained.

A low operating voltage and/or high efficiency may result in reduced power consumption, in particular in mobile electronic devices.

A long lifetime and/or low voltage rise over time may result in improved long-term stability of electronic devices.

Table 3: Physical properties of compounds of formula (I), wherein the "*" denotes the binding position

| Name | $R^1$ | $R^2$ | $R^3$ | $R^4$ | R' | R" | LUMO (eV) | Dipole moment (Debye) |
|---|---|---|---|---|---|---|---|---|
| Comparative compound 1 (CC-1) | CN | F | F | CN | 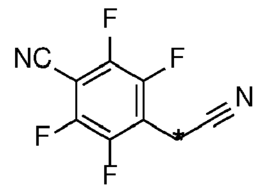 | 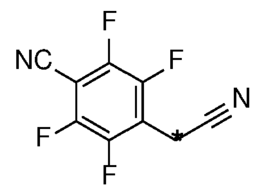 | -5.17 to -5.21 | 0.01 to 0.35 |
| G1 | F | F | F | F | 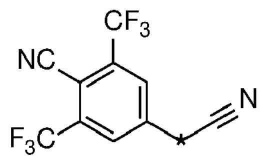 | 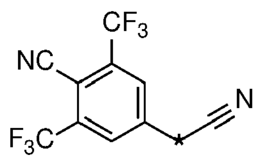 | -4.91 to -4.93 | 0.05 to 2.9 |
| G2 | F | F | F | F | 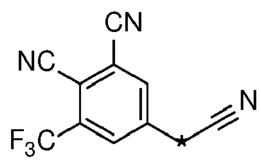 | 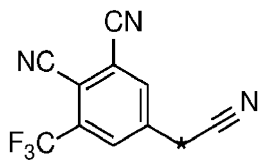 | -5.08 | 3.43 to 4.61 |
| G3 | Cl | F | F | Cl | 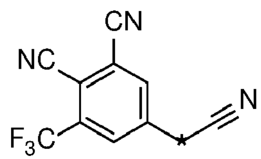 | 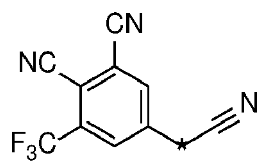 | -5.1 to - 5.16 | 0.28 to 5.43 |
| G4 | $CF_3$ | F | F | $CF_3$ | 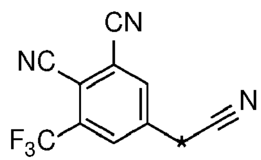 | 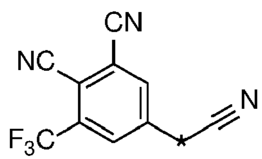 | -5.11 to -5.25 | 3.39 to 4.88 |
| G5 | CN | CN | F | F | 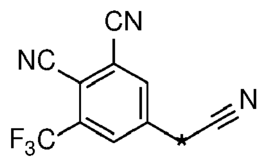 | 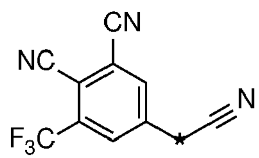 | -5.35 to - 5.4 | 1.11 to 5.15 |

(continued)

| Name | $R^1$ | $R^2$ | $R^3$ | $R^4$ | R' | R" | LUMO (eV) | Dipole moment (Debye) |
|---|---|---|---|---|---|---|---|---|
| G6 | CN | F | F | CN | 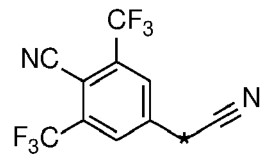 | 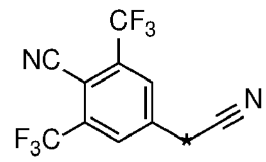 | -5.25 to -5.32 | 0.02 to 2.22 |
| G7 | CN | F | F | CN | 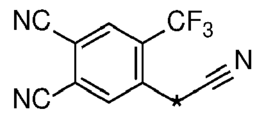 | 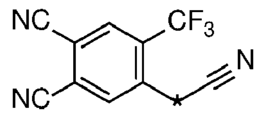 | -5.10 to -5.12 | 0.02 to 3.57 |
| G8 | CN | F | F | CN | 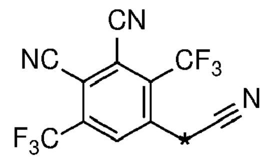 | 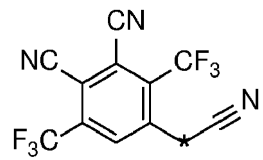 | -5.23 to -5.26 | 0.04 to 4.15 |
| G9 | CN | F | F | CN | 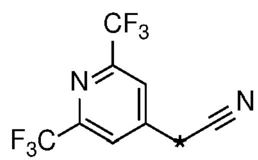 | 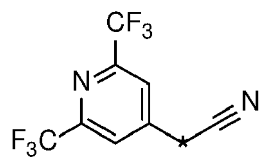 | -5.20 to -5.31 | 0.004 to 0.61 |
| G10 | CN | F | F | CN | 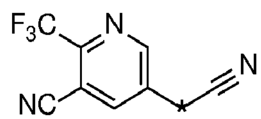 | 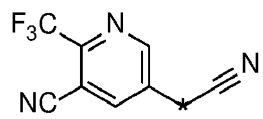 | -5.23 to -5.32 | 0.18 to 1.79 |
| G11 | CN | F | F | CN | 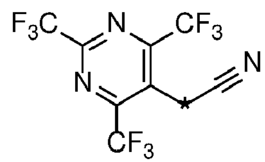 | 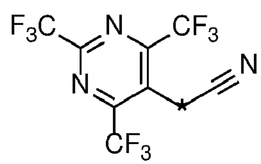 | -5.08 | 0.12 to 1.84 |

(continued)

| Name | $R^1$ | $R^2$ | $R^3$ | $R^4$ | R' | R" | LUMO (eV) | Dipole moment (Debye) |
|---|---|---|---|---|---|---|---|---|
| G12 | CN | F | F | CN | 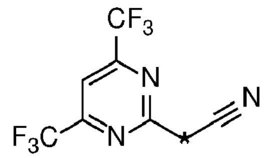 | 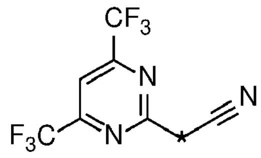 | -4.99 to -5.02 | 0.04 to 6.68 |
| G13 | CN | F | F | CN | 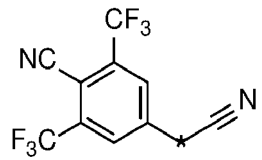 | 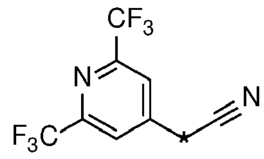 | -5.23 to -5.26 | 0.9 to 1.51 |
| G14 | F | F | F | F | 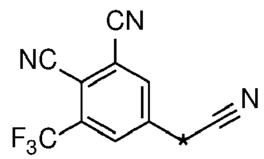 | 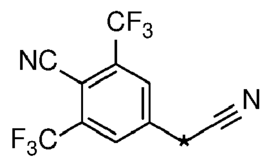 | -5.0 to -5.01 | 2.35 to 3.55 |
| G15 | CN | F | F | CN | 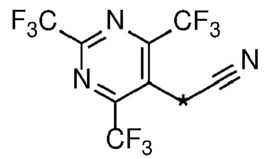 | 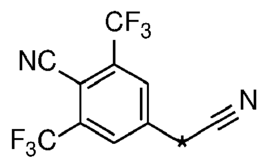 | -5.12 to -5.18 | 1.33 to 2.39 |
| G16 | $CF_3$ | CN | CN | $CF_3$ | 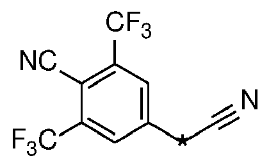 | 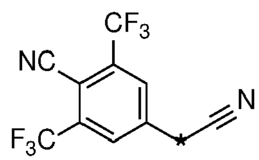 | -5.07 to -5.08 | 2.4 to 3.54 |

(continued)

| Name | $R^1$ | $R^2$ | $R^3$ | $R^4$ | R' | R" | LUMO (eV) | Dipole moment (Debye) |
|---|---|---|---|---|---|---|---|---|
| G17 | F | F | F | F | 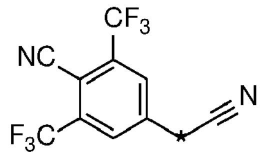 | 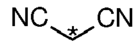 | -5.28 | 2.22 |
| G18 | CN | F | CN | F | 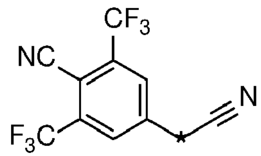 | 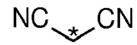 | -5.43 to -5.48 | 1.48 to 1.53 |
| G19 | CN | F | CN | F | 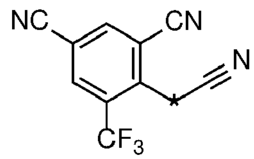 | 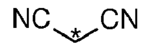 | -5.32 to -5.33 | 3.77 to 3.78 |

Table 4: Physical properties of compounds of formulas (I)

| Name | Chemical formula | LUMO (eV) | Dipole moment (Debye) |
|---|---|---|---|
| H1 | 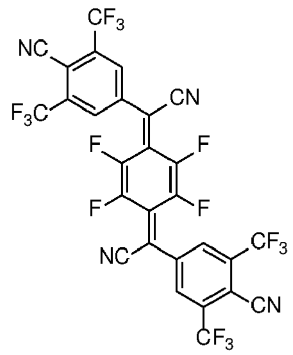 | -4.91 | 0.05 |
| H2 | 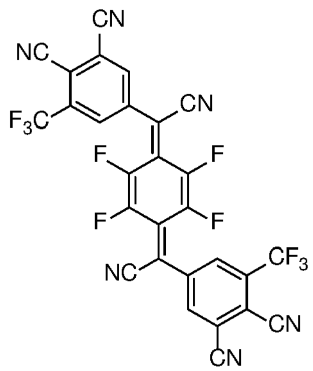 | -5.08 | 3.43 |
| H3 | 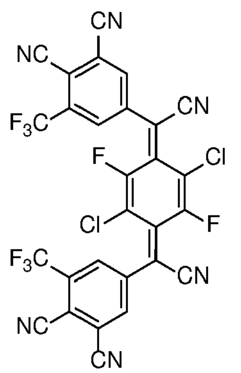 | -5.1 | 5.43 |
| H4 | 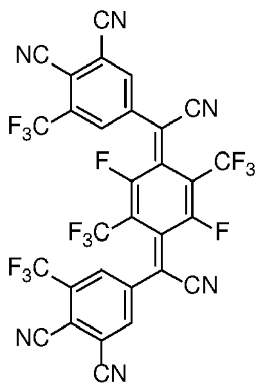 | -5.11 | 5.43 |

(continued)

| Name | Chemical formula | LUMO (eV) | Dipole moment (Debye) |
|---|---|---|---|
| H5 | 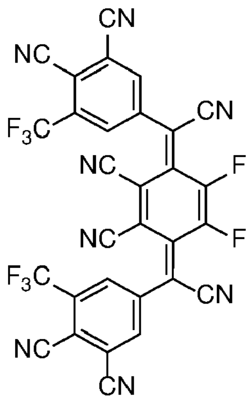 | -5.37 | 5.15 |
| H6 | 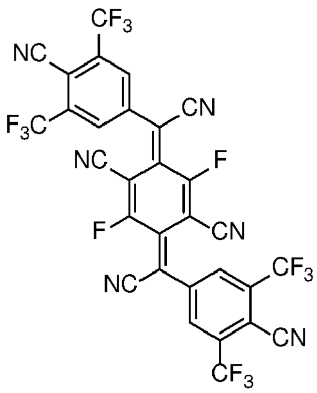 | -5.25 | 0.02 |
| H7 | 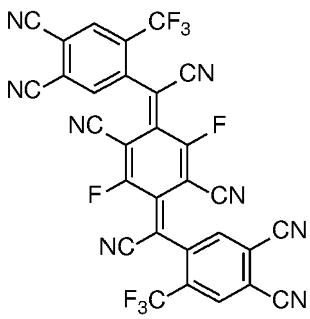 | -5.10 | 0.02 |
| H8 | 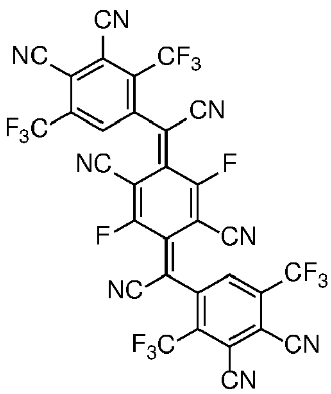 | -5.23 | 0.04 |

(continued)

| Name | Chemical formula | LUMO (eV) | Dipole moment (Debye) |
|---|---|---|---|
| H9 | 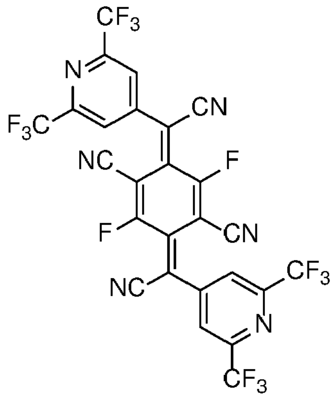 | --5.20 | 0.14 |
| H10 | 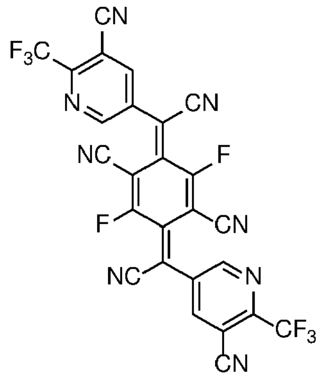 | -5.23 | 0.34 |
| H11 | 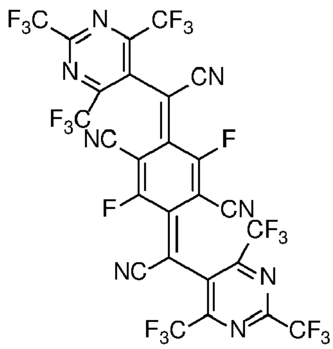 | -5.08 | 0.19 |
| H12 | 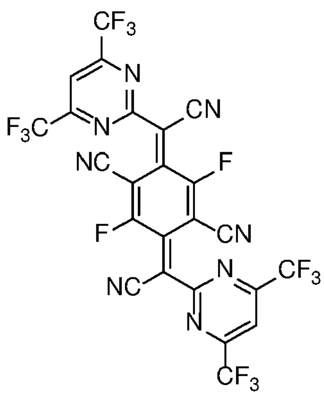 | -4.99 | 0.04 |

(continued)

| Name | Chemical formula | LUMO (eV) | Dipole moment (Debye) |
|---|---|---|---|
| H13 | 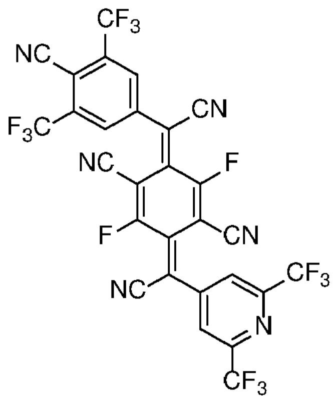 | -5.23 | 0.90 |
| H14 | 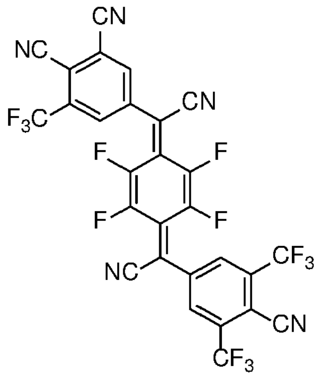 | -5.00 | 2.35 |
| H15 | 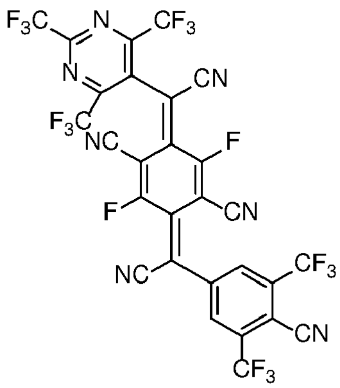 | -5.13 | 2.39 |
| H16 | 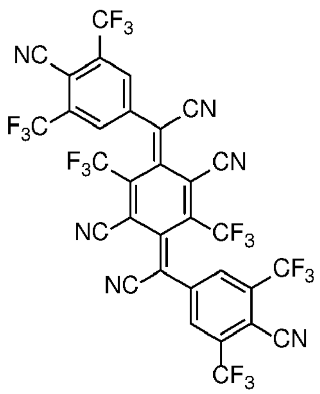 | -5.08 | 2.40 |

(continued)

| Name | Chemical formula | LUMO (eV) | Dipole moment (Debye) |
|---|---|---|---|
| H17 | 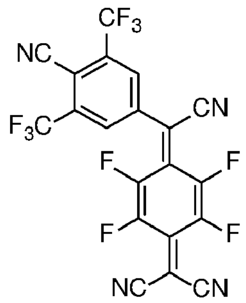 | -5.28 | 2.22 |
| H18 | 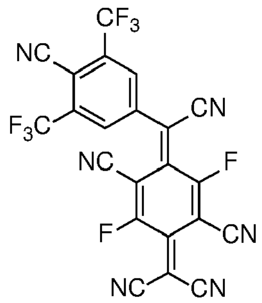 | -5.43 | 1.53 |
| H19 | 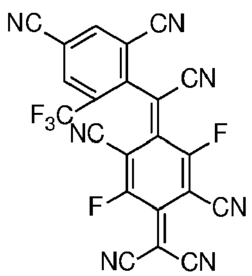 | -5.33 | 3.78 |

Table 5: Physical properties of compounds of formulas (I) and comparative compounds 1 and 2

| Name | Chemical formula | LUMO (eV) | Dipole moment (Debye) | $T_m$ (°C) | $T_g$ (°C) | TGA5% (°C) | $T_{RO}$ (°C) |
|---|---|---|---|---|---|---|---|
| Comparative compound 1 (CC-1) | 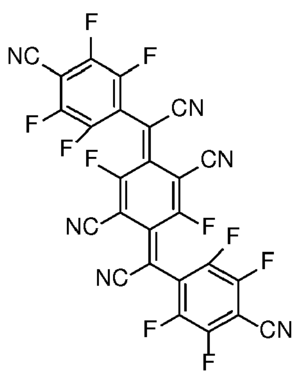 | -5.21 | 0.45 | n.d. 1) | n.d. | n.d. | 170 |

(continued)

| Name | Chemical formula | LUMO (eV) | Dipole moment (Debye) | $T_m$ (°C) | $T_g$ (°C) | TGA5% (°C) | $T_{RO}$ (°C) |
|---|---|---|---|---|---|---|---|
| Comparative compound 2 (CC-2) | 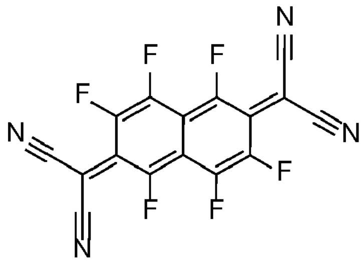 | -5.29 | 0 | 370 | n.o. 2) | 345 | 180 |
| H6 | 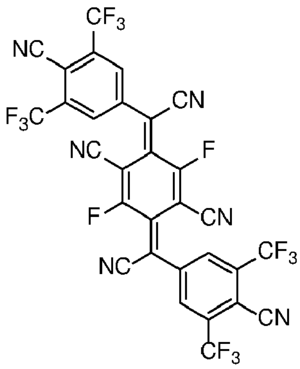 | -5.25 | 0.02 | n.o. | n.o. | 406 | 254 |
| 1) n.d. = not determined. 2) n.o. = not observed | | | | | | | |

Table 6: Data for organic electroluminescent devices comprising a hole injection layer comprising compound of formula (I) or comparative compound

| | Matrix compound | HOMO energy matrix compound [eV] | Percentage matrix compound [wt.-%] | Dopant | Percentage dopant [wt.-%] | Voltage at 10mA/cm2 [V] | Cd/A efficiency at 10mA/cm2 [cd/A] | EQE at 10mA/cm2 [%] | LT97 at 30mA/cm2 [h] | U (1-100h) at 30mA/cm2 [V] |
|---|---|---|---|---|---|---|---|---|---|---|
| Comparative example 1-1 | L3 | -4.69 | 97 | CC-2 | 3 | 4.01 | 5.81 | 12.65 | n.d. 1) | 0.489 |
| Comparative example 1-2 | L3 | -4.69 | 92 | CC-2 | 8 | 3.94 | 5.65 | 12.65 | 126 | 0.073 |
| Comparative example 1-3 | L3 | -4.69 | 88 | CC-2 | 12 | 3.92 | 5.53 | 12.48 | 120 | 0.032 |
| Example 1-1 | L3 | -4.69 | 98 | H6 | 2 | 3.73 | 6.28 | 13.79 | 115 | 0.075 |
| Example 1-2 | L3 | -4.69 | 97 | H6 | 3 | 3.72 | 6.29 | 13.80 | 116 | 0.033 |
| Example 1-3 | L18 | -4.73 | 97 | H6 | 3 | 3.73 | 6.93 | 14.95 | 88 | 0.076 |
| Example 1-4 | L18 | -4.73 | 96 | H6 | 4 | 3.72 | 6.86 | 14.88 | 87 | 0.051 |
| Example 1-5 | L10 | -4.84 | 92 | H6 | 8 | 3.65 | 5.57 | 12.86 | 184 | 0.091 |
| Example 1-6 | L10 | -4.84 | 90 | H6 | 10 | 3.62 | 5.61 | 12.94 | 157 | 0.033 |
| Example 1-7 | L10 | -4.84 | 88 | H6 | 12 | 3.61 | 5.55 | 12.88 | 155 | 0.022 |
| Example 1-8 | L4 | -4.85 | 95 | H6 | 5 | 3.68 | 6.25 | 13.79 | 122 | 0.038 |
| Example 1-9 | L4 | -4.85 | 93 | H6 | 7 | 3.66 | 6.23 | 13.74 | 122 | 0.018 |

1) n.d. = not determined

Table 7: Data for organic electroluminescent devices comprising a p-type charge generation layer comprising compound of formula (I)

| | Matrix compound | HOMO energy matrix compound [eV] | Percentage matrix compound [wt.-%] | Dopant | Percentage dopant [wt.-%] | Voltage at $10mA/cm^2$ [V] | Cd/A efficiency at $10mA/cm^2$ [cd/A] | EQE at $10mA/cm^2$ [%] | LT97 at $30mA/cm^2$ [h] | U (1-100h) at $30mA/cm^2$ [V] |
|---|---|---|---|---|---|---|---|---|---|---|
| Example 2-1 | L3 | -4.69 | 94 | H6 | 6 | 7.63 | 13.62 | 23.30 | 110 | 0.416 |
| Example 2-2 | L3 | -4.69 | 91.5 | H6 | 8.5 | 7.40 | 13.53 | 23.22 | 124 | 0.320 |
| Example 2-3 | L3 | -4.69 | 89 | H6 | 11 | 7.31 | 13.51 | 23.21 | 126 | 0.302 |

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims and the equivalents thereto. Furthermore, reference signs used in the description and claims do not limit the scope of the invention as claimed.

## Claims

1. A compound of formula (I):

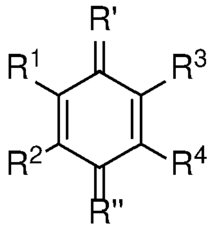

(I),

wherein

$R^1$, $R^2$, $R^3$ and $R^4$ are independently selected from the group comprising F, Cl, CN or $CF_3$;
R' is selected from formula (II)

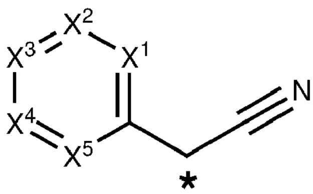

(II);

wherein

$X^1$ is selected from $CR^6$ or N;
$X^2$ is selected from $CR^7$ or N;
$X^3$ is selected from $CR^8$ or N;
$X^4$ is selected from $CR^9$ or N;
$X^5$ is selected from $CR^{10}$ or N; wherein

$R^6$, $R^7$, $R^8$, $R^9$ and $R^{10}$ are independently selected from CN, partially fluorinated or perfluorinated $C_1$ to $C_8$ alkyl, halogen, Cl, F, D or H,

wherein at least one $X^1$ to $X^5$ is selected from CH, CD or N;
R" is selected from formula (III)

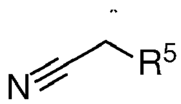

(III);

wherein

$R^5$ is selected from substituted or unsubstituted $C_6$ to $C_{19}$ aryl, substituted or unsubstituted $C_6$ to $C_{19}$ aryl with one, two or three aromatic six-member rings, substituted or unsubstituted $C_9$ to $C_{19}$ aryl with two or three fused aromatic six-member rings, substituted or unsubstituted $C_6$ to $C_{12}$ aryl with one or two aromatic six-member rings, substituted or unsubstituted phenyl, substituted or unsubstituted biphenyl, substituted or unsubstituted $C_3$ to $C_{20}$ heteroaryl, substituted or unsubstituted $C_3$ to $C_{20}$ heteroaryl with one, two or three aromatic six-member rings, substituted or unsubstituted $C_7$ to $C_{20}$ heteroaryl with two or three fused aromatic six-member rings or CN,

wherein the substituents are selected from CN, partially fluorinated or perfluorinated $C_1$ to $C_8$ alkyl, halogen, Cl, F, $C_1$ to $C_8$ alkyl or D;
wherein the "*" denotes the binding position; and optional the following formula (E1) is excluded:

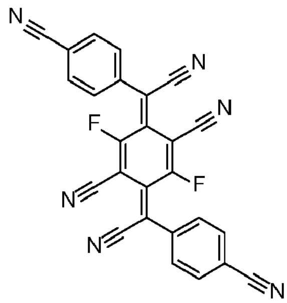 (E1).

2. The compound of formula (I) according of claim 1, wherein the compound of formula (I) is represented by formula (Ia), (Ib), (Ic) and (Id):

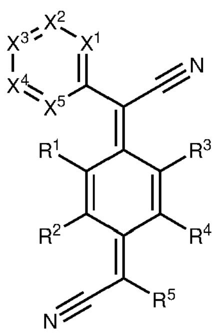 (Ia), 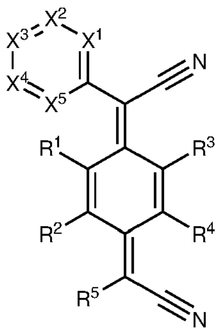 (Ib), 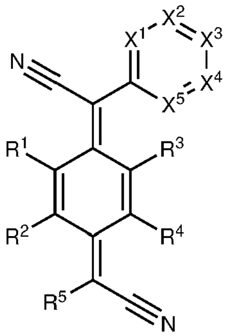 (Ic), 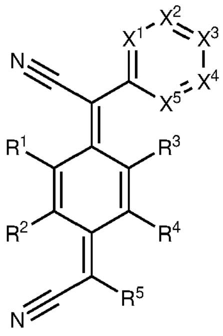 (Id),

wherein

$R^1$ to $R^4$ are independently selected from F, Cl, CN or $CF_3$;
$R^5$ is selected from substituted or unsubstituted $C_6$ to $C_{19}$ aryl, substituted or unsubstituted $C_3$ to $C_{20}$ heteroaryl or CN, wherein the substituent on $R^5$ is selected from CN, partially fluorinated or perfluorinated $C_1$ to Cs alkyl, halogen, Cl, F, $C_1$ to $C_8$ alkyl or D;
$X^1$ is selected from $CR^6$ or N;
$X^2$ is selected from $CR^7$ or N;
$X^3$ is selected from $CR^8$ or N;
$X^4$ is selected from $CR^9$ or N;
$X^5$ is selected from $CR^{10}$ or N; wherein

$R^6$, $R^7$, $R^8$, $R^9$ and $R^{10}$ are independently selected from CN, partially fluorinated or perfluorinated $C_1$ to $C_8$ alkyl, halogen, Cl, F, D or H,

wherein at least one $X^1$ to $X^5$ is selected from CH, CD or N; and
the following formula (E1) is optional excluded:

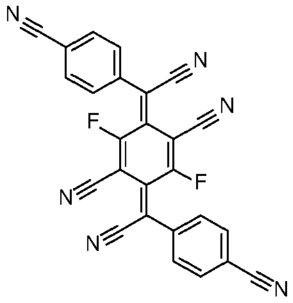

(E1).

3. The compound of formula (I) according of claim 1 or 2, wherein $R^1$, $R^2$, $R^3$ and $R^4$ are independently selected from F or CN, preferably two of $R^1$, $R^2$, $R^3$ and $R^4$ are selected from F or CN and the other two $R^1$, $R^2$, $R^3$ and $R^4$ are selected from F.

4. The compound of formula (I) according to any of the preceding claims 1 to 3, wherein

- $R^1$ and $R^4$ are selected from F and $R^2$ and $R^3$ are selected from F or CN; or
- $R^1$ and $R^4$ are selected from F and $R^2$ and $R^3$ are selected from CN; or
- $R^1$ and $R^4$ are selected from F and $R^2$ and $R^3$ are selected from F; or
- $R^1$ and $R^4$ are selected from F or CN and $R^2$ and $R^3$ are selected from F; or
- $R^1$ and $R^4$ are selected from CN and $R^2$ and $R^3$ are selected from F.

5. The compound of formula (I) according to any of the preceding claims 1 to 4, wherein $\geq 0$ and $\leq 3$ of $X^1$, $X^2$, $X^3$, $X^4$ and $X^5$ are selected from N.

6. The compound of formula (I) according to any of the preceding claims 1 to 5, wherein at least two $X^1$ to $X^5$ are selected from CH, CD or N.

7. The compound of formula (I) according to any of the preceding claims 1 to 6, wherein formula (II) and/or formula (III) are independently selected from the group comprising of A1 to A142:

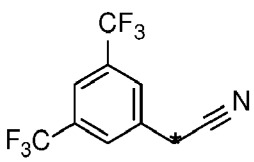

(A1),

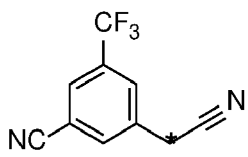

(A2),

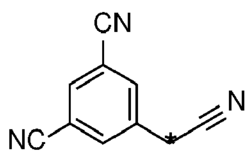

(A3),

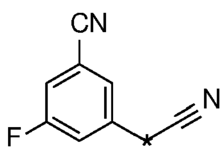

(A4),

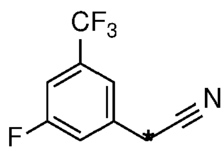

(A5),

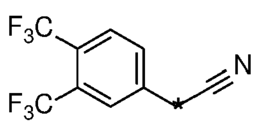

(A6),

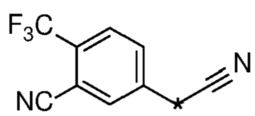

(A7),

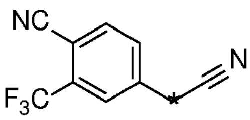

(A8),

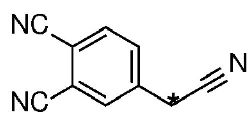

(A9),

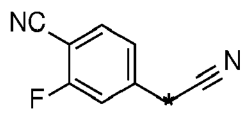

(A10),

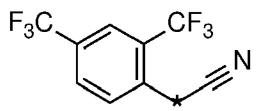
(A11),
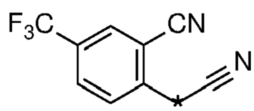
(A12),
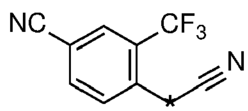
(A13),

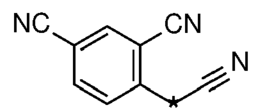
(A14),
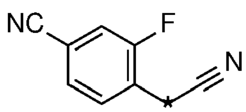
(A15),
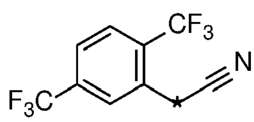
(A16),

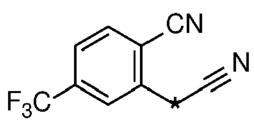
(A17),
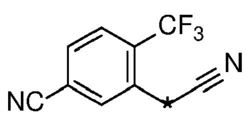
(A18),
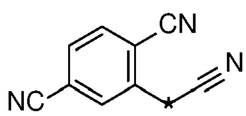
(A19),

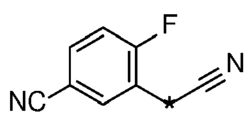
(A20),
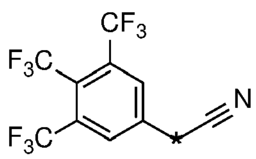
(A21),
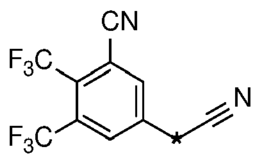
(A22),

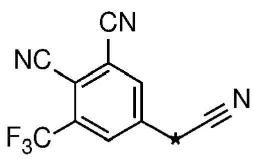
(A23),
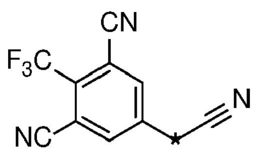
(A24),
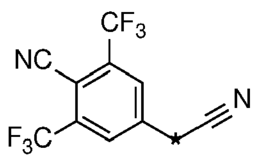
(A25),

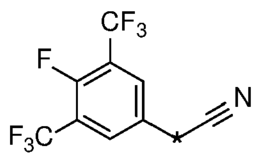
(A26),
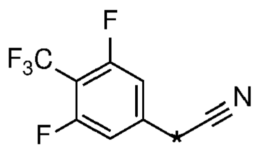
(A27),
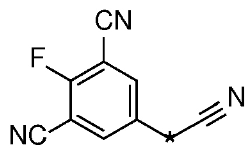
(A28),

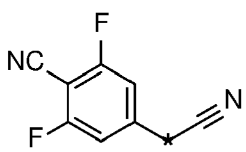
(A29),
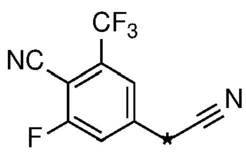
(A30),
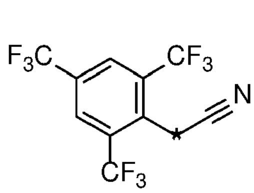
(A31),

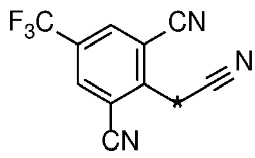 (A32), 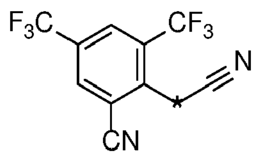 (A33), 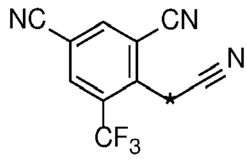 (A34),

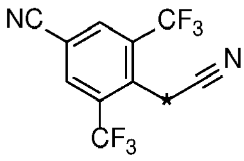 (A35), 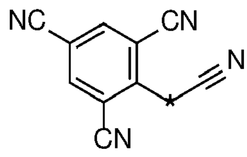 (A36), 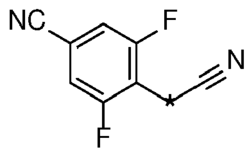 (A37),

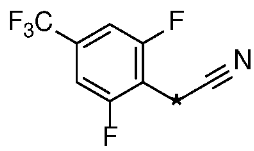 (A38), 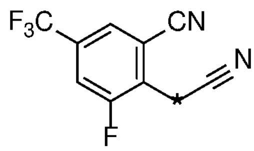 (A39), 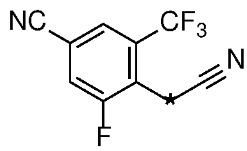 (A40),

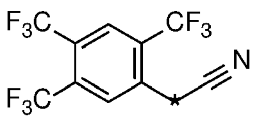 (A41), 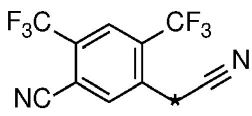 (A42), 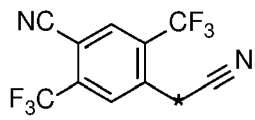 (A43),

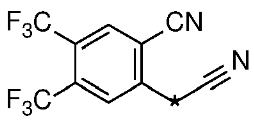 (A44), 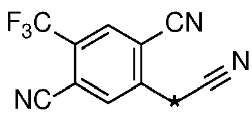 (A45), 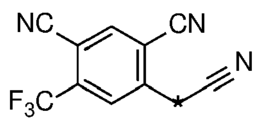 (A46),

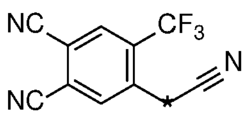 (A47), 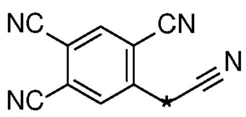 (A48), 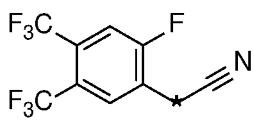 (A49),

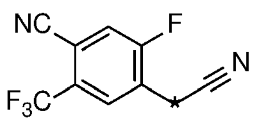 (A50), 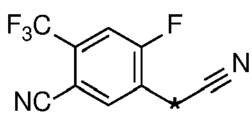 (A51), 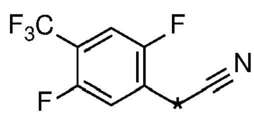 (A52),

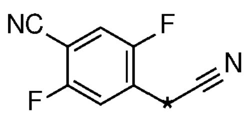 (A53), 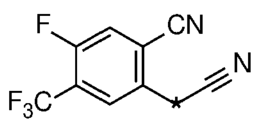 (A54), 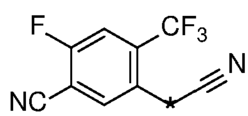 (A55),

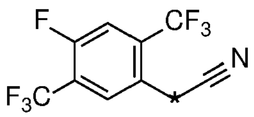 (A56), 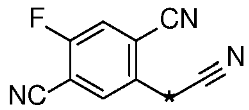 (A57), 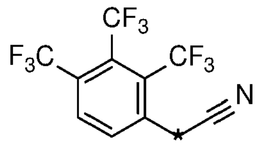 (A58),

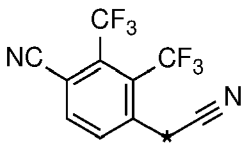 (A59), 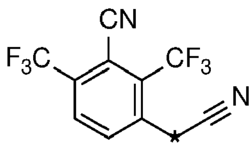 (A60), 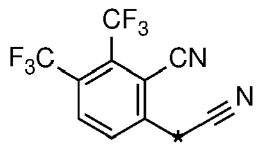 (A61),

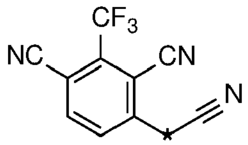 (A62), 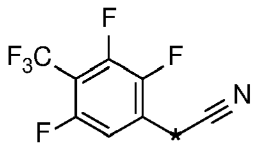 (A63), 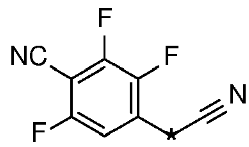 (A64),

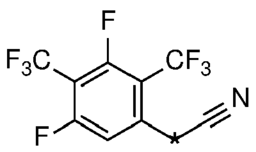 (A65), 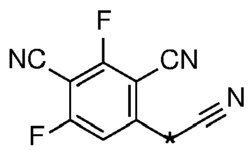 (A66), 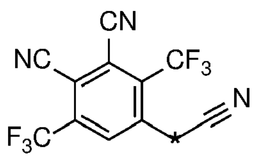 (A67),

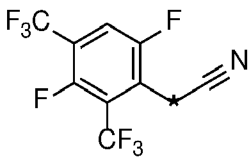 (A68), 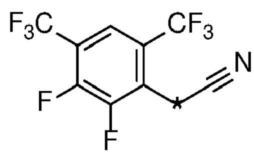 (A69), 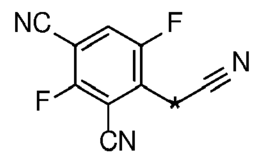 (A70),

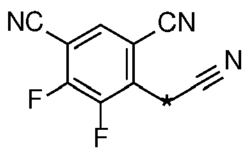 (A71), 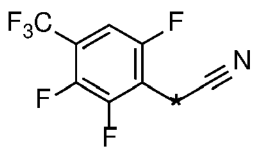 (A72), 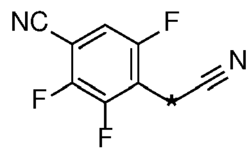 (A73),

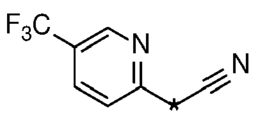 (A74), 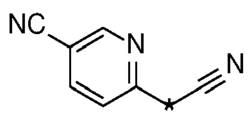 (A75), 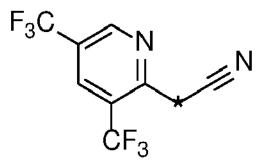 (A76),

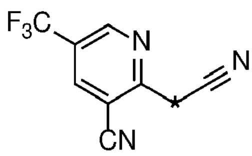 (A77), 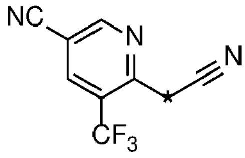 (A78), 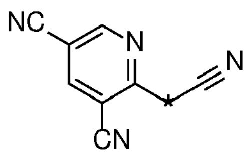 (A79),

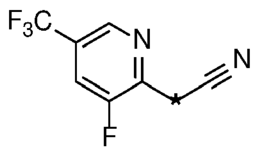 (A80), 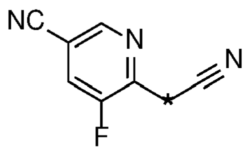 (A81), 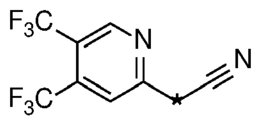 (A82),

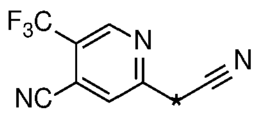 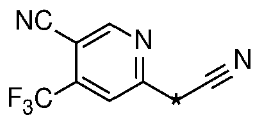

(A83), (A84),

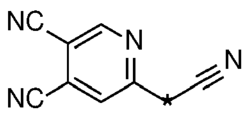 (A85), 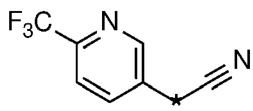 (A86), 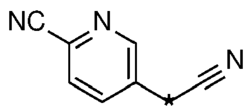 (A87),

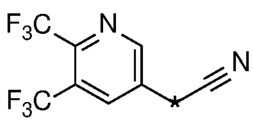 (A88), 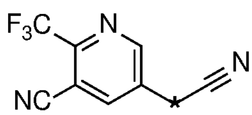 (A89), 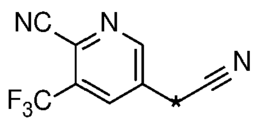 (A90),

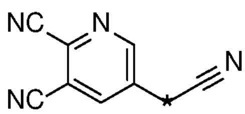 (A91), 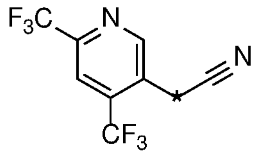 (A92), 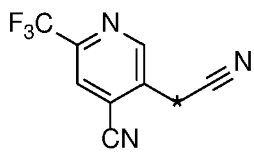 (A93),

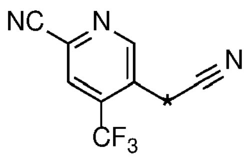 (A94), 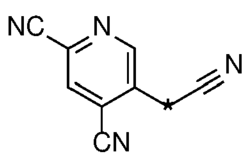 (A95), 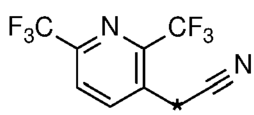 (A96),

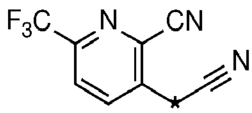 (A97), 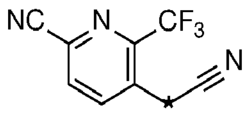 (A98), 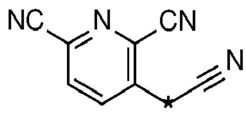 (A99),

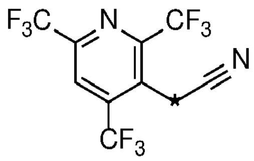 (A100), 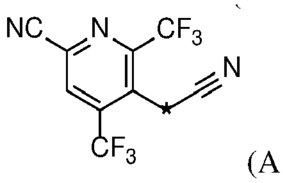 (A101), 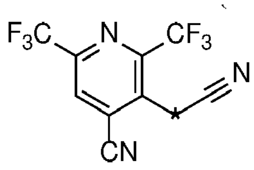 (A102),

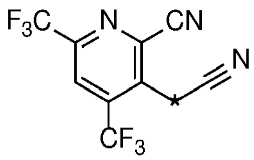 (A103), 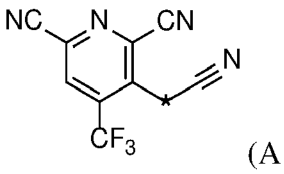 (A104), 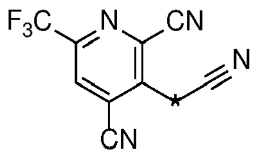 (A105),

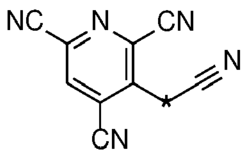 (A106), 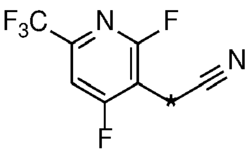 (A107), 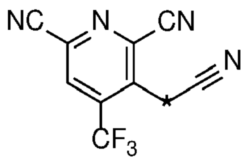 (A108),

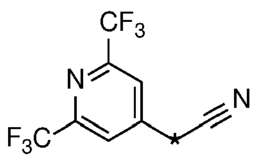 (A109), 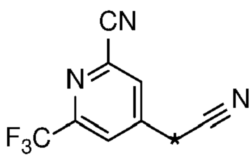 (A110), 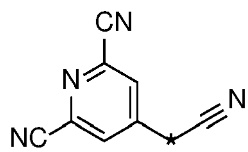 (A111),

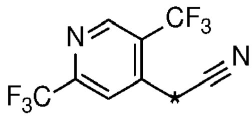 (A112), 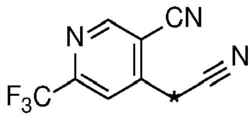 (A113), 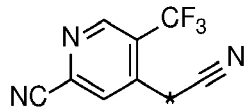 (A114),

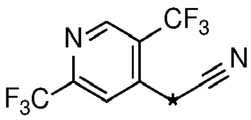 (A115), 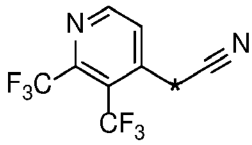 (A116), 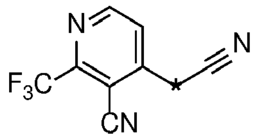 (A117),

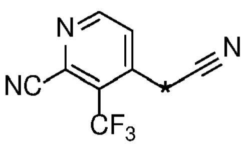 (A118), 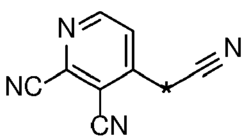 (A119), 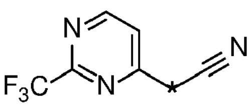 (A120),

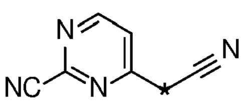 (A121), 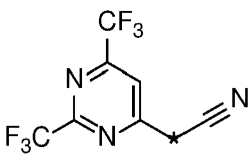 (A122), 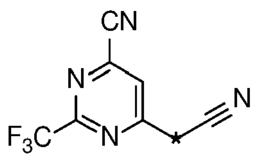 (A123),

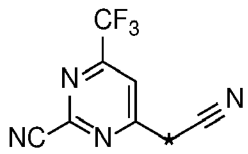
(A124),

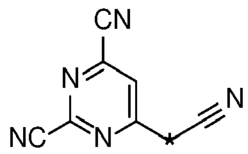
(A125),

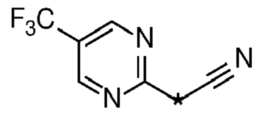
(A126),

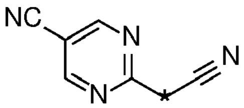
(A127),

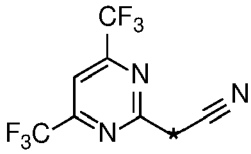
(A128),

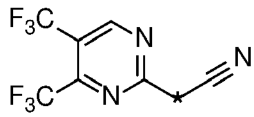
(A129),

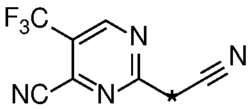
(A130),

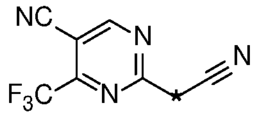
(A131),

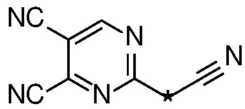
(A132),

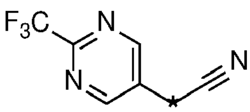
(A133),

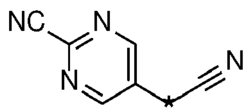
(A134),

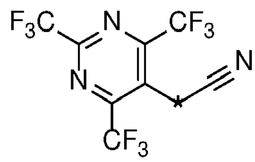
(A135),

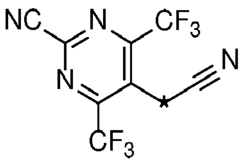
(A136),

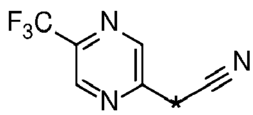
(A137),

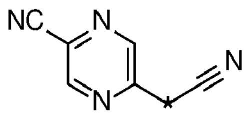
(A138),

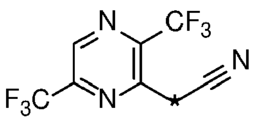
(A139),

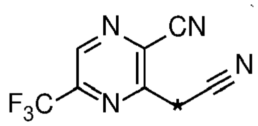
(A140),

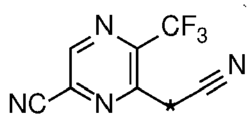
(A141),

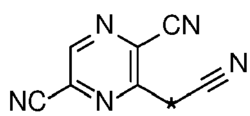
(A142) ;

wherein the "*" denotes the binding position.

**8.** The compound of formula (I) according to any of the preceding claims 1 to 7, wherein formula (III) is selected from the group comprising of A1 to A143, wherein A1 to A142 are defined according to claim 7 and formula A143 has the following formula:

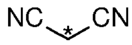

(A143);

wherein the "*" denotes the binding position.

**9.** The compound of formula (I) according to any of the preceding claims 1 to 7, wherein $R^5$ has the formulae (IVa) or (IVb), formula (IVa)

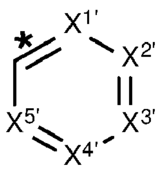

(IVa),

wherein

$X^{1'}$ is selected from $CR^{6'}$ or N;
$X^{2'}$ is selected from $CR^{7'}$ or N;
$X^{3'}$ is selected from $CR^{8'}$ or N;
$X^{4'}$ is selected from $CR^{9'}$ or N;
$X^{5'}$ is selected from $CR^{10'}$ or N;
wherein

$R^{6'}$, $R^{7'}$, $R^{8'}$, $R^{9'}$ and $R^{10'}$ are independently selected from CN, partially fluorinated or perfluorinated $C_1$ to $C_8$ alkyl, halogen, Cl, F, D, H, substituted or unsubstituted $C_6$ to $C_{13}$ aryl or substituted or unsubstituted $C_3$ to $C_{14}$ heteroaryl, preferably CN, F, $CF_3$, substituted or unsubstituted $C_6$ aryl or substituted or unsubstituted $C_3$ to $C_5$ heteroaryl;

wherein the substituent on substituted $C_6$ to $C_{13}$ aryl, substituted $C_3$ to $C_{14}$ heteroaryl, substituted or unsubstituted $C_6$ aryl, substituted or unsubstituted $C_3$ to $C_5$ heteroaryl is selected from CN, partially fluorinated or perfluorinated $C_1$ to $C_8$ alkyl, halogen, Cl, F, $C_1$ to $C_8$ alkyl or D, preferably CN, F, $CF_3$;
or
formula (IVb)

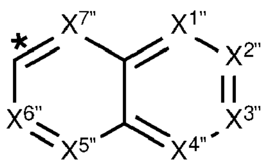

(IVb),

wherein

$X^{1''}$ is selected from $CR^{6''}$ or N;
$X^{2''}$ is selected from $CR^{7''}$ or N;
$X^{3''}$ is selected from $CR^{8''}$ or N;
$X^{4''}$ is selected from $CR^{9''}$ or N;
$X^{S''}$ is selected from $CR^{10''}$ or N;
$X^{6''}$ is selected from $CR^{11''}$ or N;
$X^{7''}$ is selected from $CR^{12''}$ or N;

wherein

$R^{6''}$ , $R^{7''}$, $R^{8''}$, $R^{9''}$, $R^{10''}$, $R^{11''}$ and $R^{12''}$ are independently selected from CN, partially fluorinated or perfluorinated $C_1$ to $C_8$ alkyl, halogen, Cl, F, D or H, preferably CN, F or $CF_3$;

wherein the "*" denotes the binding position.

**10.** The compound of formula (I) according to any of the preceding claims 1 to 8, wherein formula (IVa) is selected from the group comprising of D1 to D170:

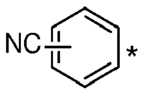 (D1), 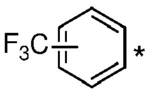 (D2), 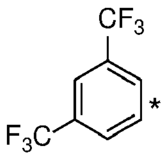 (D3), 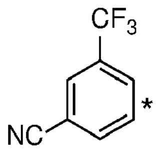 (D4), 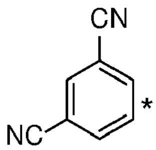 (D5),

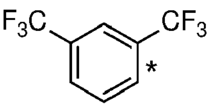 (D6), 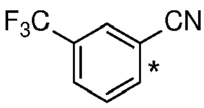 (D7), 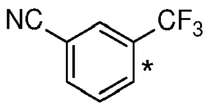 (D8), 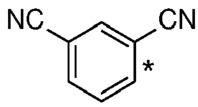 (D9),

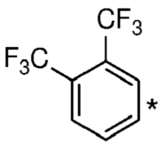 (D10), 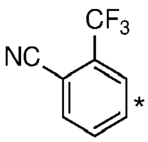 (D11), 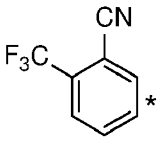 (D12), 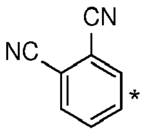 (D13), 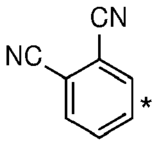 (D14),

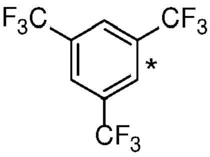 (D15), 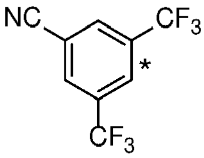 (D16), 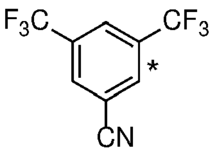 (D17), 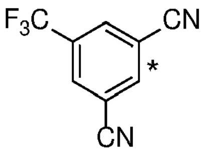 (D18),

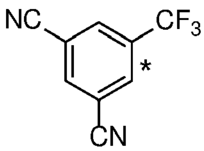 (D19), 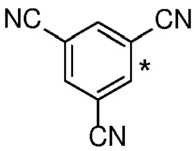 (D20), 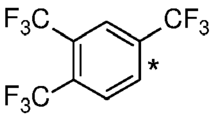 (D21), 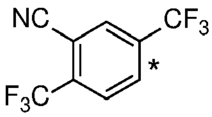 (D22),

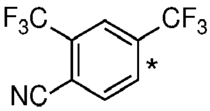 (D23), 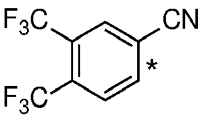 (D24), 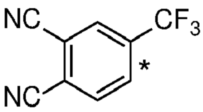 (D25), 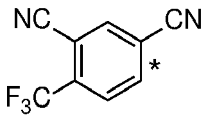 (D26),

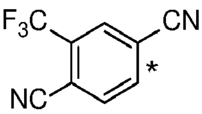 (D27), 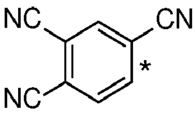 (D28), 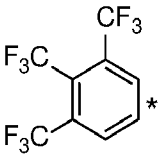 (D29), 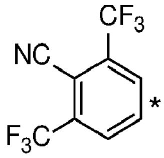 (D30),

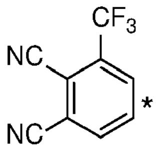 (D31), 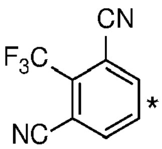 (D32), 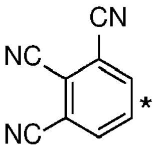 (D33), 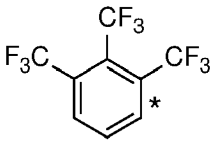 (D34),

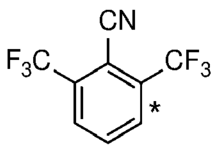 (D35), 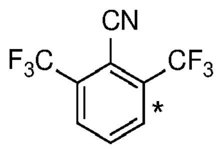 (D36), 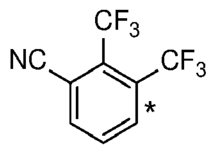 (D37), 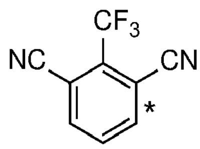 (D38),

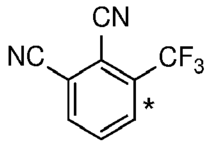 (D39), 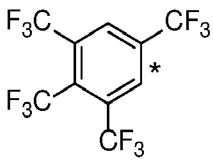 (D40), 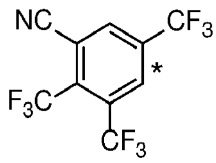 (D41), 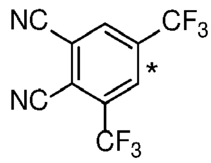 (D42),

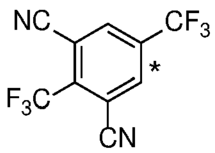 (D43), 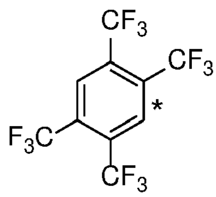 (D44), 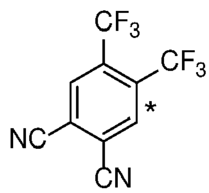 (D45), 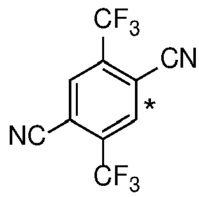 (D46),

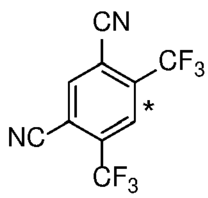 (D47), 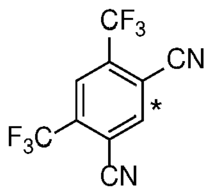 (D48), 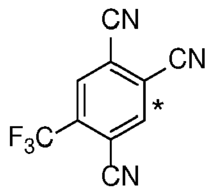 (D49), 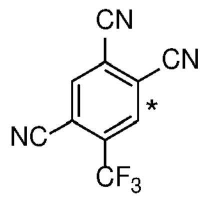 (D50),

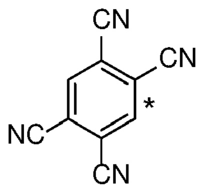 (D51), 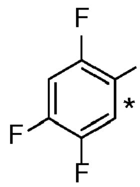 (D52), 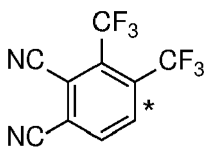 (D53), 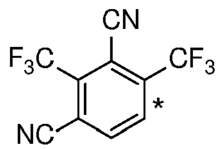 (D54),

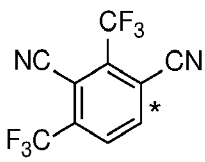 (D55), 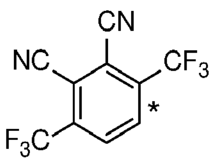 (D56), 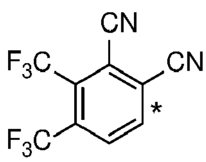 (D57), 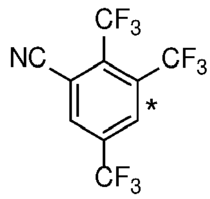 (D58),

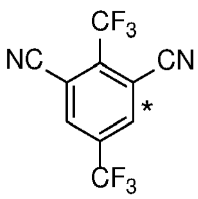 (D59), 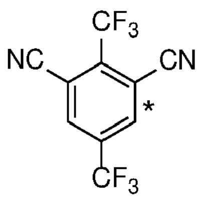 (D60), 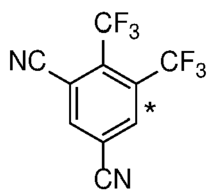 (D61), 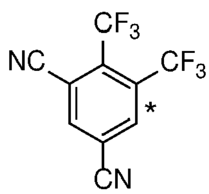 (D62),

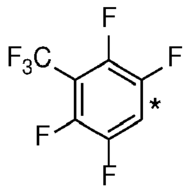 (D63), 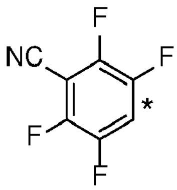 (D64), 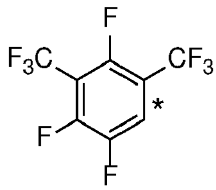 (D65), 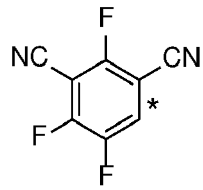 (D66),

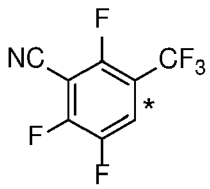 (D67), 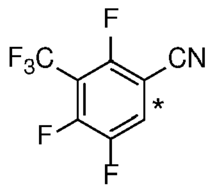 (D68), 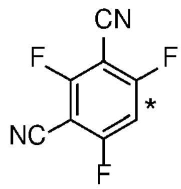 (D69), 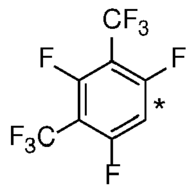 (D70),

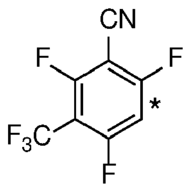 (D71), 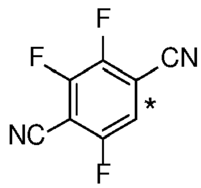 (D72), 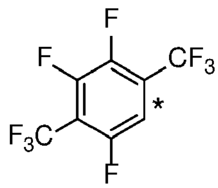 (D73), 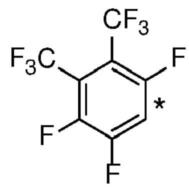 (D74),

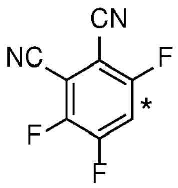 (D75), 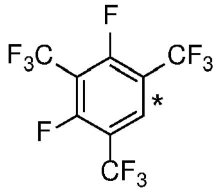 (D76), 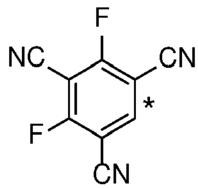 (D77), 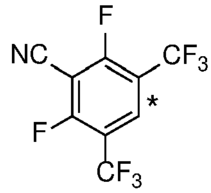 (D78),

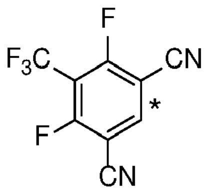 (D79), 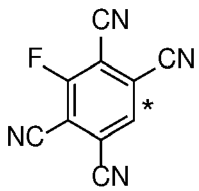 (D80), 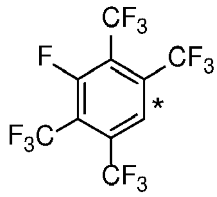 (D81), 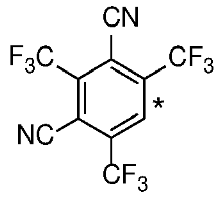 (D82),

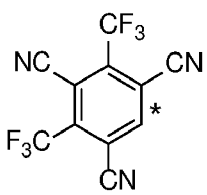 (D83), 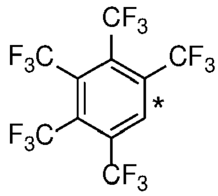 (D84), 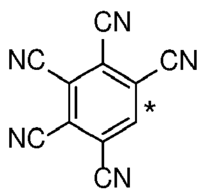 (D85), 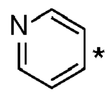 (D86),

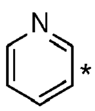 (D87), 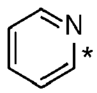 (D88), 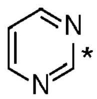 (D89), 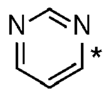 (D90), 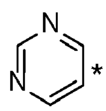 (D91), 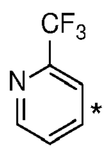 (D92),

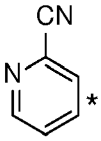 (D93), 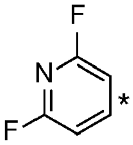 (D94), 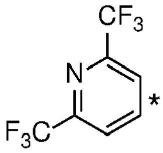 (D95), 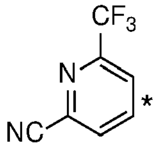 (D96), 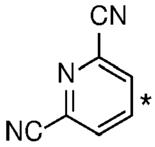 (D97),

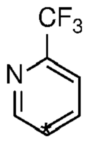 (D98), 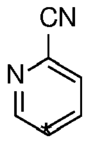 (D99), 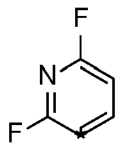 (D100), 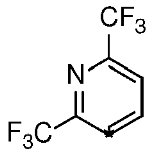 (D101), 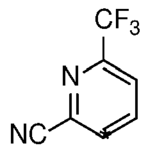 (D102),

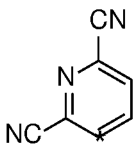 (D103), 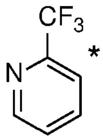 (D104), 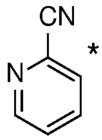 (D105), 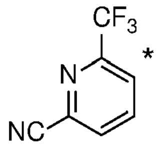 (D106), 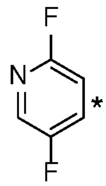 (D107),

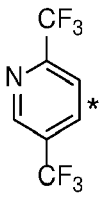 (D108), 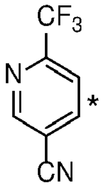 (D109), 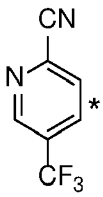 (D110), 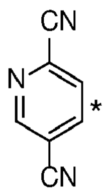 (D111), 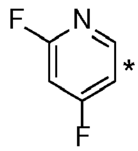 (D112),

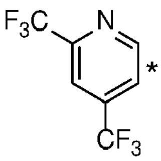 (D113), 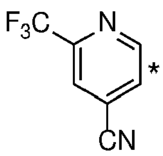 (D114), 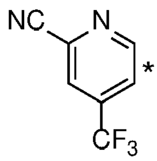 (D115), 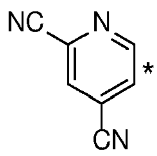 (D116),

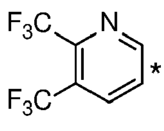 (D117), 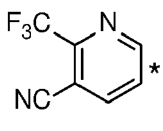 (D118), 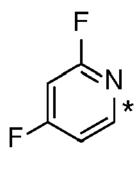 (D119), 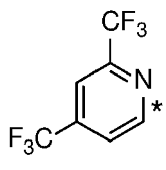 (D120),

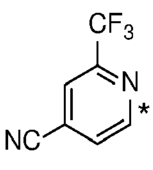 (D121), 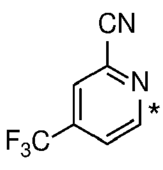 (D122), 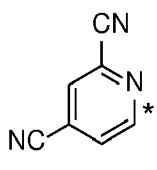 (D123), 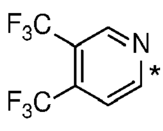 (D124),

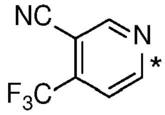 (D125), 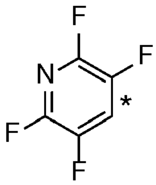 (D126), 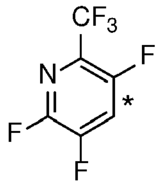 (D127), 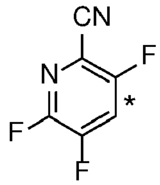 (D128),

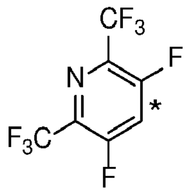 (D129), 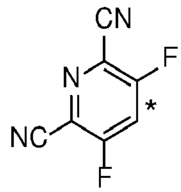 (D130), 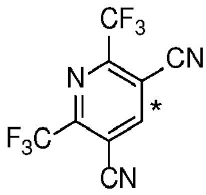 (D131), 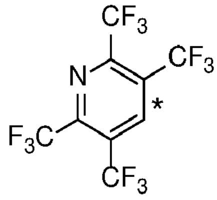 (D132),

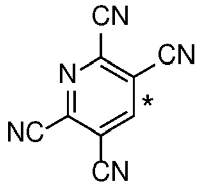 (D133), 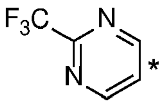 (D134), 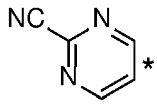 (D135), 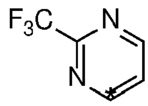 (D136),

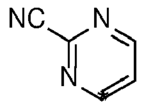 (D137), 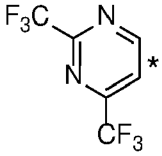 (D138), 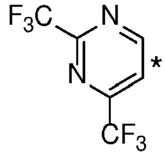 (D139), 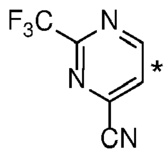 (D140),

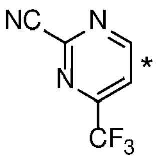 (D141), 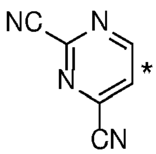 (D142), 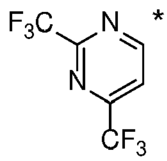 (D143), 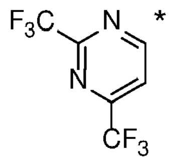 (D144),

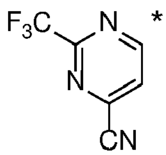 (D145), 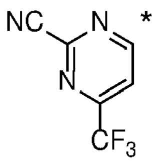 (D146), 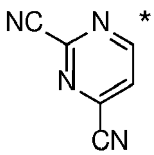 (D147), 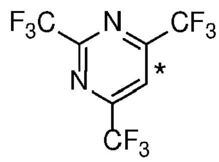 (D148),

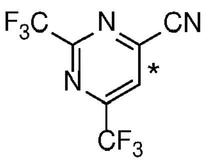 (D149), 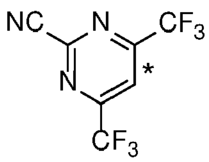 (D150), 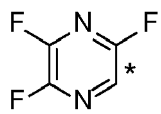 (D151), 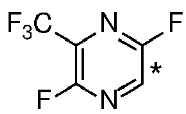 (D152),

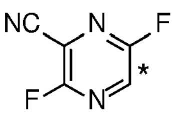 (D153), 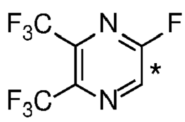 (D154), 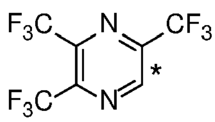 (D155), 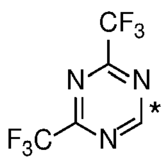 (D156),

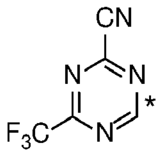 (D157), 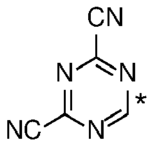 (D158), 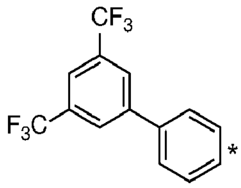 (D159), 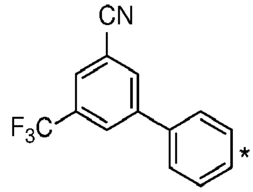 (D160),

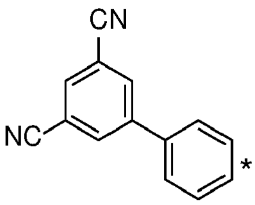 (D161), 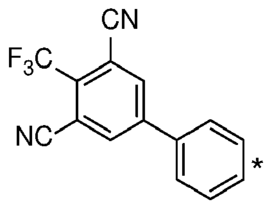 (D162), 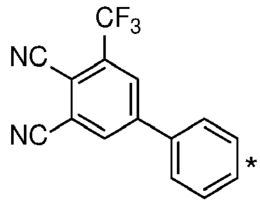 (D163),

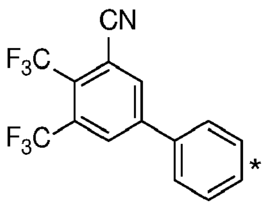 (D164), 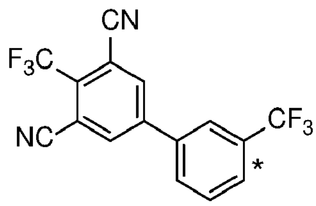 (D165), 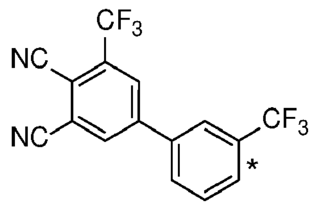 (D166),

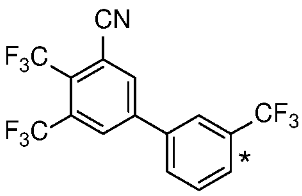 (D167), 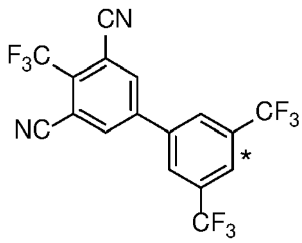 (D168), 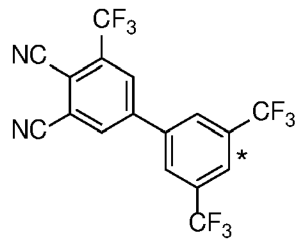 (D169),

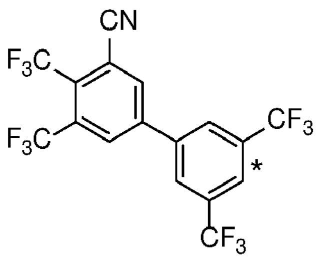 (D170);

wherein the "*" denotes the binding position.

**11.** The compound of formula (I) according to any of the preceding claims 1 to 9, wherein formula (IVb) is selected from the group comprising of F1 to F7:

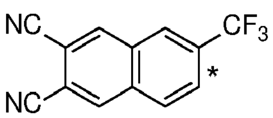 (F1), 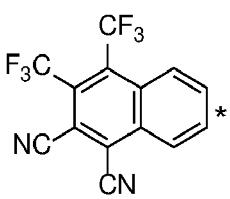 (F2), 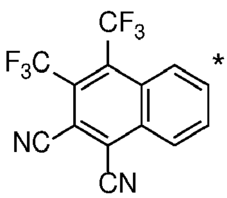 (F3),

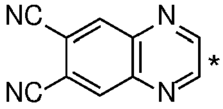 (F4), 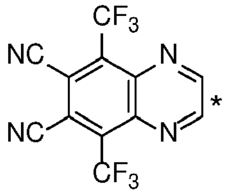 (F5), 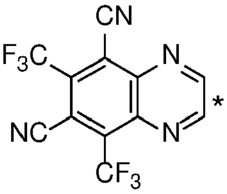 (F6),

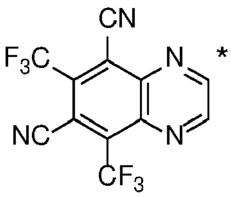 (F7);

wherein the "*" denotes the binding position.

11. The compound of formula (I) according to any of the preceding claims 1 to 10, wherein the compound of formula (I) is selected as follows:

$R^1$, $R^2$, $R^3$ and $R^4$ are F and R' is (A25) and R" is (A25);
$R^1$, $R^2$, $R^3$ and $R^4$ are F and R' is (A23) and R" is (A23);
$R^1$ is Cl, $R^2$ is F, $R^3$ is F, $R^4$ is Cl and R' is (A23) and R" is (A23);
$R^1$ is $CF_3$, $R^2$ is F, $R^3$ is F, $R^4$ is $CF_3$ and R' is (A23) and R" is (A23);
$R^1$ is CN, $R^2$ is CN, $R^3$ is F, $R^4$ is F and R' is (A23) and R" is (A23);
$R^1$ is CN, $R^2$ is F, $R^3$ is F, $R^4$ is CN and R' is (A25) and R" is (A25);
$R^1$ is CN, $R^2$ is F, $R^3$ is F, $R^4$ is CN and R' is (A47) and R" is (A47);
$R^1$ is CN, $R^2$ is F, $R^3$ is F, $R^4$ is CN and R' is (A67) and R" is (A67);
$R^1$ is CN, $R^2$ is F, $R^3$ is F, $R^4$ is CN and R' is (A109) and R" is (A109);
$R^1$ is CN, $R^2$ is F, $R^3$ is F, $R^4$ is CN and R' is (A89) and R" is (A89);
$R^1$ is CN, $R^2$ is F, $R^3$ is F, $R^4$ is CN and R' is (A135) and R" is (A135);
$R^1$ is CN, $R^2$ is F, $R^3$ is F, $R^4$ is CN and R' is (A128) and R" is (A128);
$R^1$ is CN, $R^2$ is F, $R^3$ is F, $R^4$ is CN and R' is (A25) and R" is (A109);
$R^1$ is F, $R^2$ is F, $R^3$ is F, $R^4$ is F and R' is (A23) and R" is (A25);
$R^1$ is CN, $R^2$ is F, $R^3$ is F, $R^4$ is CN and R' is (A135) and R" is (A25);
$R^1$ is $CF_3$, $R^2$ is CN, $R^3$ is CN, $R^4$ is $CF_3$ and R' is (A25) and R" is (A25);
$R^1$ is F, $R^2$ is F, $R^3$ is F, $R^4$ is F and R' is (A25) and R" is (A143);
$R^1$ is CN, $R^2$ is F, $R^3$ is CN, $R^4$ is F and R' is (A25) and R" is (A143);
$R^1$ is CN, $R^2$ is F, $R^3$ is CN, $R^4$ is F and R' is (A34) and R" is (A143).

**12.** The compound of formula (I) according to any of the preceding claims 1 to 11, wherein the compound of formula (I) is selected from the group comprising of H1 to H19:

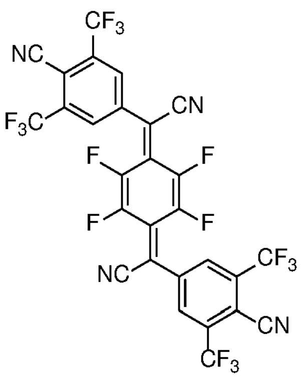 (H1), 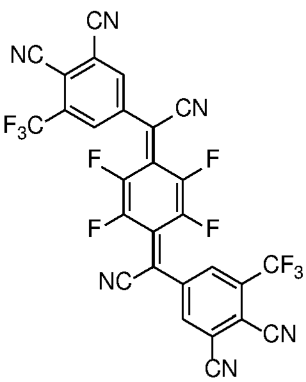 (H2), 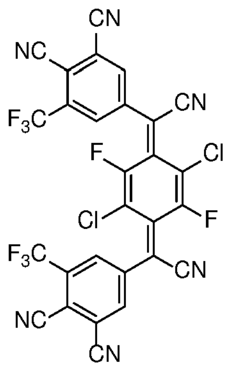 (H3),

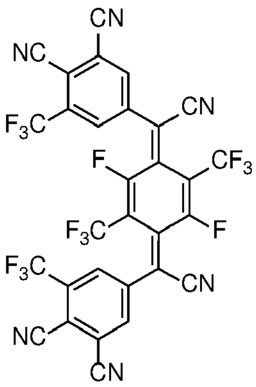 (H4), 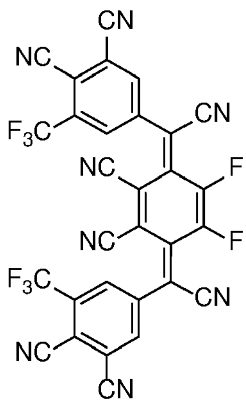 (H5), 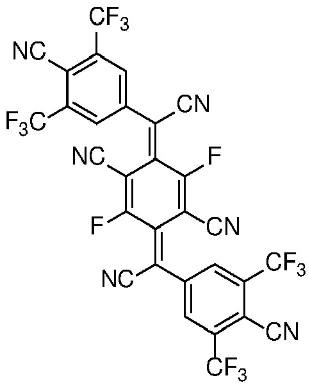 (H6),

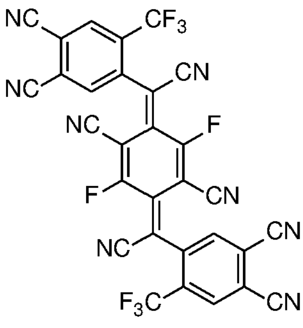 (H7), 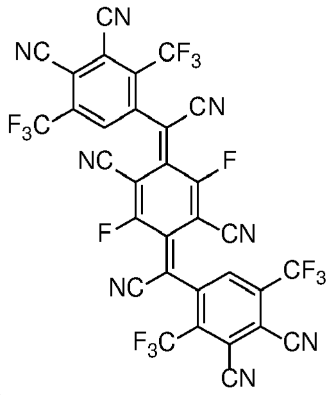 (H8), 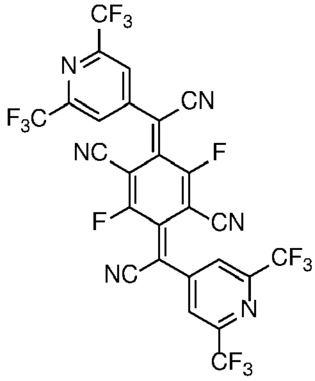 (H9),

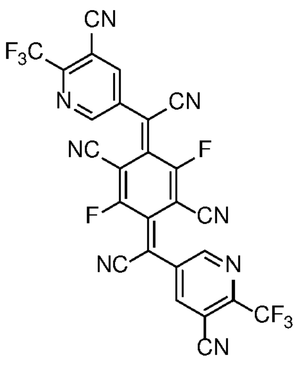 (H10), 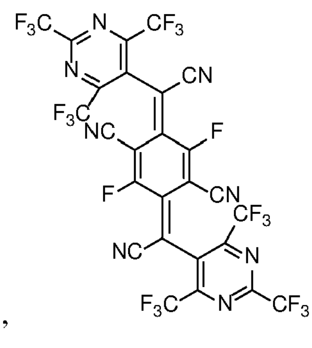 (H11), 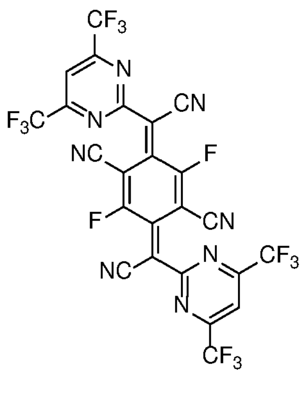 (H12),

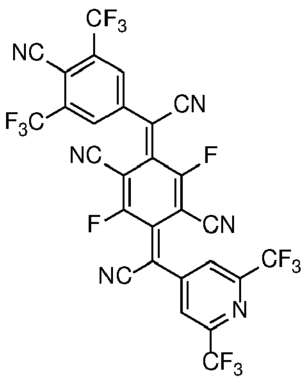 (H13), 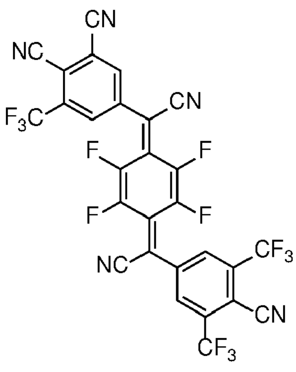 (H14), 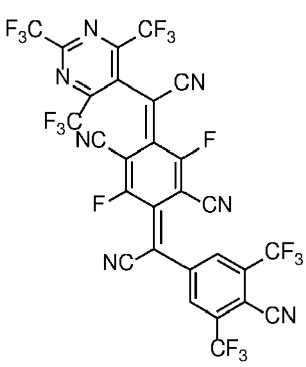 (H15),

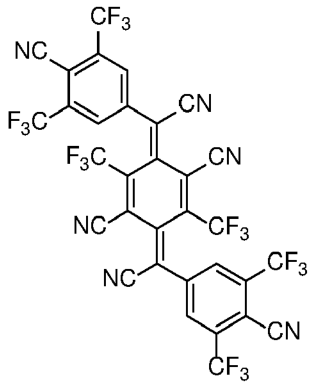 (H16), 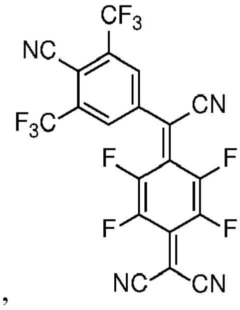 (H17), 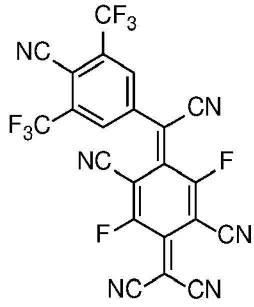 (H18),

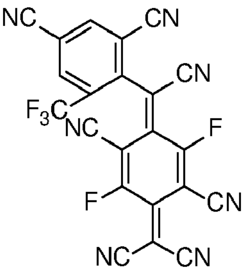 (H19).

**13.** An organic semiconductor layer, whereby the organic semiconductor layer comprises a compound of any of the preceding claims 1 to 12.

**14.** An organic electronic device comprising an anode layer, a cathode layer, and at least one organic semiconductor layer, wherein the at least one organic semiconductor layer is arranged between the anode layer and the cathode layer, and wherein the at least one organic semiconductor layer is the organic semiconductor layer according to claim 13.

**15.** An organic electronic device according to claim 14, whereby the anode layer comprises at least a first anode sub-layer and a second anode sub-layer.

**16.** The organic electronic device of claim 14 or 15, whereby the organic electronic device further comprises at least one photoactive layer, wherein the at least one photoactive layer is arranged between the anode layer and the cathode layer; preferably at least one of the at least one organic semiconductor layers is arranged between the anode layer and the at least one photoactive layer.

**17.** The organic electronic device according to any of the preceding claims 14 to 16, whereby the photoactive layer is a light emitting layer.

**18.** The organic electronic device of any of the preceding claims 14 to 17, whereby the organic electronic device is an electroluminescent device, an organic light emitting diode (OLED), a light emitting device, thin film transistor, a battery, a display device or an organic photovoltaic cell (OPV).

**19.** A display device comprising an organic electronic device according to any of the preceding claims 14 to 18.

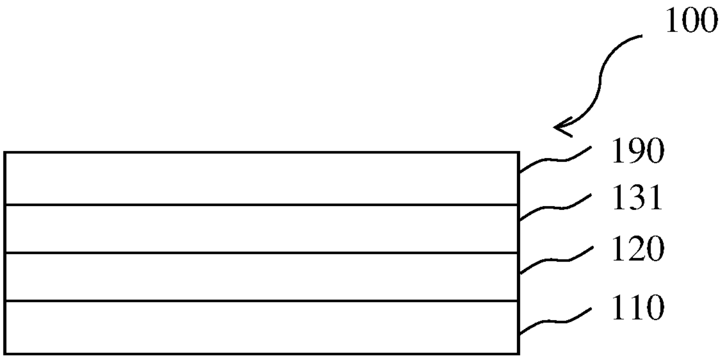


Fig. 1

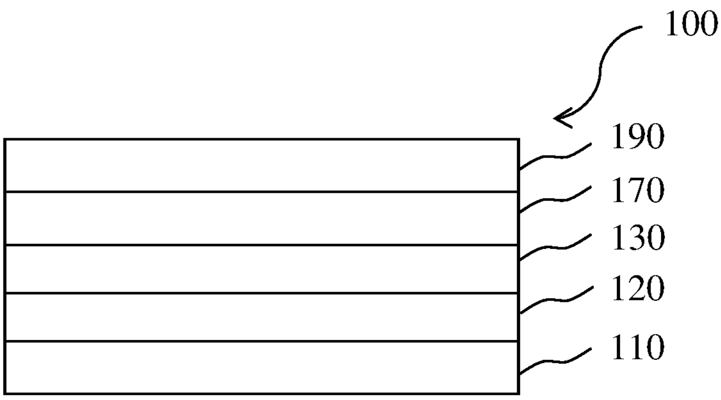


Fig. 2

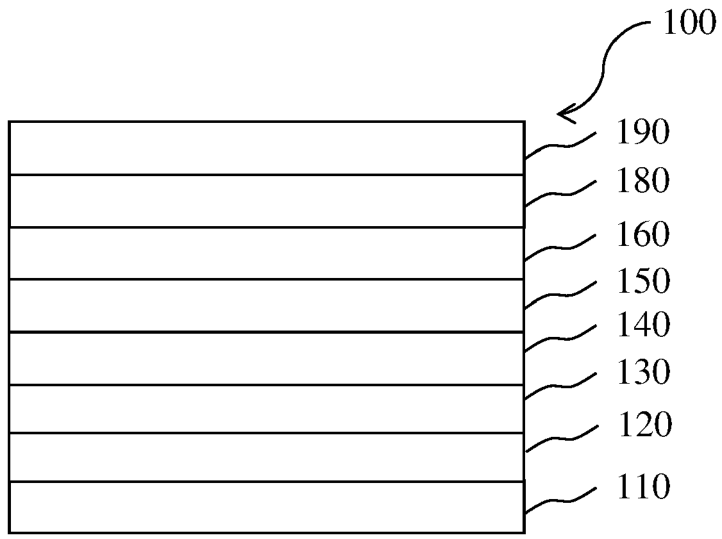
100
190
180
160
150
140
130
120
110

Fig. 3

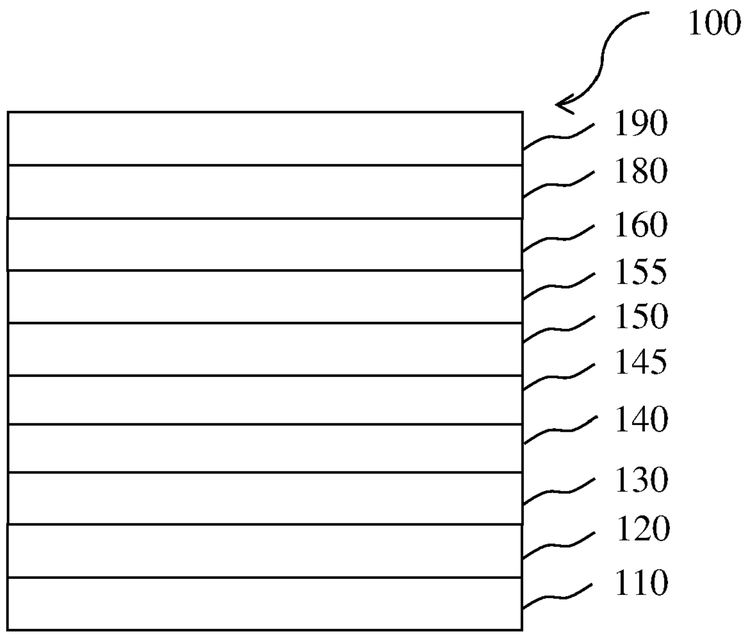
100
190
180
160
155
150
145
140
130
120
110

Fig. 4

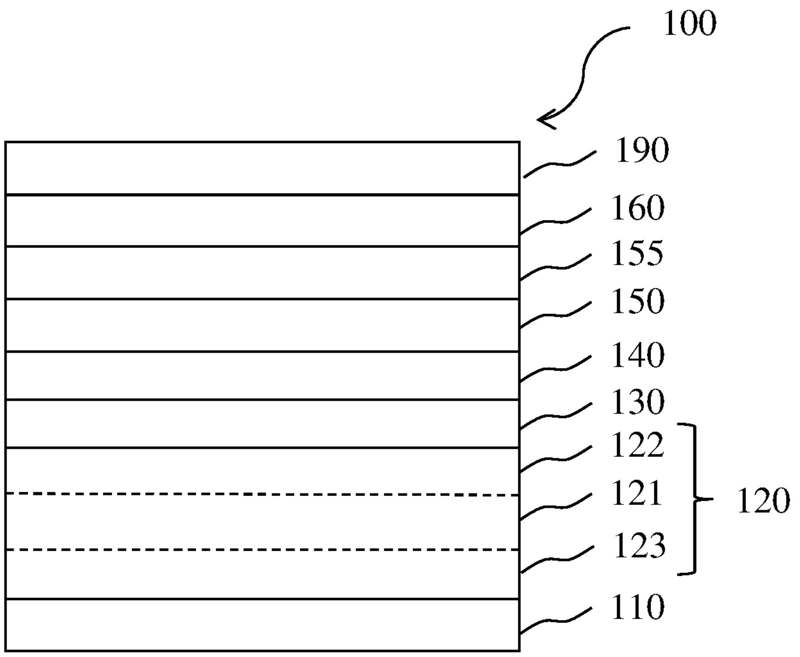
100
190
160
155
150
140
130
122
121
123
120
110

Fig. 5

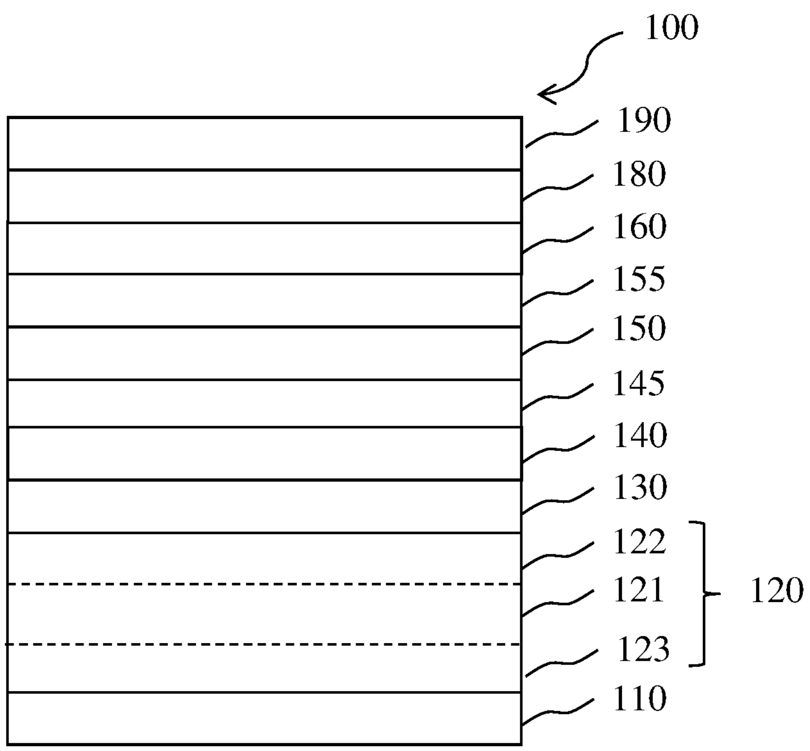
100
190
180
160
155
150
145
140
130
122
121
123
120
110

Fig. 6

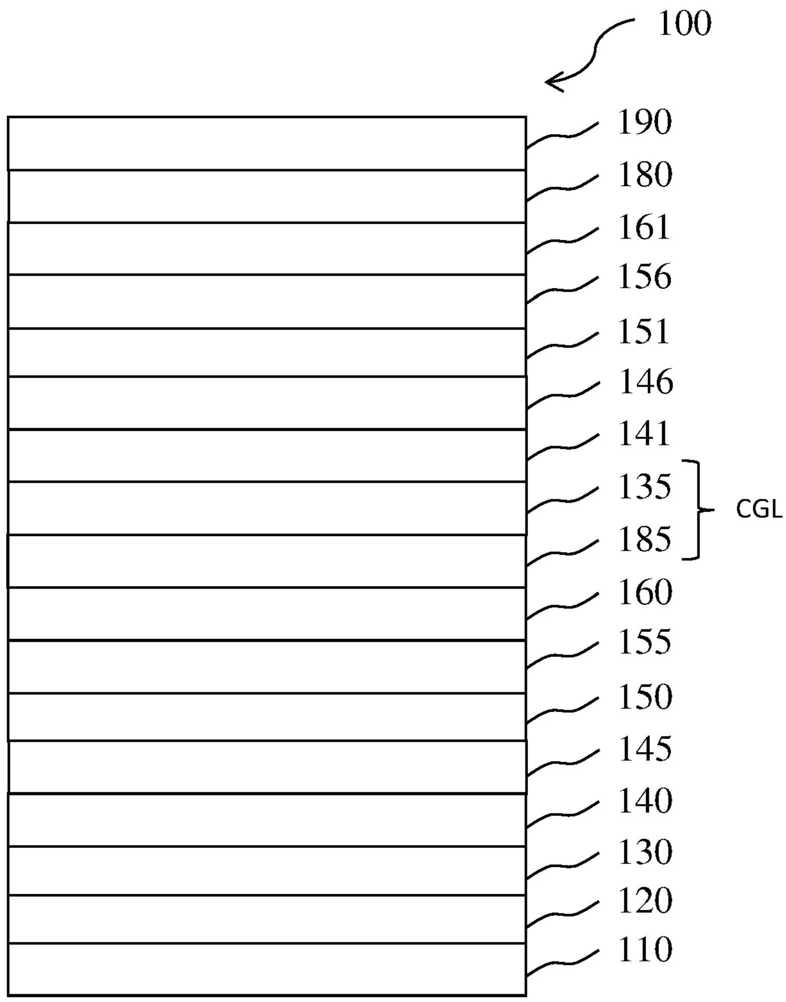
100
190
180
161
156
151
146
141
135
185
CGL
160
155
150
145
140
130
120
110

Fig. 7

Europäisches Patentamt
European Patent Office
Office européen des brevets

# EUROPEAN SEARCH REPORT

Application Number
EP 22 15 4720

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP H04 338760 A (KONISHIROKU PHOTO IND) 26 November 1992 (1992-11-26) * compounds C, C13; pages 3, 20; claim 3 * | 1-3,5,6, 9,14 | INV. C07C255/51 C07D213/26 C07D213/85 C07D239/26 H01L51/00 |
| X | US 2010/193774 A1 (ZEIKA OLAF [US] ET AL) 5 August 2010 (2010-08-05) * compounds 1; paragraphs [0001] - [0010], [0044] - [0050]; claims 1, 10, 13, 15; examples * | 1-20 | |
| X | WO 2018/178273 A1 (NOVALED GMBH [DE]) 4 October 2018 (2018-10-04) * compound P10; page 1, paragraph 1; claims 1, 15-17; examples * | 1-20 | |
| X | KR 2017 0093274 A (DONGJIN SEMICHEM CO LTD [KR]) 16 August 2017 (2017-08-16) * compounds 3, 3-1, B6-B12, B18-B24, B30-B36; paragraphs [0001] - [0008], [0018], [0057]; claims 1, 4, 8, 9; examples * | 1-20 | TECHNICAL FIELDS SEARCHED (IPC) |
| X | CN 109 928 894 A (NINGBO LUMILAN NEW MAT CO LTD) 25 June 2019 (2019-06-25) * compounds I-7, C19; paragraphs [0002] - [0008], [0022] - [0024]; claims 1, 4-8; examples 11-19 * | 1-20 | C07C C07D H01L |
| X | CN 110 372 540 A (WUHAN SUNSHINE OPTOELECTRONICS TECH CO LTD) 25 October 2019 (2019-10-25) * compounds V, 169; paragraphs [0002] - [0006], [0047], [0050], [0190] - [0199]; claims 1, 4-8; example 9 * | 1-20 | |

The present search report has been drawn up for all claims

3

| Place of search | Date of completion of the search | Examiner |
|---|---|---|
| Munich | 30 June 2022 | Ladenburger, Claude |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 15 4720

This annex lists the patent family members relating to the patent documents cited in the above-mentioned European search report. The members are as contained in the European Patent Office EDP file on
The European Patent Office is in no way liable for these particulars which are merely given for the purpose of information.

30-06-2022

| Patent document cited in search report | Publication date | Patent family member(s) | Publication date |
|---|---|---|---|
| JP H04338760 A | 26-11-1992 | NONE | |
| US 2010193774 A1 | 05-08-2010 | DE 102007031220 A1 | 08-01-2009 |
| | | JP 5421249 B2 | 19-02-2014 |
| | | JP 2010533646 A | 28-10-2010 |
| | | TW 200906772 A | 16-02-2009 |
| | | US 2010193774 A1 | 05-08-2010 |
| | | US 2017012203 A1 | 12-01-2017 |
| | | WO 2009003455 A1 | 08-01-2009 |
| WO 2018178273 A1 | 04-10-2018 | CN 110612614 A | 24-12-2019 |
| | | EP 3382770 A1 | 03-10-2018 |
| | | JP 2020519002 A | 25-06-2020 |
| | | KR 20190134687 A | 04-12-2019 |
| | | US 2020032093 A1 | 30-01-2020 |
| | | WO 2018178273 A1 | 04-10-2018 |
| KR 20170093274 A | 16-08-2017 | NONE | |
| CN 109928894 A | 25-06-2019 | CN 109928894 A | 25-06-2019 |
| | | CN 114516821 A | 20-05-2022 |
| | | CN 114560789 A | 31-05-2022 |
| | | CN 114573481 A | 03-06-2022 |
| CN 110372540 A | 25-10-2019 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2722908 A1
- US 2005121667 A1


### Non-patent literature cited in the description


- **YASUHIKO SHIROTA ; HIROSHI KAGEYAMA.** *Chem. Rev.,* 2007, vol. 107, 953-1010
- *CHEMICAL ABSTRACTS,* 1613079-70-1